(19) Europäisches Patentamt
European Patent Office
Office européen des brevets

(11) EP 0 973 763 B1

(12) # FASCICULE DE BREVET EUROPEEN

(45) Date de publication et mention de la délivrance du brevet:
**28.05.2003 Bulletin 2003/22**

(21) Numéro de dépôt: **98909540.1**

(22) Date de dépôt: **16.02.1998**

(51) Int Cl.[7]: **C07D 333/38**, A61K 31/38, A61K 31/50, A61K 31/49, C07D 409/12, C07C 235/56, C07D 295/18, C07C 275/36, C07C 235/30, C07C 281/14, C07D 311/66, C07D 209/42, C07D 409/14, C07D 243/08

(86) Numéro de dépôt international:
**PCT/FR98/00288**

(87) Numéro de publication internationale:
**WO 98/042696 (01.10.1998 Gazette 1998/39)**

(54) **NOUVEAUX DERIVES DU 2-(IMINOMETHYL)AMINO-PHENYLE, LEUR PREPARATION, LEUR APPLICATION A TITRE DE MEDICAMENTS ET LES COMPOSITIONS PHARMACEUTIQUES LES CONTENANT**

2-(IMINOETHYL)-AMINOPHENYL - DERIVATE, DEREN HERSTELLUNG, DEREN VERWENDUNG ALS MEDIKAMENTE UND DIESE ENTHALTENDE PHARMAZEUTISCHE ZUBEREITUNGEN

NOVEL 2-(IMINOMETHYL)AMINO-PHENYL DERIVATIVES, PREPARATION, APPLICATION AS MEDICINES AND PHARMACEUTICAL COMPOSITIONS CONTAINING SAME

(84) Etats contractants désignés:
**AT BE CH DE DK ES FI FR GB GR IE IT LI LU MC NL PT SE**
Etats d'extension désignés:
**RO SI**

(30) Priorité: **24.03.1997 FR 9703528**

(43) Date de publication de la demande:
**26.01.2000 Bulletin 2000/04**

(73) Titulaire: **SOCIETE DE CONSEILS DE RECHERCHES ET D'APPLICATIONS SCIENTIFIQUES S.A. (S.C.R.A.S.)**
**75016 Paris (FR)**

(72) Inventeurs:
- **CHABRIER de LASSAUNIERE, Pierre-Etienne**
  **F-75016 Paris (FR)**
- **AUVIN, Serge**
  **F-91240 Saint-Michel-sur-Orge (FR)**
- **BIGG, Dennis**
  **F-91190 Gif-sur-Yvette (FR)**
- **AUGUET, Michel**
  **F-91120 Palaiseau (FR)**

(74) Mandataire: **Bourgouin, André**
**BEAUFOUR IPSEN (S.C.A.F.)**
**42, rue du Docteur Blanche**
**75016 Paris (FR)**

(56) Documents cités:
**WO-A-94/21621** **WO-A-95/05363**
**WO-A-96/01817**

EP 0 973 763 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des nouveaux dérivés du 2-(iminométhyl)amino-phényle présentant une activité inhibitrice des enzymes NO-synthases produisant le monoxyde d'azote NO et / ou une activité piégeuse des formes réactives de l'oxygène (ROS pour "*reactive oxygen species* "). L'invention concerne les dérivés correspondant à la formule générale **(I)** définie ci-après, leurs méthodes de préparation, les préparations pharmaceutiques les contenant et leur utilisation à des fins thérapeutiques, en particulier leur utilisation en tant qu'inhibiteur des NO-synthases et piégeur de formes réactives de l'oxygène de manière sélective ou non.

Compte tenu du rôle potentiel du NO et des ROS en physiopathologie, les nouveaux dérivés décrits répondant à la formule générale **(I)** peuvent produire des effets bénéfiques ou favorables dans le traitement de pathologies où ces espèces chimiques sont impliquées. Notamment :

- troubles cardio-vasculaires et cérébro-vasculaires comprenant par exemple l'athérosclérose, la migraine, l'hypertension artérielle, le choc septique, les infarctus cardiaques ou cérébraux d'origine ischémique ou hémorragiques, les ischémies et les thromboses.
- troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique mais aussi la douleur, les traumatismes cérébraux ou de la moelle épinière, l'addiction aux opiacées, à l'alcool et aux substances induisant une accoutumance, les troubles de l'érection et de la reproduction, les désordres cognitifs, les encéphalopathies, les encéphalopathies d'origine virale ou toxique.
- troubles du muscle squelettique et des jonctions neuromusculaires (myopathie, myosite) ainsi que les maladies cutanées.
- les maladies prolifératives et inflammatoires comme par exemple l'athérosclérose, l'hypertension pulmonaire, la détresse respiratoire, la glomérulonéphrite, l'hypertension portale, le psoriasis, l'arthrose et l'arthrite rhumatoïde, les fibroses, les amyloïdoses, les inflammations du système gastro-intestinal (colite, maladie de Crohn) ou du système pulmonaire et des voies aériennes (asthme, sinusites, rhinites).
- les transplantations d'organes.
- les maladies auto-immunes et virales comme par exemple le lupus, le sida, les infections parasitaires et virales, le diabète, la sclérose en plaques.
- le cancer.
- les maladies neurologiques associées à des intoxications (empoisonnement au Cadmium, inhalation de n-hexane, pesticide, herbicide), à des traitements (radiothérapie) ou à des désordres d'origine génétique (maladie de Wilson).
- toutes les pathologies caractérisées par une production excessive ou un dysfonctionnement de NO et/ou des ROS.

Dans l'ensemble de ces pathologies, il existe des évidences expérimentales démontrant l'implication du NO ou des ROS (*J. Med. Chem.* (1995) **38,** 4343-4362 ; *Free Radic. Biol. Med.* (1996) **20,** 675-705 ; *The Neuroscientist* (1997) **3,** 327-333).

Par ailleurs, des inhibiteurs de NO Synthases, leur utilisation, et plus récemment l'association de ces inhibiteurs avec des produits possédant des propriétés antioxydantes ou antiradicalaires ont déjà décrites dans des brevets antérieurs (respectivement US patent 5,081,148; US patent 5,360,925 et demande de brevet non publiée). La demande de brevet WO-A-95/05363, qui est considéré comme étant l'état de la technique le plus proche de l'objet de la présente invention montre de dérivés du 2- (iminométhyl)amino-phényle. Les composés de ce brevet possèdent aussi une activité d'inhibition de la NO synthase et ils sont utilisés comme médicaments notamment dans le traitement des maladies neurodégénératives ou la migraine.

La présente invention a pour objet des dérivés de 2-(iminométhyl)amino-phényle, leur préparation et leur application en thérapeutique.

Les composés de l'invention répondent à la formule générale **(I)** :

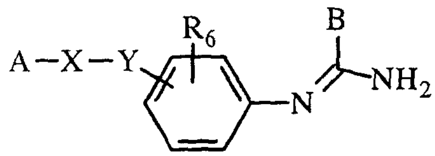

(I)

dans laquelle :

A représente :

soit un radical

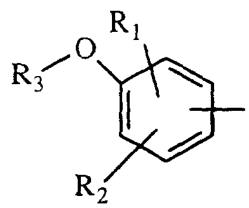

dans lequel $R_1$ et $R_2$ représentent, indépendamment, un atome d'hydrogène, un halogène, le groupe OH, un radical alkyle ou alkoxy linéaire ou ramifié ayant de 1 à 6 atomes de carbone,
$R_3$ représente un atome d'hydrogène, un radical alkyle linéaire ou ramifié ayant de 1 à 6 atomes de carbone ou un radical $-COR_4$,
$R_4$ représentant un radical alkyle linéaire ou ramifié ayant de 1 à 6 atomes de carbone,

soit un radical

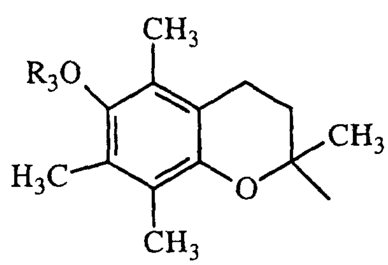

dans lequel $R_3$ a la signification indiquée ci-dessus

soit un radical

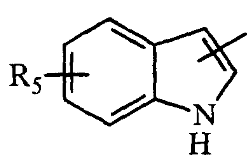

dans lequel $R_5$ représente un atome d'hydrogène, le groupe OH ou un radical alkyle ou alkoxy linéaire ou ramifié ayant de 1 à 6 atomes de carbone ;

B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;

X représente $-Z_1-$, $-Z_1-CO-$, -CH=CH-CO-, $-Z_1-NR_3-CO-$, $-Z_1-NR_3-CS-$, $-Z_1-NR_3-SO_2-$ ou une simple liaison ;

Y représente un radical choisi parmi les radicaux $-Z_2-Q$, pipérazine, homopipérazine, 2-méthylpipérazine, 2,5-diméthyl-pipérazine, 4-aminopipéridine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ ou $-S-Z_2-Q-$,
dans lesquels Q représente une simple liaison, $O-Z_3$ , $R_3-N-Z_3$ ou $S-Z_3$ ;

$Z_1$, $Z_2$ et $Z_3$ représentent indépendamment une simple liaison ou un radical alkylène linéaire ou ramifié ayant de 1 à 6 atomes de carbone; de préférence, $Z_1$, $Z_2$ et $Z_3$ représentent $-(CH_2)_m-$, m étant un entier compris entre 0 et 6 ;

$R_6$ représente un atome d'hydrogène ou un groupe OH ;

ou sont des sels de ces derniers, à l'exception toutefois du composé de formule suivante:

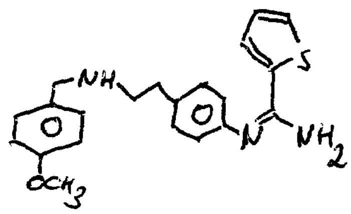

Les composés de formule générale **(I)** contenant un centre asymétrique présentent des formes isomères. Les racémiques et les énantiomères de ces composés font également partie de cette invention.
Les composés de l'invention peuvent exister à l'état de bases ou de sels d'addition notamment à des acides organiques ou inorganiques ou à des bases, et en particulier à l'état d'hydrates, de chlorhydrates, de dichlorhydrates, de fumarates ou d'hémifumarates.
Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par alkoxy linéaire ou ramifié ayant de 1 à 6 atomes de carbone, on entend les radicaux dont le radical alkyle a la signification indiquée précédemment.
Par halogène, on entend les atomes de fluor, de chlore, de brome ou d'iode.
L'invention a en particulier pour objet les composés de formule générale **(I)** suivants, décrits dans les exemples (sous la forme d'un sel pour certains) :

- 3,5-bis-(1,1-diméthyléthyl)-4-hydroxy-N- {4-[(2-thiényl (imino)méthyl)amino]phényl}-benzamide;
- 3,5-bis-(1,1-diméthyléthyl)-4-hydroxy-N-{4-[[(2-thiényl (imino)méthyl)amino]phényl]méthyl } -benzamide;
- 4-acétoxy-3,5-diméthoxy-N-{4-[[(2-thiényl(imino)méthyl)amino]phényl]méthyl}-benzamide;
- 3,5-diméthoxy-4-hydroxy-N-{4-[[(2-thiényl(imino)méthyl)amino]phényl]méthyl}-benzamide;
- 3,5-bis-(1,1-diméthyléthyl)-4-hydroxy-N- {4-[2-[(2-thiényl-(imino)méthyl)amino]phényl]éthyl}-benzamide;
- 4-acétoxy-3,5-diméthoxy-N-{4-[2-[(2-thiényl-(imino)méthyl)-amino]phényl]éthyl}-benzamide;
- 3,5-diméthoxy-4-hydroxy-N-{4-[2-[(2-thiényl-(imino)méthyl)-amino]phényl]éthyl}-benzamide;
- 3,4,5-trihydroxy-N-{4-[2-[(2-thiényl(imino)méthyl)-amino]phényl]éthyl}-benzamide;
- N-{4-[4-[3,5-bis-(1,1-diméthyléthyl)-4-hydroxybenzoyl]-1-pipérazinyl]-phényl}-2-thiophènecarboximidamide;
- N-{4-[4-[3,5 -bis-(1,1-diméthyléthyl)-4-hydroxybenzyl]-1-pipérazinyl]phényl}-2-thiophènecarboximidamide;
- N-{4-[4-[3,5-diméthoxy-4-hydroxybenzoyl]-1-pipérazinyl]-phényl}-2-thiophènecarboximidamide;
- 3,4-dihydro-6-hydroxy-2,5,7,8-tétraméthyl-N-{4-[(2-thiényl (imino)méthyl)amino]phényl )-2H-1-benzopyran-2-carboxamide;
- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- N-{4-[4-[(5-méthoxy-1H-indol-3-yl)méthylcarbonyl]-1-pipérazinyl]phényl}-2-thiophènecarboximidamide;
- N-[4-[4-[ {3-[3,5-bis-(1,1-diméthyléthyl)-4-hydroxyphényl]-1-oxo-2-propényl}-1-pipérazinyl]-phényl]]-2-thiophènecarboximidamide;

- 3,5-bis-(1,1-diméthyléthyl)-4-hydroxy-N-{3-[[(2-thiényl-(imino)méthyl)amino]phényl]méthyl}-benzamide;
- N-[3,5-bis-(1,1-diméthyléthyl)-4-hydroxyphényl]-N' - {{4-[(2-thiényl(imino)méthyl)amino]phényl}méthyl}-urée;
- N-[5-[{3-(3,5-bis-(1,1-diméthyléthyl)-4-hydroxyphényl)-1-oxo-2-propényl}amino]-2-hydroxyphényl]-2-thiophène-carboximidamide;
- N-[3-[{3-(3,5-bis-(1,1-diméthyléthyl)-4-hydroxyphényl)-1-oxo-2-propényl}-amino]-4-hydroxyphényl]-2-thiophène-carboximidamide;
- N-{4-[4-[3,4,5-trihydroxybenzoyl)-1-pipérazinyl)phényl}-2-thiophènecarboximidamide;
- N-[3,5-bis-(1,1-diméthyléthyl)-4-hydroxyphényl]-N'-{{4-[(2-thiényl(imino)méthyl)amino]phényl}carbonylamino}-urée;
- N-[3,5-bis-(1,1-diméthyléthyl)-4-hydroxyphényl]-N'-{{4-[(2-thiényl(imino)méthyl)amino]phényl}méthyl}-thiourée;
- N-[3,5-bis-(1,1-diméthyléthyl)-4-hydroxyphényl]-N'-{2-{4-[(2-thiényl(imino)méthyl)amino]phényl}éthyl}-urée;
- N-(4-{4-[(3,4-dihydro-6-méthoxy-2,5,7,8-tétraméthyl-2H-1-benzopyran-2-yl) carbonyl]-1-pipérazinyl}phényl)-2-thiophènecarboximidamide;
- N-[4-{4-[(3,4-dihydro-6-hydroxy-2,5,7,8-tétraméthyl-2H-1-benzopyran-2-yl) carbonyl]-1H-1,4-diazépin-1-yl}phényl]-2-thiophènecarboximidamide;
- (R)-N-{4-[4-[(3,4-dihydro-6-hydroxy-2,5,7,8-tétraméthyl-2H-1-benzopyran-2-yl)-carbonyl]-1-pipérazinyl]phényl }-2-thiophènecarboximidamide;
- (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- 3,5-bis-(1,1-diméthyléthyl)-4-hydroxy-N-{2-[3-[(2-thiényl(imino)méthyl)amino] phényl]éthyl }-benzamide;
- N-{4-(4-[2-(3,5-bis-(1,1-diméthyléthyl)-4-hydroxyphényl)-1-oxo-éthyl]-1-pipérazinyl)phényl}-2-thiophènecarboximidamide;
- 3,5-bis-(1,1-diméthyléthyl)-4-hydroxy-benzoate de 2-{4-[(2-thiényl(imino)méthyl)amino]phényl}éthyle;
- 3,5-bis-(1,1-diméthyléthyl)-4-hydroxy-benzoate de 2-{3-[(2-thiényl(imino)méthyl)amino]phényl}éthyle;
- 3,5-bis-(1,1-diméthyléthyl)-4-hydroxy-benzoate de 2-{2-[(2-thiényl(imino)méthyl)amino]phényl}éthyle;

ainsi que leurs sels, en particulier leurs chlorhydrates, dichlorhydrates, fumarates ou hémi-fumarates.

On préférera généralement les composés de formule générale **(I)** pour lesquels :

- X représente un radical alkylène linéaire ou ramifié ayant de 1 à 6 atomes de carbone et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$ ou $-NR_3-O-Z_2-$;
  ou
- X représente $-Z_1-CO-$ ou $-CH=CH-CO-$ et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$, $-NR_3-O-Z_2-$, $-O-Z_2-Q-$ ou bien $-NR_3-CO-Q'-$ avec $Q' = R_3-N-Z_3$;
  ou
- X représente $-Z_1-NR_3-CO-$ et Y représente $-Z_2-Q-$, $-NH-Z_2-Q-$, $-NH-CO-Z_2-Q''-$ avec $Q'' = O-Z_3-$, $R_3-N-Z_3-$ ou $S-Z_3-$, ou Y représente $-NR_3-SO_2-NR_3-Z_2-$ ou $-O-Z_2-Q-$;
  ou
- X représente $-Z_1-NH-CO-$ et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$ ou $-NR_3-O-Z_2-$;
  ou
- X représente $-Z_1-NR_3-SO_2-$ et Y représente $-Z_2-Q''-$ avec $Q'' = O-Z_3-$, $R_3-N-Z_3-$ ou $S-Z_3-$, ou bien Y représente $-NR_3-Z_2-Q-$;
  ou
- X représente $-Z_1-$ et Y représente $-O-CO-Z_2-Q-$;
  ou
- X représente $-Z_1-NR_3-CS-$ et Y représente $-NH-Z_2-Q-$, ou un radical pipérazine, homopipérazine, 2-méthylpipérazine, 2,5-diméthyl-pipérazine, 4-aminopipéridine, $-NR_3-Z_2-Q-$, $-NH-NH-Z_2-$ ou $-NR_3-O-Z_2-$;
  ou
- X représente une liaison et Y représente $-O-Z_2-NH-$, $-S-Z_2-NH-$.

On choisira par ailleurs de préférence le groupement X-Y parmi les radicaux suivants :

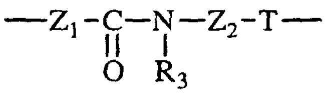

dans lequel T représente une simple liaison, le radical $-NR_3-$ ou le radical $-CO-NR_3-$,
ou

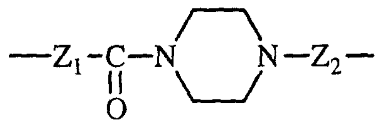

ou

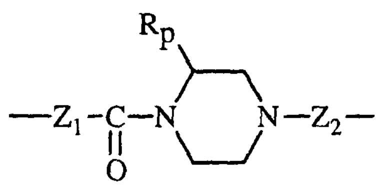

dans lequel $R_p$ représente un atome d'hydrogène ou un radical méthyle,
ou

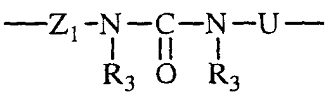

dans lequel U représente un radical $-Z_2$, $-NR_3-CO-$, $-CO-Z_2-O-$, $-CO-$, $-NR_3-$ ou un atome d'oxygène,
ou

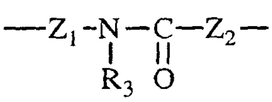

ou

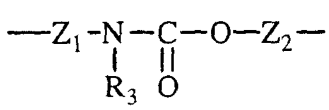

ou

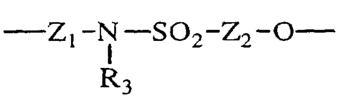

ou

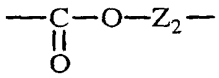

ou

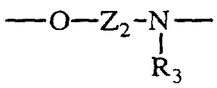

les radicaux $Z_1$, $Z_2$ et $R_3$ ayant la signification indiquée ci-dessus.

De façon plus préférentielle, les composés selon l'invention seront un des composés suivants :

- 3,5-bis-(1,1-diméthyléthyl)-4-hydroxy-N-{4-[2-[(2-thiényl-(imino)méthyl)amino]phényl]éthyl }-benzamide ;
- 3,4,5-trihydroxy-N-{4-[2-[(2-thiényl(imino)méthyl)-amino]phényl]éthyl}-benzamide ;
- N-{4-[4-[3,5-bis-(1,1-diméthyléthyl)-4-hydroxybenzoyl]-1-pipérazinyl]phényl}-2-thiophènecarboximidamide ;
- N-{4-[4-[3,5-bis-(1,1-diméthyléthyl)-4-hydroxybenzyl]-1-pipérazinyl]phényl}-2-thiophènecarboximidamide ;
- 3,4-dihydro-6-hydroxy-2,5,7,8-tétraméthyl-N-{4-[(2- thiényl (imino)méthyl)amino]phényl}-2H-1-benzopyran-2-carboxamide ;
- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide ;
- N-{4-[4-[(5 méthoxy-1H-indol-3-yl)méthylcarbonyl]-1-pipérazinyl]phényl}-2-thiophènecarboximidamide ;
- 3,5-bis-(1,1-diméthyléthyl)-4-hydroxy-N- {3-[[(2-thiényl-(imino)méthyl)amino]phényl]méthyl }-benzamide ;
- N-[3,5-bis-(1,1-diméthyléthyl)-4-hydroxyphényl]-N'-{{4-[(2-thiényl(imino)méthyl)amino]phényl}méthyl}-urée ;
- N-[5-[{3-(3,5-bis-(1,1-diméthyléthyl)-4-hydroxyphényl)-1-oxo-2-propènyl}-amino]-2-hydroxyphényl]-2-thiophènecarboximidamide ;
- N-[3-[ {3-(3,5-bis-(1,1-diméthyléthyl)-4-hydroxyphényl)-1-oxo-2-propènyl}-amino]-4-hydroxyphényl]-2-thiophènecarboximidamide ;
- N-{4-[4-[3,4,5-trihydroxybenzoyl]-1-pipérazinyl]phényl}-2-thiophènecarboximidamide ;
- N-[3,5-bis-(1,1-diméthyléthyl)-4-hydroxyphényl]-N'-{{4-[(2-thiényl(imino)méthyl)amino]phényl}carbonylamino}-urée ;

ou un sel d'un de ces derniers, en particulier un chlorhydrate, dichlorhydrate, fumarate ou hémi-fumarate d'un de ces derniers.

D'autres composés préférés pour l'invention seront les composés suivants :

- 4-acétoxy-3,5-diméthoxy-N-{4-[2-[(2-thiényl-(imino)méthyl)-amino]phényl]éthyl}-benzamide ;
- 3,5-diméthoxy-4-hydroxy-N-{4-[2-[(2-thiényl-(imino)méthyl)-amino]phényl]éthyl}-benzamide ;

ou un sel d'un de ces derniers, en particulier un chlorhydrate, dichlorhydrate, fumarate ou hémi-fumarate d'un de ces derniers.

Des composés tout particulièrement préférés pour l'invention seront les suivants :

- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (S)-N-{4-[4-[(3,4-dihydro-6-hydroxy-2,5,7,8-tétraméthyl-2H-1-benzopyran-2-yl)-carbonyl]-1-pipérazinyl]phényl }-2-thiophènecarboximidamide;

ou un sel d'un de ces derniers, en particulier un chlorhydrate, dichlorhydrate, fumarate ou hémi-fumarate d'un de ces derniers.

Enfin, on préférera particulièrement pour l'invention des composés de formule générale **(I)** présentant les caractéristiques suivantes :

<u>soit</u> :

- A représente :

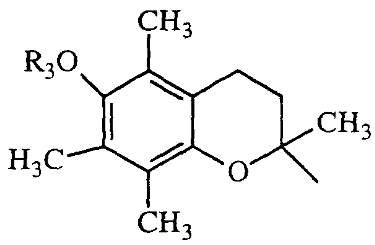

ou

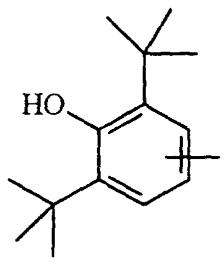

;

- X représente -CO- ou -NH-CO-;
- et Y représente un radical -NH-$Z_2$-Q- ou pipérazine, Q représentant une simple liaison ou un radical O-$Z_3$, $R_3$-N-$Z_3$ ou S-$Z_3$, et $Z_2$ et $Z_3$ représentant indépendamment une liaison ou un radical alkylène linéaire ou ramifié ayant de 1 à 6 atomes de carbone et $R_3$ représente un atome d'hydrogène ou un radical alkyle ayant de 1 à 6 atomes de carbone linéaire ou ramifié.

<u>soit</u> : $R_6$ est un groupe OH.

L'invention a également pour objet, à titre de médicaments, les composés de formule générale **(I)** décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à inhiber la NO synthase neuronale ou la NO synthase inductible, à inhiber la péroxidation lipidique ou à assurer la double fonction d'inhibition de la NO synthase et d'inhibition de la péroxidation lipidique.

Par sel pharmaceutiquement acceptable, on entend notamment des sels d'addition d'acides inorganiques, tels que chlorhydrate, sulfate, phosphate, diphosphate, bromhydrate et nitrate, ou d'acides organiques, tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci.* **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

L'invention offre aussi, à titre de produits industriels nouveaux, les intermédiaires de synthèse des produits de formule générale **(I),** à savoir les produits de formule générale **(II)A** :

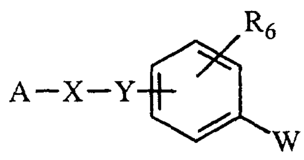

**(II)A**

dans laquelle :

W représente un radical amino ou nitro,
A représente :

soit un radical

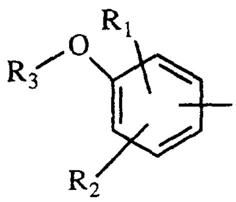

dans lequel $R_1$ et $R_2$ représentent, indépendamment, un atome d'hydrogène, un halogène, le groupe OH, un radical alkyle ou alkoxy linéaire ou ramifié ayant de 1 à 6 atomes de carbone,
$R_3$ représente un atome d'hydrogène, un radical alkyle linéaire ou ramifié ayant de 1 à 6 atomes de carbone ou un radical $-COR_4$,
$R_4$ représentant un radical alkyle linéaire ou ramifié ayant de 1 à 6 atomes de carbone,

soit un radical

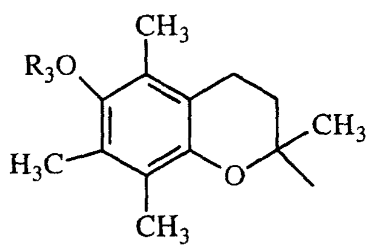

dans lequel $R_3$ a la signification indiquée ci-dessus

soit un radical

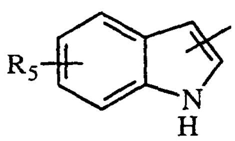

dans lequel $R_5$ représente un atome d'hydrogène, le groupe OH ou un radical alkyle ou alkoxy linéaire ou ramifié ayant de 1 à 6 atomes de carbone ;
X représente $-Z_1-$, $-Z_1-CO-$, $-CH=CH-CO-$, $-Z_1-NR_3-CO-$, $-Z_1-NR_3-CS-$, $-Z_1-NR_3-SO_2-$ ou une simple liaison ;

Y représente un radical choisi parmi les radicaux $-Z_2$-Q, pipérazine, homopipérazine, 2-méthylpipérazine, 2,5-diméthylpipérazine, 4-aminopipéridine, $-NR_3-Z_2$-Q-, $-NR_3$-CO-$Z_2$-Q-, $-NR_3$-NH-CO-$Z_2$-, -NH-NH-$Z_2$-, $-NR_3$-O-$Z_2$-, $-NR_3-SO_2-NR_3-Z_2$-, -O-$Z_2$-Q-, -O-CO-$Z_2$-Q- ou -S-$Z_2$-Q-,
dans lesquels Q représente une simple liaison, O-$Z_3$ , $R_3$-N-$Z_3$ ou S-$Z_3$ ;
$Z_1$, $Z_2$ et $Z_3$ représentent indépendamment une simple liaison ou un radical alkylène linéaire ou ramifié ayant de 1 à 6 atomes de carbone; de préférence, $Z_1$, $Z_2$ et $Z_3$ représentent $-(CH_2)_m-$, m étant un entier compris entre 0 et 6 ;
$R_6$ représente un atome d'hydrogène ou un groupe OH ;

à l'exception toutefois du 3,5-bis-(1,1-diméthyléthyl)-4-hydroxy-N-(4-nitrophényl)-benzamide ;
ou les sels de ces derniers, à l'exception toutefois des composés correspondant aux formules suivantes :

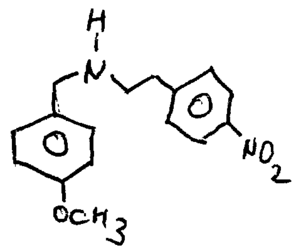

et

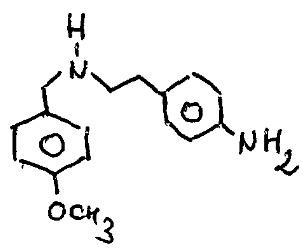

Par ailleurs, l'invention offre en particulier, à titre de produits industriels nouveaux, les composés suivants, qui sont des intermédiaires dans la synthèse de produits de formule générale **(I)** :

- 3,5-bis-(1,1-diméthyléthyl)-4-hydroxy-N-(4-aminophényl)-benzamide ;
- 3,5-bis-(1,1-diméthyléthyl)-4-hydroxy-N-[(4-nitrophényl)méthyl]-benzamide ;
- 3,5-bis-(1,1-diméthyléthyl)-4-hydroxy-N-[(4-aminophényl)méthyl]-benzamide ;
- 4-acétoxy-3,5-diméthoxy-N-[(4-nitrophényl)méthyl]-benzamide ;
- 4-acétoxy-3,5-diméthoxy-N-[(4-aminophényl)méthyl]-benzamide ;
- 3,5-bis-(1,1-diméthyléthyl)-4-hydroxy-N-[2-(4-nitrophényl)éthyl]-benzamide;
- 3,5-bis-(1,1-diméthyléthyl)-4-hydroxy-N-[2-(4-aminophényl)éthyl]-benzamide ;
- 4-acétoxy-3,5-diméthoxy-N-[2-(4-nitrophényl)éthyl]-benzamide ;
- 4-acétoxy-3,5-diméthoxy-N-[2-(4-aminophényl)éthyl]-benzamide ;
- 3,4,5-trihydroxy-N-[2-(4-nitrophényl)éthyl]-benzamide ;
- 3,4,5-trihydroxy-N-[2-(4-aminophényl)éthyl]-benzamide ;
- 2,6-bis-(1,1-diméthyléthyl)-4- {[4-(4-nitrophényl)-1-pipérazinyl]-carbonyl}-phénol ;
- 2,6-bis-(1,1-diméthyléthyl)-4- {[4-(4-aminophényl)-1-pipérazinyl]-carbonyl}-phénol ;
- 2,6-bis-(1,1-diméthyléthyl)-4-{[4-(4-nitrophényl)-1-pipérazinyl]-méthyl}-phénol ;
- 2,6-bis-(1,1-diméthyléthyl)-4-{[4-(4-aminophényl)-1-pipérazinyl]-méthyl}-phénol ;
- 2,6-diméthoxy-4-{[4-(4-nitrophényl)-1-pipérazinyl]carbonyl}-phénol ;
- 2,6-diméthoxy-4-{[4-(4-aminophényl)-1-pipérazinyl]carbonyl}-phénol ;
- 3,4-dihydro-6-hydroxy-2,5,7,8-tétraméthyl-N-(4-nitrophényl)-2H-1-benzopyran-2-carboxamide ;
- 3,4-dihydro-6-hydroxy-2,5,7,8-tétraméthyl-N-(4-aminophényl)-2H-1-benzopyran-2-carboxamide ;
- 3,4-dihydro-2,5,7,8-tétraméthyl-2-{4-[(4-nitrophényl)-1-pipérazinyl]-carbonyl}-2H-1-benzopyran-6-ol ;
- 3,4-dihydro-2,5,7,8-tétraméthyl-2-{4-[(4-aminophényl)-1-pipérazinyl]-carbonyl}-2H-1-benzopyran-6-ol ;

- 1-[(5 méthoxy-1H-indol-3-yl)méthylcarbonyl]-4-(4-nitrophényl)-pipérazine ;
- 1-[(5 méthoxy-1H-indol-3-yl)méthylcarbonyl]-4-(4-aminophényl)-pipérazine ;
- 2,6-bis-(1,1-diméthyléthyl)-4-{3-[4-(4-nitrophényl)-1-pipérazinyl]-3-oxo-2-propènyl}-phénol ;
- 2,6-bis-(1,1-diméthyléthyl)-4-{3-[4-(4-aminophényl)-1-pipérazinyl]-3-oxo-2-propènyl}-phénol ;
- 3,5-bis-(1,1-diméthyléthyl)-4-hydroxy-N-[(3-nitrophényl)méthyl]-benzamide ;
- 3,5-bis-(1,1-diméthyléthyl)-4-hydroxy-N-[(3-aminophényl)méthyl]-benzamide ;
- N-[3,5-bis-(1,1-diméthyléthyl)-4-hydroxyphényl]-N'-[(4-nitrophényl)méthyl]-urée ;
- N-[(4-aminophényl)méthyl]-N'-[3,5-bis-(1,1-diméthyléthyl)-4-hydroxyphényl]-urée ;
- 3-[(3,5-bis-(1,1-diméthyléthyl)-4-hydroxyphényl]-N-(4-hydroxy-3-nitrophényl)-2-propènamide ;
- 3-[(3,5-bis-(1,1-diméthyléthyl)-4-hydroxyphényl]-N-(4-hydroxy-3-aminophényl)-2-propènamide ;
- 3-[(3,5-bis-(1,1-diméthyléthyl)-4-hydroxyphényl]-N-(2-hydroxy-5-nitrophényl)-2-propènamide ;
- 3-[(3,5-bis-(1,1-diméthyléthyl)-4-hydroxyphényl]-N-(2-hydroxy-5-aminophényl)-2-propènamide ;
- 5-{[4-(4-nitrophényl)-1-pipérazinyl]carbonyl}-benzène-1,2,3-triol ;
- 5-{[4-(4-aminophényl)-1-pipérazinyl]carbonyl}-benzène-1,2,3-triol ;
- N-[3,5-bis-(1,1-diméthyléthyl)-4-hydroxyphényl]-N'-[(4-nitrophényl)-carbonylamino]-urée ;
- N-[(4-aminophényl)carbonylamino]-N'-[3,5-bis-(1,1-diméthyléthyl)-4-hydroxyphényl]-urée ;
- N-[3,5-bis-(1,1-diméthyléthyl)-4-hydroxyphényl]-N'-[(4-nitrophényl)méthyl]-thiourée ;
- N-[(4-aminophényl)méthyl]-N'-[3,5-bis-(1,1-diméthyléthyl)-4-hydroxyphényl]-thiourée ;
- N-[3,5-bis-(1,1-diméthyléthyl)-4-hydroxyphényl]-N'-[2-(4-nitrophényl)éihyl]-urée ;
- N-[2-(4-aminophényl)éthyl]-N'-[3,5-bis-(1,1-diméthyléthyl)-4-hydroxyphényl]-urée ;
- 1-{[3,4-dihydro-6-méthoxy-2,5,7,8-tétraméthyl-2H-1-benzopyran-2-yl]carbonyl}-4-(4-nitrophényl)pipérazine ;
- 1-{[3,4-dihydro-6-méthoxy-2,5,7,8-tétraméthyl-2H-1-benzopyran-2-yl]carbonyl}-4-(4-aminophényl)pipérazine ;
- hexahydro-4-(4-nitrophényl)-1H-1,4-diazépine ;
- 1-[(3,4-dihydro-6-hydroxy-2,5,7,8-tétraméthyl-2H-1-benzopyran-2-yl)carbonyl] hexahydro-4-(4-nitrophényl)-1H-1,4-diazépine ;
- 1-(4-aminophényl)-4-[(3,4-dihydro-6-hydroxy-2,5,7,8-tétraméthyl-2H-1-benzopyran-2-yl)carbonyl] hexahydro-1H-1,4-diazépine ;
- chlorhydrate du N-[4-{4-[(3,4-dihydro-6-hydroxy-2,5,7,8-tétraméthyl-2H-1-benzopyran-2-yl)carbonyl]-1H-1,4-diazépin-1-yl}phényl]-2-thiophènecarboximidamide ;
- (*R*)-3,4-dihydro-2,5,7,8-tétraméthyl-2-[4-[(4-nitrophényl)-1-pipérazinyl]-carbonyl}-2H-1-benzopyran-6-ol ;
- (*R*)-3,4-dihydro-2,5,7,8-tétraméthyl-2-{4-[(4-aminophényl)-1-pipérazinyl]-carbonyl}-2H-1-benzopyran-6-ol ;
- (*S*)-3,4-dihydro-2,5,7,8-tétraméthyl-2-{4-[(4-nitrophényl)-1-pipérazinyl]-carbonyl}-2H-1-benzopyran-6-ol ;
- (*S*)-3,4-dihydro-2,5,7,8-tétraméthyl-2-{4-[(4-aminophényl)-1-pipérazinyl]-carbonyl}-2H-1-benzopyran-6-ol ;
- 3,5-bis-(1,1-diméthyléthyl)-4-hydroxy-N-[2-(3-nitrophényl)éthyl]-benzamide ;
- 3,5-bis-(1,1-diméthyléthyl)-4-hydroxy-N-[2-(3-aminophényl)éthyl]-benzamide ;
- 3,5-bis-(1,1-diméthyléthyl)-4-hydroxybenzoate de 2-(4-nitrophényl)éthyle ;
- 3,5-bis-(1,1-diméthyléthyl)-4-hydroxy-benzoate de 2-(4-aminophényl)éthyle ;

ou leurs sels.

Enfin, l'invention offre des procédés de préparation de composés de formule générale **(I)** tels que définis ci-dessus et consistant, par exemple, en la réaction dans un alcool inférieur, tel que méthanol, éthanol, alcool isopropylique ou t-butanol, de préférence dans l'alcool isopropylique, à une température comprise entre 20 et 90 °C, par exemple à 50 °C, et durant une à 48 heures, de préférence durant 15 à 24 heures, éventuellement en présence de DMF, d'un composé de formule générale **(III)** tel que défini ci-dessus avec un composé de formule générale **(IV)**

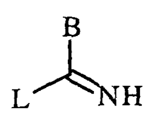

**(IV)**

ledit composé de formule générale **(IV)** pouvant éventuellement être salifié par un acide minéral G, B ayant la signification indiquée ci-dessus et L représentant un groupe partant et notamment un radical alkoxy, thioalkyl, acide sulfonique, halogénure, alcool arylique ou tosyle (d'autres groupes partants bien connus de l'homme du métier et pouvant être éventuellement utilisés pour l'invention sont décrits dans l'ouvrage suivant : *Advanced Organic Chemistry*, J. March, 3rd Edition (1985), Mc Graw-Hill, p. 315). De préférence, G représente HCl, HBr ou HI.

D'autres procédés de fabrication sont envisageables et sont accessibles dans la littérature (par exemple :

The Chemistry of amidines and imidates, Vol. 2, Saul PATAI and Zvi RAPPOPORT, John Wiley & Sons, 1991).

Conformément à l'invention, on peut préparer les composés de formule générale **(I)** par le procédé décrit ci-après.

**<u>Préparation des composés de formule générale (I)</u> :**

Les composés de formule générale **(I)** peuvent être préparés à partir des intermédiaires de formule générale **(II)** selon le schéma 1.

La réduction de la fonction nitro des intermédiaires de formule générale **(II)** est généralement effectuée par hydrogènation catalytique, dans l'éthanol, en présence de Pd/C, sauf dans les cas où X = -CH=CH-CO- ou Y = $-O-CH_2-$, le groupement nitro est sélectivement réduit à l'aide, par exemple, de $SnCl_2$ (*J. Heterocyclic Chem.* (1987), **24,** 927-930; *Tetrahedron Letters* (1984), **25,** (8), 839-842). La réaction s'effectue alors en chauffant le mélange vers 70° C, pendant au moins trois heures, dans l'acétate d'éthyle parfois additionné d'éthanol.

Les dérivés d'aniline de formule générale **(III),** ainsi obtenus, peuvent être condensés sur des dérivés de formule générale **(IV),** par exemple des dérivés du type O-alkyl thioimidate ou S-alkyl thioimidate, pour conduire aux composés finaux de formule générale **(I)** (cf. schéma 1). Par exemple, pour B = thiophène, on peut condenser les dérivés de formule générale **(III)** sur l'iodhydrate de S-méthylthiophène thiocarboxamide, préparé selon une méthode de la littérature (*Ann. Chim.* (1962), **7,** 303-337). La condensation peut s'effectuer par chauffage dans un alcool (par exemple dans du méthanol ou de l'isopropanol), éventuellement en présence de DMF à une température de préférence comprise entre 50 et 100 °C pour une durée généralement comprise entre quelques heures et une nuit.

<u>Schéma 1</u> :

(II)

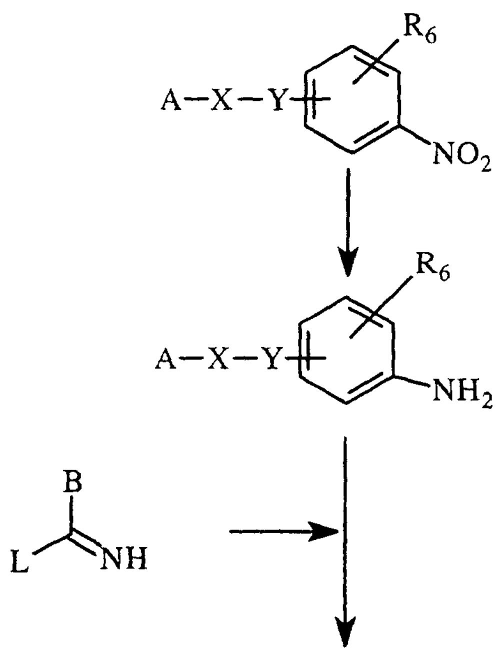

(III)

(IV)

(I)

**<u>Préparation des intermédiaires de formule générale (II)</u> :**

Les intermédiaires de formule générale **(II)** peuvent être préparés par différents procédés selon les fonctions chimiques qui sont mises en place : aminés, carboxamides, urées, thiourées, sulfonamides, aminosulfonylurées, sulfamides, carbamates, éthers, esters, thioéthers, acylurées, etc. :

<u>Lorsque</u> :

X = radical alkylène linéaire ou ramifié ayant de 1 à 6 atomes de carbone
et Y = pipérazine, homopipérazine, 2-méthylpipérazine, 2,5-diméthyl-pipérazine, 4-aminopipéridine, $-NR_3-Z_2-Q-$,

$-NR_3-NH-CO-Z_2-$, $-NH-NH-Z_2-$, $-NR_3-O-Z_2-$

Les amines de formule générale **(II),** schéma 2, dans lesquelles A, X, Y et $R_6$ sont tels que définis ci-dessus, peuvent être obtenues par substitution nucléophile des dérivés halogénés de formule générale **(VI)** par une amine de formule générale **(VII).**

La réaction s'effectue, par exemple, dans le DMF en présence de $K_2CO_3$ à 20° C. Les dérivés halogénés de formule générale **(VI)** sont accessibles, par exemple, par bromation des alcools primaires de formule générale **(V)** à l'aide de $PBr_3$, à 0°C, dans le THF anhydre. Les alcools de formule générale **(V)** qui ne sont pas commerciaux peuvent être préparés selon des méthodes décrites dans la littérature (*Tetrahedron Lett.* (1983), **24**, (24), 2495-2496).

Schéma 2 :

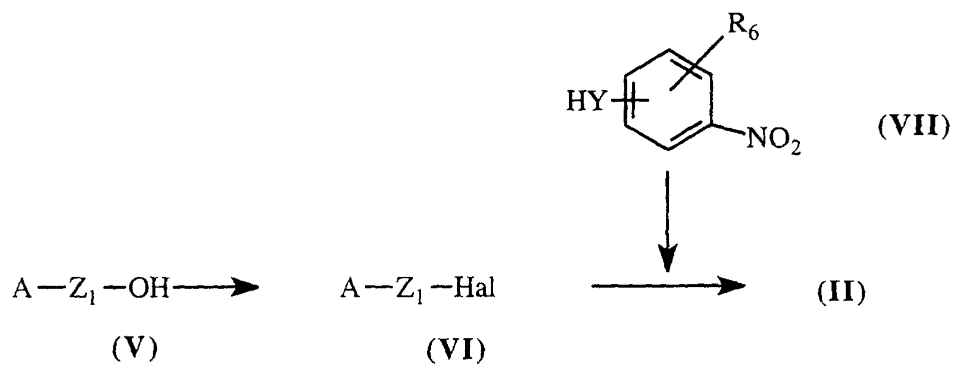

Les amines de formule générale **(VII)** dans lesquelles Y représente homopipérazine, 2,5-diméthylpipérazine, 4-aminopipéridine ou plus généralement $-NR_3-Z_2-NR_3-$, sont synthétisées en trois étapes à partir des diamines commerciales correspondantes. Les diamines sont sélectivement mono-protégées sous forme de carbamate *(Synthesis* (1984), (12), 1032-1033; *Synth. Commun.* (1990), **20,** (16), 2559-2564) avant de réagir par substitution nucléophile sur un fluoronitrobenzène, en particulier le 4-fluoronitrobenzène. Les amines, précédemment protégées, sont libérées à la dernière étape, selon des méthodes décrites dans la littérature (T.W. Greene et P.G.M. Wuts, Protective Groups in Organic Synthesis, Second edition (Wiley-Interscience, 1991)), pour conduire aux intermédiaires de formule générale **(VII).**

Lorsque :

X = $-Z_1-CO-$, -CH=CH-CO-
et Y = pipérazine, homopipérazine, 2-méthylpipérazine, 2,5-diméthyl-pipérazine, 4-aminopipéridine, $-NR_3-Z_2-Q-$, $-NR_3-NH-CO-Z_2-$, $-NH-NH-Z_2-$, $-NR_3-O-Z_2-$

Les carboxamides de formule générale **(II),** schéma 3, dans lesquels A, X, Y et $R_6$ sont tels que définis ci-dessus, sont préparés par condensation des acides carboxyliques commerciaux de formule générale **(VIII)** pour X = $-Z_1-CO-$ et de formule générale **(IX)** pour X = -CH=CH-CO- avec des amines de formule générale **(VII).** Les acides non commerciaux peuvent être synthétisés selon des méthodes similaires à celles décrites dans la littérature (*J. Org. Chem.* (1974), **39** (2), 219-222; *J. Amer. Chem. Soc.* (1957), **79,** 5019-5023, et *CHIMIA* (1991), **45** (4), 121-123 lorsque A représente un radical 6-alkoxy-2,5,7,8-tétraméthylchromane). Les amines de formule générale **(VII)** dans laquelle Y représente homopipérazine, 2,5-diméthylpipérazine, 4-aminopipéridine, ou plus généralement $-NR_3-Z_2-NR_3-$ sont préparées selon des méthodes analogues à celles décrites au paragraphe précédent. Les liaisons carboxamides sont formées dans des conditions classiques de synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) (*J. Med. Chem.* (1992), **35** (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)).

Schéma 3 :

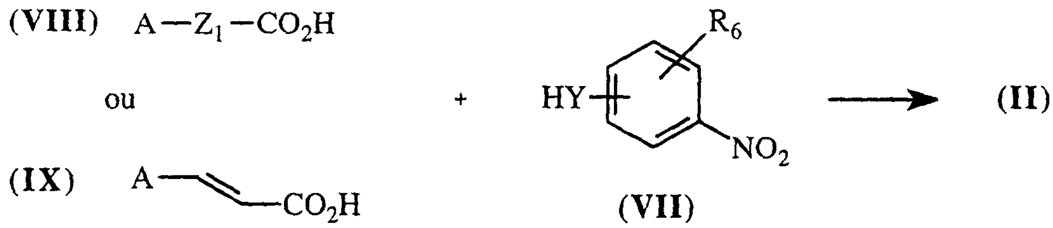

(VIII) ou (IX) (VII) (II)

Lorsque :

X = $-Z_1-NR_3-CO-$
et Y = $-Z_2-Q-$

Les carboxamides de formule générale **(II)** dans lesquels A, X, Y et $R_6$ sont tels que définis ci-dessus, peuvent également être préparés, schéma 4, par condensation peptidique d'une amine de formule générale **(X)** avec un acide commercial de formule générale **(XI).** Lorsque X = $-NR_3-CO-$ et $R_3$ = H, les composés de formule générale **(X)** sont des anilines qui sont obtenues par hydrogénation, en présence d'une quantité catalytique de Pd/C, des dérivés nitrobenzène correspondants, eux-mêmes synthétisés selon une méthode décrite dans la littérature (*J. Org. Chem.* (1968), **33** (1), 223-226). Lorsque X ≠ $-NR_3-CO-$ et $R_3$ est un radical alkyle linéaire ou ramifié ayant de 1 à 6 atomes de carbone, les monoalkylamines peuvent être obtenues selon un procédé décrit dans la littérature (brevets U.S. 3,208,859 et U. S. 2,962,531). Les acides carboxyliques de formule générale **(XI),** non commerciaux, sont accessibles à partir de méthodes décrites dans la littérature (*Acta Chem. Scand.* (1983), **37,** 911-916; *Synth. Commun.* (1986), **16** (4), 479-483 ; *Phophorus, Sulfur Silicon Relat. Elem.* (1991), **62,** 269-273).

Schéma 4 :

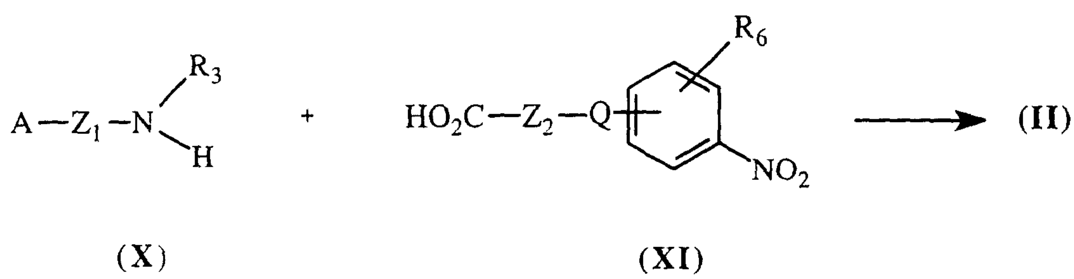

(X) (XI) (II)

Lorsque :

X = $-Z_1-NR_3-CO-$
et Y = $-NH-Z_2-Q-$, $-NH-CO-Z_2-Q-$ avec Q = $O-Z_3-$, $R_3-N-Z_3-$ ou $S-Z_3-$,

Les urées de formule générale **(II)**, schéma 5, dans lesquelles A, X, Y et $R_6$ sont tels que définis ci-dessus, sont préparées par addition d'une amine de formule générale (X) sur un isocyanate de formule générale **(XII), (XIII)** ou **(XIV)** dans un solvant tel que le chloroforme à 20°C. La synthèse des isocyanates de formule générale **(XII)** non commerciaux est décrite dans la littérature *(J. Med. Chem.* (1992), **35** (21), 3745-3754). Les urées halogénés intermédiaires **(XV)** et **(XVII)** sont ensuite substituées par un dérivé de formule générale **(XVI),** dans lequel Q représente $O-Z_3-$, $R_3-N-Z_3-$ ou $S-Z_3-$, en présence d'une base telle que, par exemple, $K_2CO_3$ ou NaH dans un solvant aprotique tel que le THF ou le DMF pour conduire finalement aux urées de formule générale **(II).**

Schéma 5 :

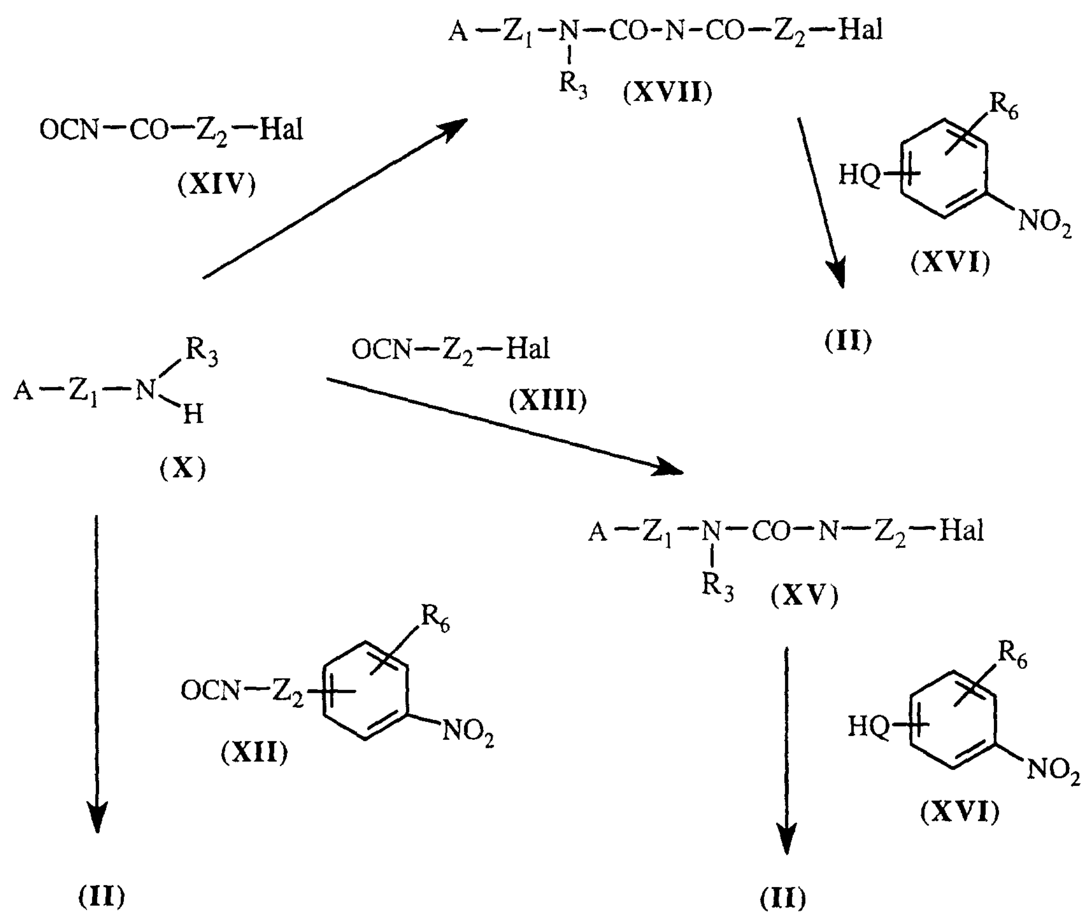

Lorsque :

X = $-Z_1$-NH-CO-
et Y = pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$, $-NR_3-O-Z_2-$

Les urées de formule générale **(II),** schéma 6, dans lesquelles A, X, Y et $R_6$ sont tels que définis ci-dessus, sont préparées par l'addition d'une amine de formule générale **(VII),** précédemment décrite, sur un isocyanate de formule général **(XVIII)** en présence d'une base telle que la diisopropyléthylamine.

Les isocyanates de formule générale **(XVIII)** sont synthétisés à partir des amines primaires de formule générale **(X),** précédemment décrites, de triphosgène et d'une amine tertiaire (*J. Org. Chem.* (1994), **59** (7), 1937-1938).

Les amines de formule générale **(VII)** dans lesquelles Y ≠ -NH-O- sont préparées selon une méthode décrite dans la littérature (*J. Org. Chem.* (1984), **49** (8), 1348-1352).

Schéma 6 :

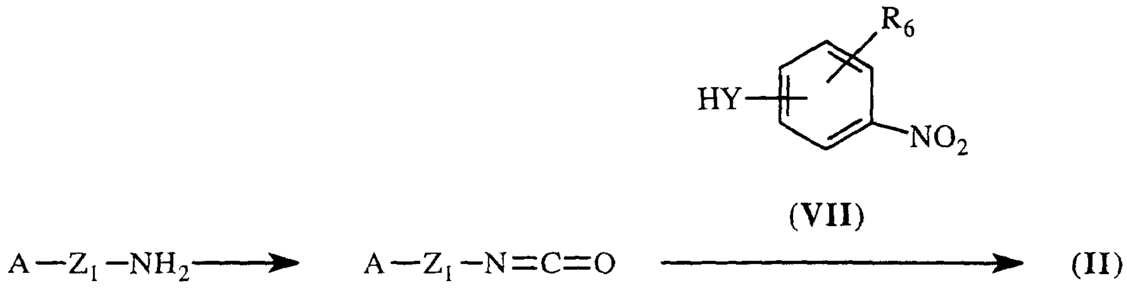

(VII)

(II)

(X)

(XVIII)

Lorsque :

X = $-Z_1-NR_3-CO-$
et Y = $-NR_3-SO_2-NR_3-Z_2-$

Les aminosulfonylurées de formule générale **(II)**, schéma 7, dans lesquelles A, X, Y et R6 sont tels que définis ci-dessus, sont préparées par addition des amines de formule générale (X), précédemment décrites, sur le chlorosulfonylisocyanate (*J. Med. Chem.* (1996), **39** (6), 1243-1252). La chlorosulfonylurée intermédiaire **(XIX)** est ensuite condensée sur les amines de formule générale **(VII),** précédemment décrites, pour conduire aux aminosulfonylurées de formule générale **(II)** qui peuvent éventuellement être alkylées par un dérivé halogéné en présence d'une base telle que, par exemple, NaH pour conduire aux dérivés de formule générale **(II)**.

Schéma 7 :

(X)

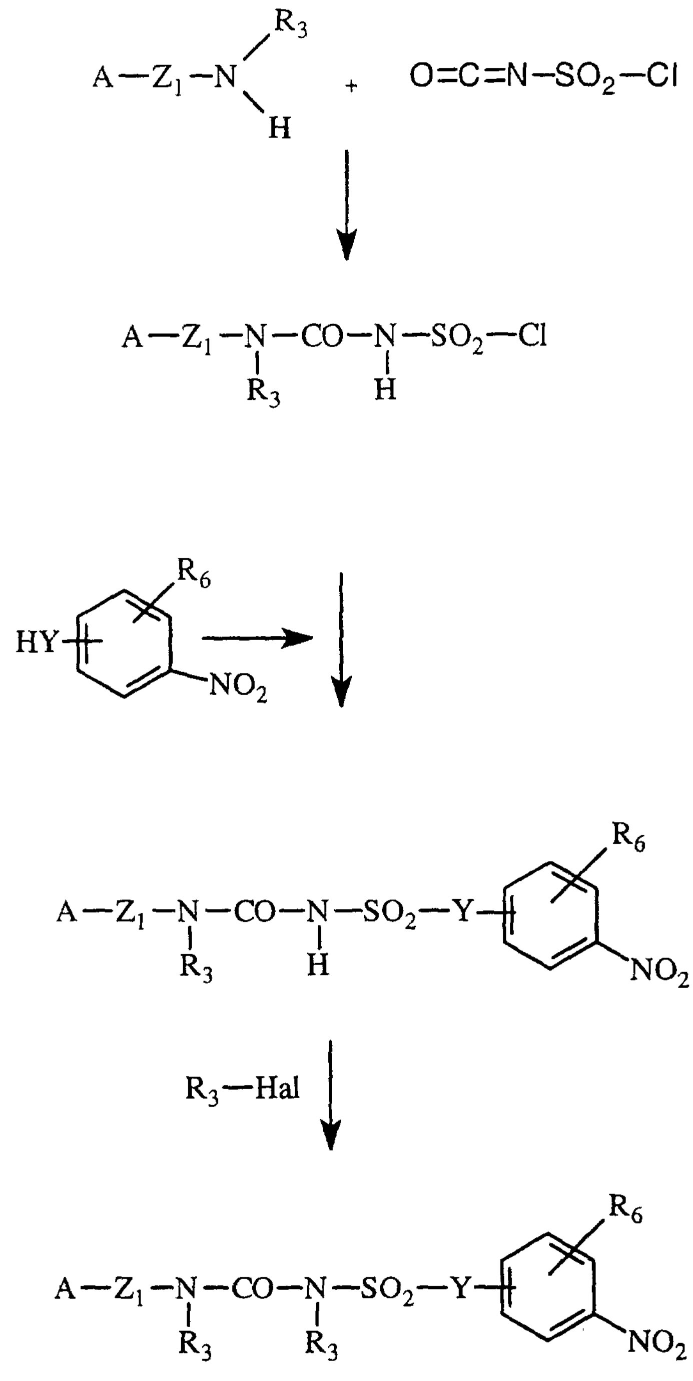


(XIX)

(VII)

(II)

(II)

Lorsque :

X = $-Z_1-NR_3-SO_2-$
et Y = $-Z_2-Q-$, avec Q = $O-Z_3-$, $R_3-N-Z_3-$ ou $S-Z_3-$,

Les sulfonamides de formule générale **(II),** schéma 8, dans lesquels A, X, Y et $R_6$ sont tels que définis ci-dessus, sont préparés par addition des amines de formule générale **(X)**, précédemment décrites, sur des chlorures de halogénoalkylsulfonyle de formule générale **(XX).** Les halogénoalkylsulfonamides de formule générale **(XXI),** intermédiairement obtenus, sont ensuite condensés sur un alcool, une amine ou un thiol de formule générale **(XVI)** en présence d'une base telle que, par exemple, $K_2CO_3$ ou NaH, dans un solvant polaire tel que, par exemple, l'acétonitrile ou le DMF.

Schéma 8 :

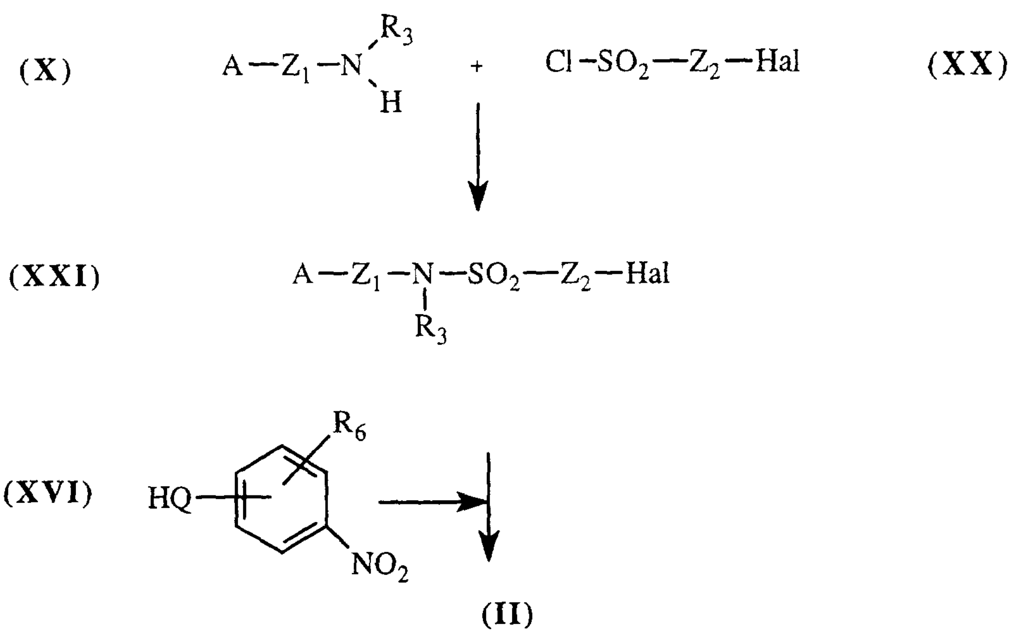

(X) (XX) (XXI) (XVI) (II)

Lorsque :

X = $-Z_1-NR_3-SO_2-$
et Y = $-NR_3-Z_2-Q-$

Les sulfamides de formule générale **(II),** schéma 9, dans lesquels A, X, Y et $R_6$ sont tels que définis ci-dessus sont préparés en trois étapes à partir des amines de formule générale **(X)** et du chlorosulfonylisocyanate. La réaction d'un alcool, tel que tBuOH, sur la fonction isocyanate du chlorosulfonylisocyanate (*Tetrahedron Lett*. (1991), **32** (45), 6545-6546) conduit à un intermédiaire de type chlorosulfonylcarbamate, lequel réagit en présence d'une amine de formule générale **(X)** pour donner un dérivé de type carboxylsulfamide de formule générale **(XXII)**. Le traitement de cet intermédiaire en milieu acide fort permet d'obtenir le dérivé sulfamide de formule générale **(XXIII).** L'alkylation des composés de formule générale **(XXIII)** par les dérivés halogénés de formule générale **(XXIV)** en présence d'une base telle que, par exemple, NaH dans un solvant aprotique polaire permet d'obtenir les dérivés sulfamides de formule générale **(II).**

Schéma 9 :

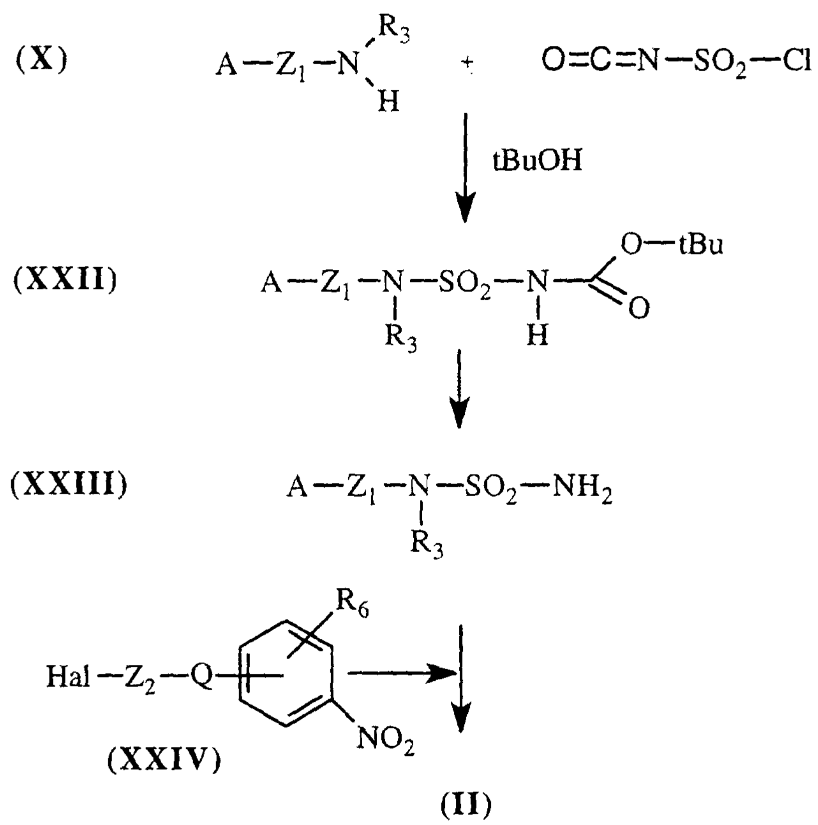


(X)

(XXII)

(XXIII)

(XXIV)

(II)

Lorsque :

X = $-Z_1-NR_3-CO-$
et Y = $-O-Z_2-Q-$

Les carbamates de formule générale **(II),** schéma 10, dans lesquels A, X, Y et $R_6$ sont tels que définis ci-dessus, sont préparés par réaction des amines de formule générale **(X)**, précédemment décrites, avec des dérivés chloroformiates de formule générale **(XXV)** préparés selon une méthode décrite dans la littérature (*Tetrahedron Lett.* (1993), **34** (44), 7129-7132).

Schéma 10 :

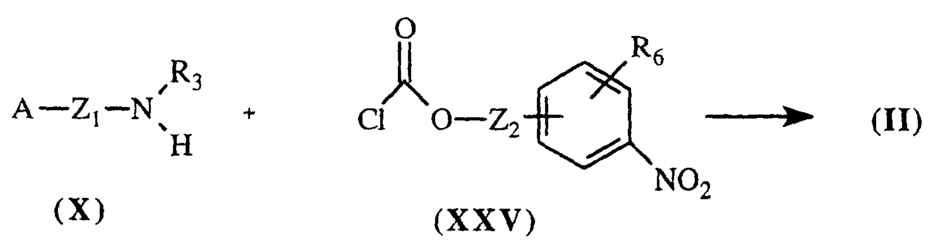

(X)

(XXV)

(II)

Lorsque :

X = $-Z_1-CO-$, $-CH=CH-CO-$
et Y = $-O-Z_2-Q-$

Les esters de formule générale **(II),** schéma 11, dans lesquels A, X, Y et $R_6$ sont tels que définis ci-dessus, sont préparés par réaction des acides de formule générale **(VIII)** ou **(IX)** et des alcools de formule générale **(XXVI)** en présence de dicyclohexylcarbodiimide et d'une quantité catalytique de 4-diméthylaminopyridine dans un solvant tel que, par exemple, le THF ou le DMF à 20° C.

Schéma 11 :

**(VIII)**

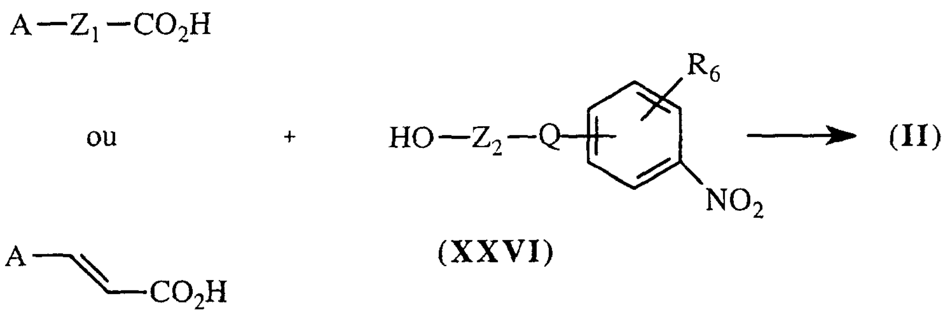

ou

**(IX)**

**(XXVI)**

**(II)**

Lorsque :

X = $-Z_1-$
et Y = $-O-CO-Z_2-Q-$

Les esters de formule générale **(II),** schéma 12, dans lesquels A, X, Y et $R_6$ sont tels que définis ci-dessus, peuvent également être préparés par réaction des acides de formule générale **(XI),** précédemment décrits, avec les alcools de formule générale **(V)** dans les conditions précédemment décrites.

Schéma 12 :

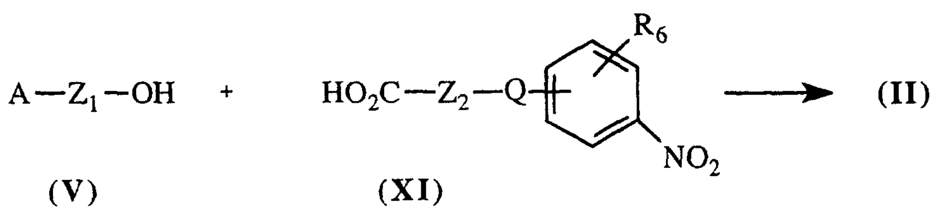

**(V)** **(XI)**

Lorsque :

X = $-Z_1-NR_3-CS-$
et Y = $-NH-Z_2-Q-$, pipérazine, homopipérazine, 2-méthylpipérazine, 2,5-diméthylpipérazine, 4-aminopipéridine, $-NR_3-Z_2-Q-$, $-NH-NH-Z_2-$, $-NR_3-O-Z_2-$

Les thiourées de formule générale **(II)** dans lesquelles A, X, Y et $R_6$ sont tels que définis ci-dessus, sont préparées à partir des urées précédemment décrites à l'aide du réactif de Lawesson, en suivant un protocole expérimental décrit dans la littérature (*J. Med. Chem.* (1995), **38** (18), 3558-3565).

Lorsque :

X représente une liaison
Y = $-O-Z_2-Q-$, $-S-Z_2-Q-$

et Q = -HN-

Les étheroxydes ou thioétheroxydes de formule générale **(II),** schéma 13, dans lesquels A, X, Y et $R_6$ sont tels que définis ci-dessus sont préparés à partir des dihydroquinones de formule générale **(XXVII)** (*J. Chem. Soc., Perkin Trans. I*, (1981), 303-306) ou des thiophénols de formule générale **(XXVIII)** (*Bio. Med. Chem. Letters*, (1993), **3** (12), 2827-2830) et d'un électrophile **($E^+$)** tel que , par exemple, le bromoacétonitrile ou la 4-nitrophényloxazolinone, en présence de $K_2CO_3$ (*J. Heterocyclic Chem.*, (1994), **31,** 1439-1443). Les nitriles doivent être réduits (hydrure de lithium ou hydrogénation catalytique) pour conduire aux intermédiaires de formule générale **(XXIX)** ou **(XXX).** L'ouverture des nitrophényloxazolinones, accessibles par réaction des nitroanilines correspondantes avec le chloroéthylchloroformiate comme décrit dans la littérature (*J. Am. Chem. Soc.*,(1953), **75,** 4596), par les phénols ou thiophénols conduit directement aux composés de formule générale **(XXIX)** ou **(XXX)** qui sont ensuite condensés sur le fluoronitrobenzène pour conduire aux intermédiaires de formule générale **(II).**

<u>Schéma 13</u> :

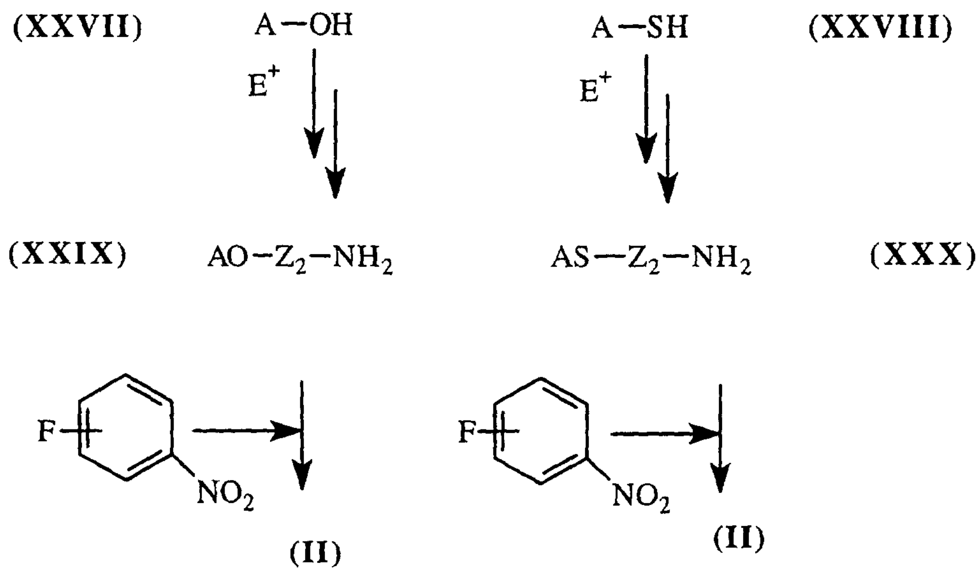

(XXVII) (XXVIII) (XXIX) (XXX) (II) (II)

<u>Lorsque</u> :

X représente -$Z_1$-CO- ou -CH=CH-CO-
Y = -$NR_3$-CO-Q-
et Q = $R_3$-N-$Z_3$

Les acylurées de formule générale **(II),** schéma 14, dans lesquelles A, X, Y et $R_6$ sont tels que définis ci-dessus sont préparées par condensation des acides de formule générale **(VIII)** ou **(IX),** schéma 3, et des urées de formule générale **(XXXI)** en présence d'un agent de couplage classiquement utilisé en synthèse peptidique, comme décrit précédemment, dans un solvant tel que, par exemple, le dichlorométhane ou le DMF. Les urées de formule générale **(XXXI)** sont accessibles à partir des isocyanates de formule générale **(XII),** schéma 5, selon une méthode de la littérature (*J. Chem. Soc.*, *Perkin Trans. 1*, (1985), (1), 75-79).

Schéma 14 :

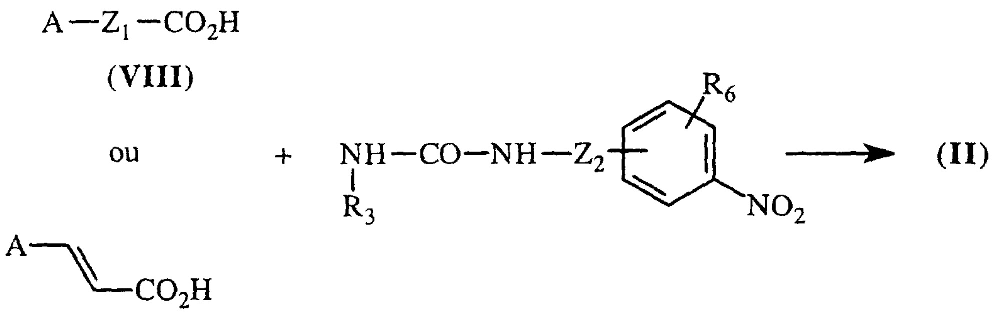

(VIII)

ou

+

(II)

(IX)

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

**Exemples :**

**Exemple 1 : chlorhydrate de 3,5-bis-(1,1-diméthyléthyl)-4-hydroxy-N-{4-[(2-thiényl (imino)méthyl)amino]phényl}-benzamide : 1**

**1.1) 3,5-bis-(1,1-diméthyléthyl)-4-hydroxy-N-(4-nitrophényl)-benzamide :**

Dans un ballon de 250 ml contenant 20 ml de THF, on introduit 1,38 g (10 mmoles) de 4-nitroaniline, 2,5 g (10 mmoles) d'acide 3,5-di-*tert*-butyl-4-hydroxybenzoïque et 2,26 g (11 mmoles) de dicyclohexylcarbodiimide. Le mélange réactionnel est agité pendant 15 heures à température ambiante, le précipité apparu est filtré et rincé avec de l'acétate d'éthyle. Après concentration de la solution sous pression réduite, le résidu est dilué dans 20 ml d'acétate d'éthyle et l'insoluble est filtré. Le solvant est éliminé sous vide et le résidu est précipité dans de l'éther diéthylique. Le solide est récupéré par filtration, rincé abondamment à l'éther diéthylique pour obtenir une poudre blanche avec un rendement de 65 %. Point de fusion : 277-278 °C.

RMN $^1H$ (100 MHz, DMSO d6, $\delta$) : 10,72 (s, 1H, CONH); 8,30 (m, 4H, Ph-$NO_2$) ; 7,80 (s, 2H, Ph); 1,60 (s, 18H, 2x tBu).

**1.2) 3,5-bis-(1,1-diméthyléthyl)-4-hydroxy-N-(4-aminophényl)-benzamide :**

Dans une bouteille de Parr de 250 ml, 2,4 g (6.5 mmoles) de 3,5-bis-(1,1-diméthyléthyl)-4-hydroxy-N-(4-nitrophényl)-benzamide sont dissous dans 50 ml d'un mélange éthanol absolu/dichlorométhane (1/1) en présence de Pd/C à 10 %. Le mélange est agité sous 20 PSI d'hydrogène, à 30 °C, pendant une heure. Après filtration sur célite, le filtrat est concentré sous vide. Le résidu d'évaporation est repris par 25 ml d'une solution 1M de HCl. Le précipité formé est filtré et rincé par 50 ml d'éther diéthylique suivi de 50 ml d'acétate d'éthyle. L'amine est libérée de son sel par agitation dans un mélange de 50 ml d'acétate d'éthyle et 50 ml de NaOH 1M. Après décantation, la phase organique est lavée par 25 ml de NaOH 1M et 25 ml de saumure. La solution organique est séchée sur sulfate de sodium, filtrée, rincée et concentrée à sec sous pression réduite pour conduire à 1,09 g (49 %) d'une poudre blanche. Point de fusion : 220-221 °C.

RMN $^1H$ (100 MHz, DMSO d6, $\delta$) : 9,80 (s, 1H, CONH); 7,78 (s, 2H, Ph) ; 7,05 (m, 4H, Ph-$NH_2$) ; 5.02 (s, 2H, OH) ; 1,60 (s, 18H, 2x tBu).

**1.3) chlorhydrate de 3,5-bis-(1,1-diméthyléthyl)-4-hydroxy-N-{4-[(2-thiényl (imino)méthyl)-amino]phényl}-benzamide : 1**

Dans un ballon de 100 ml contenant une solution de 1,05 g (3,08 mmoles) de 3,5-bis-(1,1-diméthyléthyl)-4-hydroxy-N-(4-aminophényl)-benzamide dans 20 ml de 2-propanol, on ajoute 880 mg (3,08 mmoles) de iodhydrate de S-méthyl-2-thiophènethiocarboximide (Ann. *Chim.* (1962), 7, 303-337). Après chauffage à 50° C pendant 15 heures, le mélange réactionnel est concentré à sec sous vide. Le résidu est repris par 50 ml d'acétate d'éthyle et 50 ml d'une solution saturée de carbonate de sodium. Après décantation, la phase organique est lavée successivement par 50 ml d'une solution saturée de carbonate de sodium, 50 ml d'eau et 50 ml de saumure. La solution organique est séchée sur sulfate de sodium, filtrée et évaporée sous pression réduite. Les cristaux obtenus sont repris par de l'éther diéthylique, filtrés et lavés successivement par de l'acétate d'éthyle et de l'acétone. On obtient 0,77 g de base avec un rendement de 58 %.

On prépare le chlorhydrate à partir de 0,77 g (1,71 mmole) de base dissoute dans 60 ml de méthanol et salifiée en présence de 3,42 ml (3,42 mmoles) d'une solution molaire de HCl dans l'éther diéthylique anhydre. Après une demi-heure d'agitation à température ambiante, le solvant est évaporé sous vide et le résidu précipité en présence d'éther diéthylique. Les cristaux obtenus sont filtrés et rincés abondamment à l'éther diéthylique pour finalement obtenir après séchage 0,65 g (43 %) d'une poudre jaune pâle. Point de fusion : 290-291 °C.
RMN $^1H$ (400 MHz, DMSO d6, $\delta$) : 11,55 (s, 1H, $NH^+$) ; 10,40 (s, 1H, CONH) ; 9,83 (s, 1H, $NH^+$) ; 8,85 (s, 1H, $NH^+$) ; 8,21 (m, 2H, thiophène) ; 7,70 (s, 2H, Ph) ; 7,67 (m, 4H, Ph-NH) ; 7,60 (s, 1H, OH) ; 7,40 (m, 1H, thiophène) ; 1,42 (s, 18H, 2x tBu).
IR : $\nu_{OH}$ : 3624 $cm^{-1}$, 3430 $cm^{-1}$; $\nu_{C=O}$ (amide) : 1653 $cm^{-1}$; $\nu_{C=N}$ (amidine) : 1587 $cm^{-1}$.

**Exemple 2 : chlorhydrate de 3,5-bis-(1,1-diméthyléthyl)-4-hydroxy-N-{4-[[(2-thiényl-(imino)méthyl)amino]phényl]méthyl}-benzamide : 2**

**2.1) 3,5-bis-(1,1-diméthyléthyl)-4-hydroxy-N-[(4-nitrophényl)méthyl]-benzamide :**

Dans un ballon de 250 ml contenant 25 ml de THF, on introduit 1,88 g (10 mmoles) de chlorhydrate de p-nitrobenzylamine, 2,5 g (10 mmoles) d'acide 3,5-di-tert-butyl-4-hydroxybenzoïque, 1,38 ml (10 mmoles) de triéthylamine et 2,26 g (11 mmoles) de dicyclohexylcarbodiimide. Le mélange réactionnel est agité pendant 15 heures à température ambiante, le précipité apparu est filtré et rincé avec le minimum d'acétate d'éthyle. Après concentration de la solution sous pression réduite, le résidu est précipité dans un mélange d'acétate d'éthyle/éther diéthylique (1/4) et filtré. Les cristaux sont lavés abondamment à l'éther diéthylique pour finalement obtenir, après séchage, une poudre blanche avec un rendement de 74 % (2,85 g). Point de fusion : 230-231 °C.
RMN $^1H$ (100 MHz, $CDCl_3$, $\delta$) : 7,85 (m, 4H, Ph-$NO_2$); 7,69 (s, 2H, Ph); 6,82 (m, 1H, NHCO); 5,67 (s, 1H, OH); 4,75 (d, 2H, $CH_2$-NHCO, J = 6,5 Hz); 1,49 (s, 18H, 2x tBu).

**2.2) 3,5-bis-(1,1-diméthyléthyl)-4-hydroxy-N-[(4-aminophényl)méthyl]-benzamide :**

Dans une bouteille de Parr de 250 ml, 2,85 g (7,4 mmoles) de 3,5-bis-(1,1-diméthyléthyl)-4-hydroxy-N-[(4-nitrophényl)méthyl]-benzamide sont dissous dans 30 ml d'un mélange éthanol absolu/dichlorométhane (1/1) en présence de Pd/C à 10 %. Le mélange est agité sous 20 PSI d'hydrogène, à 30° C, pendant une heure. Après filtration sur célite, le filtrat est concentré sous vide. Le résidu d'évaporation cristallise spontanément. On le laisse reposer une nuit, on filtre les cristaux et on les rince avec un mélange d'éther diéthylique (45 ml) et d'acétone (5 ml). On obtient 1,63 g (62 %) d'une poudre blanche. Point de fusion : 188-189° C.
RMN $^1H$ (100 MHz, $CDCl_3$, $\delta$) : 7,62 (s, 2H, Ph); 6,95 (m, 4H, Ph-$NH_2$); 6,20 (m, 1H, NHCO); 5,58 (s, 1H, OH); 4,50 (d, 2H, $CH_2$-NHCO, J = 6,5 Hz); 3,70 (s large, 2H, $NH_2$); 1,47 (s, 18H, 2x tBu).

**2.3) chlorhydrate de 3,5-bis-(1,1-diméthyléthyl)-4-hydroxy-N-{4-[[(2-thiényl-(imino)méthyl)-amino]phényl]méthyl}-benzamide : 2**

Le protocole expérimental utilisé est le même que celui décrit pour le composé **1**, le 3,5-bis-(1,1-diméthyléthyl)-4-hydroxy-N-[(4-aminophényl)méthyl]-benzamide remplaçant le 3,5-bis-(1,1-diméthyléthyl)-4-hydroxy-N-(4-aminophényl)-benzamide. Après salification par une solution molaire de HCl dans l'éther diéthylique anhydre, on obtient une poudre blanche avec un rendement de 56 %. Point de fusion : 218-219 °C.
RMN $^1H$ (400 MHz, DMSO d6, $\delta$) : 11,60 (s, 1H, $NH^+$); 9,83 (s, 1H, $NH^+$); 9,02 (s, 1H, CONH); 8,90 (s, 1H, $NH^+$); 8,18 (m, 2H, thiophène); 7,70 (s, 2H, Ph); 7,42 (m, 6H, thiophène, Ph-NH, OH); 4,50 (d, 2H, $CH_2$-NHCO, J = 5,7 Hz); 1,40 (s, 18H, 2x tBu).

IR : $\nu_{OH}$ : 3624 $cm^{-1}$, 3424 $cm^{-1}$; $\nu_{C=O}$ (amide) : 1644 $cm^{-1}$ ; $\nu_{C=N}$ (amidine) : 1568 $cm^{-1}$.

**Exemple 3 : 4-acétoxy-3,5-diméthoxy-N-{4-[[(2-thiényl(imino)méthyl)amino]phényl]méthyl}-benzamide : 3**

**3.1) Acide 4-acétoxy-3,5-diméthoxy-benzoïque :**

Dans un ballon de 100 ml, sous atmosphère d'azote, on dissout 1,50 g (7,57 mmoles) d'acide syringique dans 15 ml de pyridine sèche. On ajoute goutte à goutte 0,86 ml (9,08 mmoles) d'anhydride acétique et le mélange est agité à température ambiante pendant 18 heures. La pyridine est évaporée sous pression réduite, le résidu est repris par 25 ml de dichlorométhane et lavé par 10 ml d'une solution molaire d'HCl puis par 2 x 10 ml d'eau. La phase organique est séchée sur sulfate de sodium, filtrée et évaporée sous vide. On obtient 1,72 g (95 %) d'une poudre beige. Point de fusion : 181-183 °C.
RMN $^1H$ (100 MHz, $CDCl_3$, δ) : 8,15 (s, 1H, $CO_2H$) ; 7,40 (s, 2H, Ph) ; 3,90 (s, 6H, 2x $OCH_3$) ; 2,40 (s, 3H, $CH_3$).

**3.2) 4-acétoxy-3,5-diméthoxy-N-[(4-nitrophényl)méthyl]-benzamide :**

Le protocole expérimental utilisé est le même que celui décrit pour l'intermédiaire 2.1, l'acide 4-acétoxy-3,5-diméthoxy-benzoïque remplaçant l'acide 3,5-di-*tert*-butyl-4-hydroxy-benzoïque. On obtient une huile incolore avec un rendement de 28 %.
RMN $^1H$ (100 MHz, DMSO d6, δ) : 9,26 (t, 1H, NHCO, J = 6,0 Hz) ; 7,91 (m, 4H, Ph-$NO_2$) ; 7,31 (s, 2H, Ph) ; 4,65 (d, 2H, $CH_2$, J = 6,0 Hz) ; 3,83 (s, 6H, 2x $OCH_3$) ; 2,28 (s, 3H, $CH_3$).

**3.3) 4-acétoxy-3,5-diméthoxy-N-[(4-aminophényl)méthyl]-benzamide :**

Le protocole expérimental utilisé est le même que celui décrit pour l'intermédiaire 2.2, le 4-acétoxy-3,5-diméthoxy-N-[(4-nitrophényl)méthyl]-benzamide remplaçant le 3,5-bis-(1,1-diméthyléthyl)-4-hydroxy-N-[(4-nitrophényl)méthyl]-benzamide. On obtient une huile incolore avec un rendement de 82 %. Le produit est utilisé directement dans l'étape suivante sans purification supplémentaire.

**3.4) 4-acétoxy-3,5-diméthoxy-N-{4-[[(2-thiényl (imino)méthyl)amino]phényl]méthyl}-benzamide : 3**

Le protocole expérimental utilisé est le même que celui décrit pour le composé **1**, le 4-acétoxy-3,5-diméthoxy-N-[(4-aminophényl)méthyl]-benzamide remplaçant le 3,5-bis-(1,1-diméthyléthyl)-4-hydroxy-N-(4-aminophényl)-benzamide. On obtient la base 3 sous forme d'une poudre beige avec un rendement de 65 %. Point de fusion : 47-48 °C.
RMN $^1H$ (400 MHz, DMSO d6, δ) : 9,08 (s large, 1H, CONH) ; 7,75 (m, 1H, thiophène) ; 7,62 (m, 1H, thiophène) ; 7,30 (s, 2H, Ph) ; 7,10 (m, 1H, thiophène) ; 7,07 (m, 4H, Ph-N) ; 6,48 (s large, 2H, $NH_2$) ; 4,50 (d, 2H, $CH_2$, J = 4,6 Hz) ; 3,80 (s, 6H, 2x $OCH_3$) ; 2,30 (s, 3H, $CH_3$).
IR : $\nu_{C=O}$ (ester) : 1760 $cm^{-1}$ ; $\nu_{c=o}$ (amide) : 1630 $cm^{-1}$ ; $\nu_{C=N}$ (amidine) : 1540 $cm^{-1}$.

**Exemple 4 : 3,5-diméthoxy-4-hydroxy-N-{4-[[(2-thiényl(imino)méthyl)amino]phényl]méthyl}-benzamide : 4**

Dans un ballon de 50 ml, on ajoute goutte à goutte 1 ml (2 mmoles) d'acide chlorhydrique 2N à une solution de 0,59 g (1 mmole) du composé **3** dans 5 ml d'éthanol. Le mélange réactionnel est agité pendant 18 heures à 50 °C. Les solvants sont évaporés à sec, le résidu est repris par du dichlorométhane (5 ml) et lavé par une solution de soude molaire (3 x 5 ml). Après séchage de la phase organique, filtration et concentration à sec, l'huile obtenue est purifiée par chromatographie sur une colonne de gel de silice (éluant : dichlorométhane/méthanol : 9/1). Les fractions pures sont collectées et après évaporation sous vide on obtient une poudre beige avec un rendement de 60%. Point de fusion : 55-58° C.
RMN $^1H$ (400 MHz, DMSO d6, δ) : 8,92 (s, 1H, OH) ; 8,84 (m, 1H, CONH) ; 7,75 (m, 1H, thiophène); 7,63 (m, 1H, thiophène) ; 7,26 (s, 2H, Ph); 7,10 (m, 1H, thiophène) ; 7,05 (m, 4H, Ph-N); 6,50 (s, 2H, $NH_2$) ; 4,45 (d, 2H, $CH_2$, J = 5,7 Hz) ; 3,81 (s, 6H, 2x $OCH_3$).
IR: $\nu_{OH}$ : 3300 $cm^{-1}$ ; $\nu_{C=O}$ (amide) : 1630 $cm^{-1}$ ; $\nu_{C=N}$ (amidine) : 1590 $cm^{-1}$.

**Exemple 5 : iodhydrate de 3,5-bis-(1,1-diméthyléthyl)-4-hydroxy-N-{4-[2-[(2-thiényl-(imino)méthyl)amino]phényl]éthyl}-benzamide : 5**

**5.1) 3,5-bis-(1,1-diméthyléthyl)-4-hydroxy-N-[2-(4-nitrophényl)éthyl]-benzamide :**

Dans un ballon de 100 ml contenant 20 ml de THF, on introduit 2,02 g (10 mmoles) de chlorhydrate de 4-nitrophénétylamine, 2,5 g (10 mmoles) d'acide 3,5-di-*tert*-butyl-4-hydroxy-benzoïque, 1,38 ml (10 mmoles) de triéthylamine et 2,26 g (11 mmoles) de dicyclohexylcarbodiimide. Le mélange réactionnel est agité pendant 15 heures à température ambiante, le précipité apparu est filtré et rincé avec de l'acétate d'éthyle. Après concentration du filtrat sous pression réduite, le résidu est précipité dans de l'éther diéthylique. Le solide est récupéré par filtration et rincé par de l'éther diéthylique. On obtient une poudre blanche avec un rendement de 73 %. Point de fusion : 204-206 °C.
RMN $^1H$ (100 MHz, $CDCl_3$, δ) : 7,52 (s, 2H, Ph) ; 6,85 (m, 4H, $Ph\text{-}NO_2$) ; 6,02 (m, 1H, NHCO) ; 3,62 (m, 2H, $\underline{CH_2}$-NHCO) ; 2,82 (m, 2H, $\underline{CH_2}\text{-}Ph\text{-}NO_2$) ; 1,48 (s, 18H, 2x tBu).

**5.2) 3,5-bis-(1,1-diméthyléthyl)-4-hydroxy-N-[2-(4-aminophényl)éthyl]-benzamide :**

Le protocole expérimental utilisé est le même que celui décrit pour l'intermédiaire 2.2, le 3,5-bis-(1,1-diméthyléthyl)-4-hydroxy-N-[2-(4-nitrophényl)éthyl]-benzamide remplaçant le 3,5-bis-(1,1-diméthyléthyl)-4-hydroxy-N-[(4-nitrophényl)méthyl]-benzamide. On obtient une poudre blanche avec un rendement de 76%. Point de fusion : 193-195 °C.
RMN $^1H$ (100 MHz, $CDCl_3$, δ) : 7,80 (m, 4H, $Ph\text{-}NH_2$); 7,55 (s, 2H, Ph); 6,10 (m, 1H, NHCO); 5,55 (s, 1H, OH); 3,75 (m, 2H, $\underline{CH_2}$-NHCO); 3,10 (m, 2H, $\underline{CH_2}\text{-}Ph\text{-}NH_2$); 1,50 (s, 18H, 2x tBu).

**5.3) iodhydrate de 3,5-bis-(1,1-diméthyléthyl)-4-hydroxy-N-{4-[2-[(2-thiényl(imino)méthyl)-amino]phényl] éthyl}-benzamide : 5**

Dans un ballon de 50 ml contenant 1,01 g (2,74 mmoles) de 3,5-bis-(1,1-diméthyléthyl)-4-hydroxy-N-[2-(4-aminophényl)éthyl]-benzamide dissous dans 20 ml de 2-propanol, on ajoute 0,78 g (2,74 mmoles) de iodhydrate de S-méthyl-2-thiophène-thiocarboximide (Ann. *Chim.* (1962), **7,** 303-337). Le mélange réactionnel est chauffé à 40 °C pendant 4 heures. Le solvant est évaporé sous vide et le résidu est précipité en présence de 50 ml d'un mélange eau/acétate d'éthyle (1/1). Les cristaux formés sont filtrés et lavés successivement par de l'acétate d'éthyle et de l'éther diéthylique. On obtient une poudre jaune pâle après séchage, avec un rendement de 68%. Point de fusion : 185-186 °C.
RMN $^1H$ (400 MHz, DMSO d6, δ) : 9,80 (s, 1H, $NH^+$); 8,88 (s, 1H, $NH^+$); 8,40 (s, 1H, CONH); 8,12 (m, 2H, thiophène); 7,60 (s, 2H, Ph); 7,42 (m, 6H, thiophène, $\underline{Ph}$-NH, OH); 3,52 (d, 2H, $\underline{CH_2}$-NHCO, J = 5,9 Hz); 2,90 (m, 2H, $\underline{CH_2}$-Ph-NH); 1,40 (s, 18H, 2x tBu).
IR : $\nu_{OH}$ : 3624 $cm^{-1}$, 3423 $cm^{-1}$ ; $\nu_{C=O}$ (amide) : 1636 $cm^{-1}$ ; $\nu_{C=N}$ (amidine) : 1569 $cm^{-1}$.

**Exemple 6 : fumarate de 4-acétoxy-3,5-diméthoxy-N-{4-[2-[(2-thiényl-(imino)méthyl)-amino]phényl]éthyl}-benzamide : 6**

**6.1) 4-acétoxy-3,5-diméthoxy-N-[2-(4-nitrophényl)éthyl]-benzamide :**

Le protocole expérimental utilisé est le même que celui décrit pour l'intermédiaire 5.1, l'acide 4-acétoxy-3,5-diméthoxy-benzoïque (intermédiaire 3.1) remplaçant l'acide 3,5-di-*tert*-butyl-4-hydroxy benzoïque. On obtient une huile incolore avec un rendement de 70 %. Le produit est utilisé directement dans l'étape suivante.

**6.2) 4-acétoxy-3,5-diméthoxy-N-[2-(4-aminophényl)éthyl]-benzamide :**

Le protocole expérimental utilisé est le même que celui décrit pour l'intermédiaire 2.2, le 4-acétoxy-3,5-diméthoxy-N-[2-(4-nitrophényl)éthyl]-benzamide remplaçant le 3,5-bis-(1,1-diméthyléthyl)-4-hydroxy-N-[(4-nitrophényl)méthyl]-benzamide. On obtient une huile incolore avec un rendement quantitatif. Le produit est utilisé directement dans l'étape suivante sans purification supplémentaire.

**6.3) fumarate de 4-acétoxy-3,5-diméthoxy-N-{4-[2-[(2-thiényl(imino)méthyl)amino]-phényl]éthyl}-benzamide : 6**

Le protocole expérimental utilisé pour obtenir la base libre est le même que celui décrit pour la synthèse du composé **1**, le 4-acétoxy-3,5-diméthoxy-N-[2-(4-aminophényl)éthyl]-benzamide remplaçant le 3,5-bis-(1,1-diméthyléthyl)-4-hydroxy-N-(4-aminophényl)-benzamide.

Le produit de la réaction est salifié en présence d'une quantité équimolaire d'acide fumarique dans l'éthanol à reflux. On obtient le composé **6** sous forme d'une poudre beige avec un rendement de 74%. Point de fusion : 178-180 °C.
RMN $^1H$ (400 MHz, DMSO d6, δ) : 8,60 (m, 1H, CONH); 7,75 (m, 1H, thiophène); 7,64 (d, 1H, thiophène, J = 5,0 Hz); 7,20 (s, 2H, Ph); 7,11 (t, 1H, thiophène, J = 9,0 Hz); 7,02 (m, 4H, Ph-N); 6,61 (s, 2H, CH=CH fumarate); 3,81 (s, 6H, 2x $OCH_3$); 3,50 (q, 2H, $CH_2$-N, J = 6,5 Hz); 2,82 (t, $\underline{CH_2}$-Ph, J = 7,0 Hz); 2,27 (s, 3H, $CH_3$).
IR : $\nu_{C=O}$ (ester) : 1750 $cm^{-1}$ ; $\nu_{C=O}$ (amide) : 1640 $cm^{-1}$ ; $\nu_{C=N}$ (amidine) : 1550 $cm^{-1}$.

**Exemple 7 : chlorhydrate de 3,5-diméthoxy-4-hydroxy-N-{4-[2-[(2-thiényl-(imino)méthyl)-amino]phényl]éthyl}-benzamide : 7**

Dans un ballon de 50 ml, on ajoute goutte à goutte 1,40 ml (2,80 mmoles) d'une solution d'acide chlorhydrique 2N à une solution de 0,64 g (1,37 mmoles) du composé **6** sous forme de base libre dans 5 ml d'éthanol. Le mélange réactionnel est agité pendant 18 heures à 50 °C. Les solvants sont évaporés à sec et le résidu d'évaporation est précipité dans un mélange de 5 ml d'une solution de soude 2N et 10 ml de dichlorométhane. Après filtration, le solide est repris par de l'éthanol chlorhydrique (4N). Un précipité léger est alors éliminé. Le solvant est évaporé sous pression réduite et le résidu repris dans de l'acétone. Le produit **7** précipite sous forme de chlorhydrate avec un rendement de 58 %. Point de fusion : 164-167 °C.
RMN $^1H$ (400 MHz, DMSO d6, δ) : 9,80 (s large, 1H, $NH^+$); 8,90 (s, 2H, $NH^+$, OH); 8,54 (m, 1H, CONH); 8,18 (s, 1H, thiophène); 8,16 (s, 1H, thiophène); 7,40 (m, 4H, Ph-N); 7,21 (s, 2H, Ph); 7,11 (m, 1H, thiophène); 3,81 (s, 6H, 2x $OCH_3$); 3,51 (q, 2H, $CH_2$-N, J = 7,0 Hz); 2,92 (t, $\underline{CH_2}$-Ph, J = 7,0 Hz).
IR: $\nu_{OH}$ : 3300 $cm^{-1}$; $\nu_{C=O}$ (amide) : 1620 $cm^{-1}$; $\nu_{C=N}$ (amidine): 1560 $cm^{-1}$.

**Exemple 8 : hémi-fumarate de 3,4,5-trihydroxy-N-{4-[2-[(2-thiényl(imino)méthyl)-amino]phényl]éthyl}-benzamide : 8**

**8.1) 3,4,5-trihydroxy-N-[2-(4-nitrophényl)éthyl]-benzamide :**

Dans un ballon de 100 ml contenant 30 ml de DMF anhydre, on introduit 2 g (11,5 mmoles) d'acide gallique, 2,5 g (11,5 mmoles) de chlorhydrate de 4-nitrophénétylamine, 1,8 g (11,5 mmoles) de 1-hydroxybenzotriazole hydraté, 2,25 g (11,5 mmoles) de chlorhydrate de 1-(3-diméthylaminopropyl)-3-éthylcarbodiimide et 3,3 ml (23 mmoles) de triéthylamine. La solution orangée obtenue est agitée, à 20 °C, pendant 20 heures et diluée dans un mélange de dichlorométhane (50 ml) et d'eau (30 ml). Après décantation, la phase organique est lavée par une solution molaire d'acide chlorhydrique (20 ml) et par de l'eau (3x20 ml) jusqu'à neutralité. Après séchage de la phase organique sur sulfate de magnésium, filtration et concentration sous vide, le résidu est purifié sur une colonne de gel de silice (éluant : dichlorométhane/méthanol : 9/1). On obtient le produit attendu sous forme d'une huile incolore avec un rendement de 42 % (1,57 g).
RMN $^1H$ (100 MHz, DMSO d6, δ) : 8,95 (m, 3H, 3x OH); 7,85 (m, 4H, Ph-$NO_2$); 6,80 (s, 2H, Ph); 3,36 (m, 2H, $CH_2$-N); 2,97 (t, 2H, $\underline{CH_2}$-Ph, J =6,0 Hz).

**8.2) 3,4,5-trihydroxy-N-[2-(4-aminophényl)éthyl]-benzamide :**

Le protocole expérimental utilisé est le même que celui décrit pour l'intermédiaire 2.2, le 3,4,5-trihydroxy-N-[2-(4-nitrophényl)éthyl]-benzamide remplaçant le 3,5-bis-(1,1-diméthyléthyl)-4-hydroxy-N-[(4-nitrophényl) méthyl]-benzamide. On obtient une poudre beige avec un rendement de 89 %. Point de fusion : 167-169 °C.
RMN $^1H$ (100 MHz, DMSO d6, δ) : 8,80 (m, 3H, OH); 8,07 (t, 1H, NHCO, J = 5,0 Hz); 6,81 (s, 2H, Ph); 6,68 (m, 4H, Ph-$NH_2$); 3,28 (m, 2H, $CH_2$-N); 2,60 (t, 2H, $\underline{CH_2}$-Ph, J =7,0 Hz).

**8.3) hémi-fumarate de 3,4,5-trihydroxy-N-{4-[2-[(2-thiényl(imino)méthyl)amino)-phényl]éthyl}-benzamide : 8**

Le protocole expérimental utilisé est le même que celui décrit pour le composé **1,** le 3,4,5-trihydroxy-N-[2-(4-aminophényl)éthyl]-benzamide remplaçant le 3,5-bis-(1,1-diméthyléthyl)-4-hydroxy-N-(4-aminophényl)-benzamide. On obtient la base **8** sous forme d'une poudre qui est salifiée, par chauffage à reflux de l'éthanol, en présence d'un équivalent d'acide fumarique. Le sel cristallise spontanément à 20° C. Après filtration et lavage par de l'éthanol on obtient le produit attendu sous forme d'une poudre beige avec un rendement de 53 %. Point de fusion : 245-246 °C.
RMN $^1H$ (400 MHz, DMSO d6, δ) : 8,85 (m, 3H, 3x OH); 8,14 (t, 1H, NHCO, J = 5,0 Hz); 7,73 (s, 1H, thiophène); 7,60 (d, 1H, thiophène, J = 5,0 Hz); 7,16 (s, 2H, Ph); 7,09 (t, 1H, thiophène, J = 4,0 Hz); 6,80 (m, 4H, Ph-N); 6,59 (s large, 2H, 1/2-CH=CH, NH); 3,41 (m, 3H, $CH_2$-$N^+$NH) ; 2,76 (t, 2H, $CH_2$, J = 7,5 Hz).

IR : $\nu_{OH}$ : 3300 $cm^{-1}$; $\nu_{C=O}$ (amide) : 1620 $cm^{-1}$; $\nu_{C=N}$ (amidine) : 1590 $cm^{-1}$.

**Exemple 9 : chlorhydrate de N-{4-[4-[3,5-bis-(1,1-diméthyléthyl)-4-hydroxybenzoyl]-1-pipérazinyl]phényl}-2-thiophènecarboximidamide : 9**

**9.1) 2,6-bis-(1,1-diméthyléthyl)-4-{[4-(4-nitrophényl)-1-pipérazinyl]-carbonyl}-phénol :**

Dans un ballon de 100 ml contenant 25 ml de DMF, on introduit 2,07 g (10 mmoles) de 1-(4-nitrophényl) pipérazine, 2,5 g (10 mmoles) d'acide 3,5-di-*tert*-butyl-4-hydroxybenzoïque et 2,26 g (11 mmoles) de dicyclohexylcarbodiimide. Le mélange réactionnel est agité pendant 15 heures à température ambiante, le précipité apparu est filtré et rincé avec de l'acétate d'éthyle. Après concentration du filtrat sous pression réduite, le résidu est dilué dans 20 ml d'acétate d'éthyle et un nouvel insoluble est éliminé par filtration. Le solvant est évaporé sous vide et le résidu est précipité dans de l'éther diéthylique. Le solide est filtré, rincé par 2x20 ml d'acétate d'éthyle pour obtenir une poudre jaune avec un rendement de 89 %. Point de fusion : 159.5-160.5 °C.
RMN $^1H$ (100 MHz, $CDCl_3$, δ) : 7,58 (m, 4H, Ph-$NO_2$); 7,30 (s, 2H, Ph); 5,50 (s, 1H, OH); 3,85 (m, 4H, pipérazine); 3,55 (m, 4H, pipérazine); 1,46 (s, 18H, 2x tBu).

**9.2) 2,6-bis-(1,1-diméthyléthyl)-4-{[4-(4-aminophényl)-1-pipérazinyl]-carbonyl}-phénol :**

Dans une bouteille de Parr de 250 ml, 2,19 g (5,0 mmoles) de l'intermédiaire 9.1 sont dissous dans 50 ml d'éthanol absolu en présence de Pd/C à 10 %. Le mélange est agité sous 20 PSI d'hydrogène, à 30 °C, pendant une heure. Après filtration sur célite, le filtrat est concentré sous vide. Le résidu d'évaporation est repris par 25 ml d'éther diéthylique, filtré et rincé par 2x20 ml d'éther diéthylique. On obtient une poudre rose pâle avec un rendement de 82%. Point de fusion : 221-222 °C.
RMN$^1H$ (100 MHz, $CDCl_3$, δ) : 7,30 (s, 2H, Ph); 6,75 (m, 4H, Ph-$NH_2$); 5,45 (s, 1H, OH); 3,80 (m, 4H, pipérazine); 3,10 (m, 4H, pipérazine); 1,49 (s, 18H, 2x tBu).

**9.3) chlorhydrate de N-{4-[4-[3,5-bis-(1,1-diméthyléthyl)-4-hydroxybenzoyl]-1-pipérazinyl]-phényl}-2-thiophènecarboximidamide : 9**

Le protocole expérimental utilisé est le même que celui décrit pour le composé **1,** le 2,6-bis-(1,1-diméthyléthyl)-4-{[4-(4-aminophényl)-1-pipérazinyl]-carbonyl}-phénol remplaçant le 3,5-bis-(1,1-diméthyléthyl)-4-hydroxy-N-(4-aminophényl)-benzamide. Après traitement par une solution molaire de HCl dans l'éther diéthylique anhydre, on obtient une poudre beige avec un rendement de 75 %. Point de fusion : 235-236 °C.
RMN $^1H$ (400 MHz, DMSO d6, δ) : 11,45 (s, 1H, $NH^+$); 9,78 (s, 1H, $NH^+$); 8,75 (s, 1H, $NH^+$); 8,19 (m, 2H, thiophène); 7,29 (m, 5H, Ph-N, thiophène); 7,10 (s, 2H, Ph); 5,60 (s large, 1H, OH); 3,70 (m, 4H, pipérazine); 3,30 (m, 4H, pipérazine); 1,40 (s, 18H, 2x tBu).
IR : $\nu_{OH}$ : 3633 $cm^{-1}$, 3433 $cm^{-1}$; $\nu_{C=O}$ (amide) : 1617 $cm^{-1}$ ; $\nu_{C=N}$ (amidine) : 1590 $cm^{-1}$.

**Exemple 10 : chlorhydrate de N-{4-[4-[3,5-bis-(1,1-diméthyléthyl)-4-hydroxybenzyl]-1-pipérazinyl]phényl}-2-thiophènecarboximidamide : 10**

**10.1) 2,6-bis-(1,1-diméthyléthyl)-4-bromométhylphénol :**

Dans un tricol de 250 ml sous atmosphère d'azote, on dissout 2,36 g (10 mmoles) d'alcool 3,5 di-tert-butyl-4-hydroxybenzylique dans 25 ml de THF anhydre. La solution est refroidie à l'aide d'un bain de glace avant addition goutte à goutte de 0,95 ml (10 mmoles) de tribromure de phosphore dilué par 25 ml de THF anhydre. Après 15 minutes d'agitation à 0 °C, la solution est diluée par 100 ml de dichlorométhane et lavée par 3x30 ml d'eau suivi de 30 ml de saumure. La phase organique est séchée sur sulfate de sodium, filtrée et concentrée sous vide pour donner une huile marron qui est utilisée directement dans l'étape suivante.

**10.2) 2,6-bis-(1,1-diméthyléthyl)-4-{[4-(4-nitrophényl)-1-pipérazinyl]-méthyl}-phénol :**

Dans un ballon de 100 ml contenant une solution de 2,99 g (10 mmoles) de 2,6-bis-(1,1-diméthyléthyl)-4-bromométhylphénol dans 30 ml de DMF, on ajoute successivement 1,38 g (10 mmoles) de carbonate de potassium et 2,07 g (10 mmoles) de 1-(4-nitrophényl)pipérazine. Après deux heures d'agitation à température ambiante, le mélange réactionnel est dilué par 150 ml de dichlorométhane et lavé successivement par 3x40 ml d'eau suivi de 40 ml de saumure. La solution organique est séchée sur sulfate de sodium, filtrée et concentrée sous pression réduite. Le résidu

marron obtenu est purifié sur colonne de gel de silice (éluant : éther de pétrole (Eb 40-70 °C) / acétate d'éthyle : 8/2). Après concentration des fractions pures, on obtient 2,31 g (54%) d'une poudre marron. Point de fusion : 177,5-178,5 °C.
RMN $^1H$ (100 MHz, $CDCl_3$, δ) : 7,50 (m, 4H, Ph-$NO_2$); 7,12 (s, 2H, Ph); 5,19 (s, 1H, OH); 3,50 (s, 2H, $\underline{CH_2}$-Ph); 3,49 (m, 4H, pipérazine); 2,60 (m, 4H, pipérazine); 1,49 (s, 18H, 2x tBu).

**10.3) 2,6-bis-(1,1-diméthyléthyl)-4-{[4-(4-aminophényl)-1-pipérazinyl]-méthyl}-phénol :**

Le protocole expérimental utilisé est le même que celui décrit pour l'intermédiaire 9.2, le 2,6-bis-(1,1-diméthyléthyl)-4-{[[4-(4-nitrophényl)-1-pipérazinyl]-carbonyl]-méthyl}-phénol remplaçant le 2,6-bis-(1,1-diméthyléthyl)-4-([4-(4-nitrophényl)-1-pipérazinyl]-carbonyl}-phénol. On obtient une poudre rose pâle avec un rendement de 75 %. Point de fusion : 152-154 °C.
RMN $^1H$ (100 MHz, $CDCl_3$, δ) : 7,12 (s, 2H, Ph); 6,78 (m, 4H, Ph-$NH_2$); 3,59 (s, 2H, $CH_2$-Ph); 3,18 (m, 4H, pipérazine); 2,70 (m, 4H, pipérazine); 1,47 (s, 18H, 2x tBu).

**10.4) chlorhydrate de N-{4-[4-[3,5-bis-(1,1-diméthyléthyl)-4-hydroxybenzyl]-1-pipérazinyl]-phényl}-2-thiophènecarboximidamide : <u>10</u>**

Dans un ballon de 100 ml contenant 0,59 g (1,5 mmole) de l'intermédiaire 10.3 dans 20 ml de 2-propanol, on ajoute 0,43 g (1,5 mmole) d'iodhydrate de S-méthyl-2-thiophène-thiocarboximide (Ann. *Chim.* (1962), **7,** 303-337). Après chauffage à reflux pendant 15 heures, le mélange réactionnel est concentré à sec sous vide. Le résidu est purifié sur une colonne de gel de silice (éluant : dichlorométhane/éthanol : 90/10). Les fractions pures sont concentrées sous vide et le résidu d'évaporation est salifié en présence d'une solution molaire de HCl dans l'éther diéthylique anhydre. On obtient une poudre jaune pâle avec un rendement de 40 %. Point de fusion : 234-236 °C.
RMN $^1H$ (400 MHz, DMSO d6, δ) : 11,60 (s, 1H, $NH^+$); 11,40 (s, 1H, $NH^+$); 9,75 (s, 1H, $NH^+$); 8,70 (s, 1H, $NH^+$); 8,17 (m, 2H, thiophène); 7,39 (s, 2H, Ph); 7,38 (m, 1H, thiophène); 7,24 (m, 5H, <u>Ph</u>-N, OH); 4,26 (d, 2H, $CH_2$-Ph, J = 4,6 Hz); 3,90 (m, 2H, pipérazine); 3,35 (m, 4H, pipérazine); 3,15 (m, 2H, pipérazine); 1,41 (s, 18H, 2x tBu).
IR : $\nu_{OH}$ : 3624 $cm^{-1}$, 3418 $cm^{-1}$; $\nu_{C=N}$ (amidine) : 1610 $cm^{-1}$.

**<u>Exemple 11</u> : chlorhydrate de N-{4-[4-[3,5-diméthoxy-4-hydroxybenzoyl]-1-pipérazinyl]-phényl}-2-thiophènecarboximidamide : <u>11</u>**

**11.1) 2,6-diméthoxy-4-{[4-(4-nitrophényl)-1-pipérazinyl]carbonyl}-phénol :**

Dans un ballon de 100 ml, on dissout 0,99 g (5 mmoles) d'acide syringique, 0,74 g (5,5 mmoles) d'hydroxybenzotriazole, 1,10 g (5,5 mmoles) de dicyclohexylcarbodiimide et 1,04 g (5 mmoles) de 1-(4-nitrophényl)pipérazine dans 10 ml de DMF. Après 7 heures d'agitation à température ambiante, le mélange est filtré et le précipité rincé par 20 ml de DMF suivi de 100 ml de chloroforme. On obtient 2 g d'une poudre jaune qui contient environ 20% de dicyclohexylurée. Le produit est utilisé tel quel dans l'étape suivante.
RMN $^1H$ (100 MHz, DMSO d6, δ) : 7,69 (m, 4H, Ph-$NO_2$); 6,88 (s, 2H, Ph); 5,72 (m, 1H, OH); 3,91 (s, 6H, 2x $OCH_3$); 3,75 (m, 4H, pipérazine); 3,49 (m, 4H, pipérazine).

**11.2) 2,6-diméthoxy-4-{[4-(4-aminophényl)-1-pipérazinyl]carbonyl}-phénol :**

Dans une bouteille de Parr de 250 ml, 2 g de l'intermédiaire 11.1 sont dissous dans 40 ml d'éthanol absolu/ DMSO (1/3) en présence de Pd/C à 10 %. Le mélange est agité sous 20 PSI d'hydrogène, à 25 °C, pendant 15 heures. Après filtration sur célite, le filtrat est concentré sous vide. Le résidu d'évaporation brun est repris par 50 ml d'acétate d'éthyle, le précipité formé est éliminé par filtration, rincé par 20 ml d'acétate d'éthyle et le filtrat extrait par 2x25 ml d'une solution molaire de HCl. La phase aqueuse est alcalinisée par addition de carbonate de sodium en poudre et extraite par 2x50 ml d'acétate d'éthyle. La solution organique est séchée sur sulfate de sodium, filtrée et concentrée sous vide. La poudre obtenue est reprise par 20 ml d'éther diéthylique contenant 3 ml de méthanol, filtrée et rincée à l'aide d'éther diéthylique. On recueille 400 mg (22 % sur les deux étapes) de cristaux marrons. Point de fusion : 182-183 °C.
RMN $^1H$ (100 MHz, DMSO d6, δ) : 6,80 (s, 2H, Ph); 6,74 (m, 4H, Ph-$NH_2$); 4,80 (m, 2H, $NH_2$); 3,91 (s, 6H, 2x $OCH_3$); 3,77 (m, 4H, pipérazine); 3,08 (m, 4H, pipérazine).

**11.3) chlorhydrate de N-{4-[4-[3,5-diméthoxy-4-hydroxybenzoyl]-1-pipérazinyl]-phényl}-2-thiophènecarboximidamide : 11**

Dans un ballon de 100 ml contenant une solution de 0,4 g (1,13 mmole) d'intermédiaire 11.2 dans 10 ml de 2-propanol, on ajoute 0,32 g (1,13 mmole) d'iodhydrate de S-méthyl-2-thiophènethiocarboximide (*Ann. Chim.* (1962), **7**, 303-337). Après chauffage à 50 °C pendant 15 heures, le mélange réactionnel est concentré à sec sous vide. Le résidu d'évaporation est ensuite repris par 100 ml d'un mélange acétate d'éthyle/solution saturée de carbonate de sodium (1/1). Un précipité apparaît, il est filtré et rincé successivement par 20 ml d'eau, 20 ml d'acétate d'éthyle et par 50 ml d'éther. La base obtenue est salifiée en présence d'une solution molaire de HCl dans l'éther diéthylique anhydre. Après filtration, rinçage par 10 ml d'acétone et séchage, on recueille 0,12 g (20%) d'une poudre jaune pâle. Point de fusion : 184-185 °C.
RMN $^1H$ (400 MHz, DMSO d6, $\delta$) : 11,47 (s, 1H, $NH^+$); 9,78 (s, 1H, $NH^+$); 8,76 (s, 1H, $NH^+$); 8,18 (m, 2H, thiophène); 7,37 (m, 1H, thiophène); 7,28 (m, 4H, Ph-N); 6,74 (s, 2H, Ph); 4,27 (s large, 1H, OH); 3,80 (s, 6H, 2x $OCH_3$); 3,70 (m, 4H, pipérazine); 3,33 (m, 4H, pipérazine).
IR : $\nu_{ON}$ : 3423 $cm^{-1}$; $\nu_{C=O}$ (amide) : 1610 $cm^{-1}$; $V_{C=N}$ (amidine) : 1587 $cm^{-1}$.

**Exemple 12 : chlorhydrate de 3,4-dihydro-6-hydroxy-2,5,7,8-tétraméthyl-N-{4-[(2-thiényl (imino)méthyl)amino] phényl}-2H-1-benzopyran-2-carboxamide : 12**

**12.1) 3,4-dihydro-6-hydroxy-2,5,7,8-tétraméthyl-N-(4-nitrophényl)-2H-1-benzopyran-2-carboxamide :**

Dans un ballon de 100 ml, on additionne 1,62 g (10 mmoles) de 1,1'-carbonyldiimidazole à une solution de 2,5 g (10 mmoles) de Trolox® dans 25 ml de THF. Après une heure d'agitation à température ambiante, on ajoute goutte à goutte une solution de 4-nitroaniline dans 20 ml de THF. L'agitation continue pendant 15 heures et on évapore le solvant sous vide. Le résidu est dilué dans 50 ml de dichlorométhane et lavé successivement par 25 ml d'une solution molaire d'acide chlorhydrique, 25 ml d'eau et 25 ml de saumure. La phase organique est séchée sur sulfate de sodium, filtrée et concentrée sous pression réduite. L'huile obtenue est purifiée sur une colonne de gel de silice (éluant : éther de pétrole (Eb 40-70 °C) / acétate d'éthyle : 7/3). Après concentration des fractions pures, on obtient une poudre jaune pâle avec un rendement de 77 %. Point de fusion : 150-151 °C.
RMN $^1H$ (100 MHz, $CDCl_3$, $\delta$) : 8,68 (s, 1H, CONH); 7.91 (m, 4H, Ph); 4,59 (s, 1H, OH); 2,95-0,87 (m, 16H, Trolox®).

**12.2) 3,4-dihydro-6-hydroxy-2,5,7,8-tétraméthyl-N-(4-aminophényl)-2H-1-benzopyran-2-carboxamide :**

Le protocole expérimental utilisé est le même que celui décrit pour l'intermédiaire 9.2, le 3,4-dihydro-6-hydroxy-2,5,7,8-tétraméthyl-N-(4-nitrophényl)-2H-1-benzopyran-2-carboxamide remplaçant le 2,6-bis-(1,1-diméthyléthyl)-4-{[4-(4-nitrophényl)-1-pipérazinyl]-carbonyl}-phénol. Le produit de la réaction est purifié sur une colonne de gel de silice (éluant : éther de pétrole (Eb 40-70° C) / acétate d'éthyle : 6/4). Les fractions pures sont collectées, après évaporation du solvant sous vide, on obtient une huile incolore avec un rendement de 45 %.
RMN $^1H$ (100 MHz, $CDCl_3$, $\delta$) : 8,19 (s, 1H, CONH); 7,00 (m, 4H, Ph); 4,59 (s, 1H, OH); 3,65 (s large, 2H, $NH_2$); 2,95-0,87 (m, 16H, Trolox®).

**12.3) chlorhydrate de 3,4-dihydro-6-hydroxy-2,5,7,8-tétraméthyl-N-{4-[2-thiényl (iminométhyl)amino]phényl}-2H-1-benzopyran-2-carboxamide : 12**

Le protocole expérimental utilisé est le même que celui décrit pour le composé **1,** le 3,4-dihydro-6-hydroxy-2,5,7,8-tétraméthyl-N-(4-aminophényl)-2H-1-benzopyran-2-carboxamide remplaçant le 3,5-bis-(1,1-diméthyléthyl)-4-hydroxy-N-(4-aminophényl)-benzamide. Point de fusion : 279-280 °C.
RMN $^1H$ (400 MHz, DMSO d6, $\delta$) : 9,80 (s, 1H, $NH^+$); 9,50 (s, 1H, $NH^+$); 8,73 (s, 1H, NHCO); 8,18 (m, 2H, thiophène); 7,60 (s, 1H, OH); 7,59 (m, 4H, Ph); 7,36 (m, 1H, thiophéne); 2,60-1,57 (m, 16H, Trolox®).
IR: $\nu_{OH}$ : 3236 $cm^{-1}$; $\nu_{C=O}$ (amide) : 1683 $cm^{-1}$; $\nu_{C=N}$ (amidine) : 1577 $cm^{-1}$.

**Exemple 13 : chlorhydrate de N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 13**

**13.1) 3,4-dihydro-2,5,7,8-tétraméthyl-2-{4-[(4-nitrophényl)-1-pipérazinyl]-carbonyl}-2H-1-benzopyran-6-ol :**

Dans un ballon de 100 ml, on additionne 1,62 g (10 mmoles) de 1,l'-carbonyl-diimidazole à une solution de 2,5 g (10 mmoles) de Trolox® dans 25 ml de THF. Après une heure d'agitation à température ambiante, on ajoute

goutte à goutte une solution de 1-(4-nitrophényl)pipérazine dans 10 ml de DMF. L'agitation continue pendant 15 heures, le mélange réactionnel est ensuite concentré sous vide. Le résidu d'évaporation est dissous dans 50 ml de dichlorométhane et lavé successivement par 3x25 ml d'eau et 25 ml de saumure. La phase organique est séchée sur sulfate de sodium, filtrée et concentrée sous pression réduite. L'huile obtenue est précipitée dans 30 ml d'un mélange (95/5) acétate d'éthyle/méthanol, le solide est filtré et lavé par 2x20 ml d'acétate d'éthyle. On obtient une poudre jaune pâle avec un rendement de 79 %. Point de fusion : 199-200 °C.
RMN $^1H$ (100 MHz, $CDCl_3$, δ) : 7,45 (m, 4H, Ph); 4,41-3,35 (m, 8H, pipérazine); 2,95-1,25 (m, 16H, Trolox®).

**13.2) 3,4-dihydro-2,5,7,8-tétraméthyl-2-{4-[(4-aminophényl)-1-pipérazinyl]-carbonyl}-2H-1-benzopyran-6-ol :**

Le protocole expérimental utilisé est le même que celui décrit pour l'intermédiaire 2.2, le 3,4-dihydro-2,5,7,8-tétraméthyl-2-{4-[(4-nitrophényl)-1-pipérazinyl]-carbonyl}-2H-1-benzopyran-6-ol remplaçant le 3,5-bis-(1,1-diméthyléthyl)-4-hydroxy-N-[(4-nitrophényl)méthyl]-benzamide. Le produit de la réaction est purifié sur une colonne de gel de silice (éluant : dichlorométhane/méthanol : 9/1). Les fractions pures sont collectées pour conduire, après évaporation du solvant sous vide, à une huile brune avec un rendement de 66 %.
RMN $^1H$ (100 MHz, $CDCl_3$, δ) : 6,70 (m, 4H, Ph); 4,15-2,97 (m, 8H, pipérazine); 2,80-0,90 (m, 18H, Trolox®).

**13.3) chlorhydrate de N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : <u>13</u>**

Le protocole expérimental utilisé est le même que celui décrit pour le composé **<u>1</u>**, le 3,4-dihydro-2,5,7,8-tétraméthyl-2-{4-[(4-aminophényl)-1-pipérazinyl]-carbonyl}-2H-1-benzopyran-6-ol remplaçant le 3,5-bis-(1,1-diméthyléthyl)-4-hydroxy-N-(4-amino-phényl)-benzamide. Cependant la réaction est plus lente et nécessite 15 heures de chauffage. La base obtenue après extraction est purifiée sur une colonne de gel de silice (éluant : éther de pétrole (Eb 40-70 °C) / acétate d'éthyle : 3/7). Les fractions pures sont concentrées sous vide et le résidu d'évaporation est salifié en présence d'une solution molaire de HCl dans l'éther diéthylique anhydre. On obtient une poudre jaune pâle avec un rendement de 40 %. Point de fusion : 210-211 °C. RMN $^1H$ (400 MHz, DMSO d6, δ) : 11,50 (s, 1H, $NH^+$); 9,79 (s, 1H, $NH^+$); 8,69 (s, 1H, $NH^+$); 8,19 (m, 2H, thiophène); 7,38 (m, 1H, thiophène); 7,20 (m, 4H, Ph); 4,58 (s large, 1H, OH); 4,11 (m, 2H, pipérazine); 3,61 (m, 2H, pipérazine); 3,19 (m, 4H, pipérazine); 2,62-1,55 (m, 16H, Trolox®).
IR: $\nu_{OH}$ : 3410 $cm^{-1}$ ; $\nu_{C=O}$ (amide) : 1642 $cm^{-1}$ ; $\nu_{C=N}$ (amidine): 1613 $cm^{-1}$,

**<u>Exemple 14</u> : N-{4-[4-[(5 méthoxy-1H-indol-3-yl)méthylcarbonyl]-1-pipérazinyl]phényl}-2-thiophènecarboximidamide : <u>14</u>**

**14.1) 1-[(5-méthoxy-1H-indol-3-yl)méthylcarbonyl]-4-(4-nitrophényl)-pipérazine :**

Dans un ballon de 100 ml, on additionne 1,62 g (10 mmoles) de 1,1'-carbonyldiimidazole à une solution de 2,05 g (10 mmoles) de l'acide 5-méthoxyindole-3-acétique dans 10 ml de THF. Après une heure d'agitation à température ambiante, on ajoute goutte à goutte une solution de 1-(4-nitrophényl)pipérazine dans 10 ml de DMF. L'agitation continue pendant 15 heures. Le mélange réactionnel est ensuite concentré sous vide et le résidu d'évaporation est précipité dans 50 ml d'un mélange acétate d'éthyle/eau (1/1). Après filtration, le solide est rincé successivement par 50 ml d'eau, 50 ml d'acétate d'éthyle et 50 ml de dichlorométhane. On obtient une poudre jaune, après séchage sous vide, avec un rendement de 91 %. Point de fusion : 239-240 °C.
RMN $^1H$ (100 MHz, DMSO d6, δ) : 10,90 (m, 1H, NH); 7,63 (m, 4H, Ph-$NO_2$); 7,40-7,15 (m, 3H, indole); 6,87 (dd, 1H indole, $J_{ortho}$ = 8,7 Hz, $J_{meta}$ = 2,8 Hz); 3,90 (s, 2H, $CH_2$-CO); 3,88 (s, 3H, $OCH_3$); 3,79 (m, 4H, pipérazine); 3,50 (m, 4H, pipérazine).

**14.2) 1-[(5 méthoxy-1H-indol-3-yl)méthylcarbonyl]-4-(4-aminophényl)-pipérazine :**

Dans une bouteille de Parr de 250 ml, 1 g (2,53 mmoles) de l'intermédiaire 14.1 est dissous dans 30 ml de DMSO en présence de Pd/C à 10%. Le mélange est agité sous 20 PSI d'hydrogène, à 25°C, pendant 7 heures. Après filtration sur célite, le filtrat est concentré sous vide. Le résidu d'évaporation est dilué dans 50 ml d'acétate d'éthyle et lavé par 3x50 ml d'eau. La phase organique est ensuite extraite par 2 x 25 ml d'une solution molaire de HCl. Après avoir lavé la solution acide par 2x25 ml d'acétate d'éthyle, celle-ci est alcalinisée à l'aide de carbonate de sodium en poudre. Une fois le produit réextrait à l'aide de 2x50 ml d'acétate d'éthyle, la solution organique est séchée sur sulfate de sodium, filtrée et le solvant évaporé sous vide. Le résidu est purifié sur une colonne de gel de silice (éluant : dichlorométhane/méthanol: 98/2). Les fractions pures sont collectées et après évaporation du solvant sous pression réduite, on obtient 0,39 g d'une poudre jaune pâle avec un rendement de 46 %. Point de fusion : 119-120° C.

RMN $^1H$ (100 MHz, $CDCl_3$, δ) : 8,32 (s, 1H, NH indolique); 7,27-6,80 (m, 4H, indole) ; 6,69 (m, 4H, Ph-$NH_2$); 3,82 (s, 3H, $OCH_3$); 3,80 (s, 2H, $CH_2$-CO); 3,80 (m, 2H, pipérazine); 3,62 (m, 2H, pipérazine); 3,48 (s, 2H, $NH_2$); 2,90 (m, 4H, pipérazine).

**14.3) N-{4-[4-[(5 méthoxy-1H-indol-3-yl)méthylcarbonyl]-1-pipérazinyl]phényl}-2-thiophènecarboximidamide : <u>14</u>**

Le protocole expérimental utilisé est le même que celui décrit pour le composé **<u>1</u>**, la 1-[(5 méthoxy-1H-indol-3-yl)méthylcarbonyl]-4-(4-aminophényl)-pipérazine remplaçant le 3,5-bis-(1,1-diméthyléthyl)-4-hydroxy-N-(4-aminophényl)-benzamide. Le produit attendu est isolé sous forme de base libre avec un rendement de 20 % (poudre jaune pâle). Point de fusion : 221-222 °C.
RMN $^1H$ (400 MHz, DMSO d6, δ) : 10,78 (s, 1H, NH indolique); 7,72 (m, 1H, thiophène); 7,59 (m, 1H, thiophène); 7,22 (d, 1H, indole, J = 8,7 Hz); 7,19 (m, 1H, thiophène); 7,09 (m, 2H, indole); 6,82 (m, 4H, Ph); 6,72 (m, 1H indole); 6,35 (s, 2H, $NH_2$); 3,80 (s, 2H, $CH_2$); 3,73 (s, 3H, $CH_3$); 3,62 (m, 4H, pipérazine); 2,95 (m, 4H, pipérazine).
IR : $\nu_{OH}$ : 3414 $cm^{-1}$ ; $\nu_{C=O}$ (amide) : 1628 $cm^{-1}$ ; $\nu_{C=N}$ (amidine) : 1590 $cm^{-1}$.

**<u>Exemple 15</u> : fumarate de N-[4-[4-[{3-[3,5-bis-(1,1-diméthyléthyl)-4-hydroxyphényl]-1-oxo-2-propènyl}-1-pipérazinyl]-phényl]]-2-thiophènecarboximidamide : <u>15</u>**

**15.1) 2,6-bis-(1,1-diméthyléthyl)-4-{3-[4-(4-nitrophényl)-1-pipérazinyl]-3-oxo-2-propènyl}-phénol :**

Le protocole expérimental utilisé est le même que celui décrit pour l'intermédiaire 11.1, l'acide 3,5-di-tert-butyl-4-hydroxycinnamique remplaçant l'acide syringique. On obtient une huile avec un rendement de 60 %.
RMN $^1H$ (100 MHz, $CDCl_3$, δ) : 7,71 (d, 1H, C=CH, J = 15,0 Hz); 7,51 (m, 4H, Ph-$NO_2$); 7,38 (s, 2H, Ph); 6,69 (d, 1H, HC=C, J = 15,0 Hz); 5,50 (s, 1H, OH); 3,88 (m, 4H, pipérazine); 3,53 (m, 4H, pipérazine); 1,47 (s, 18 H, 2x tBu).

**15.2) 2,6-bis-(1,1-diméthyléthyl)-4-{3-[4-(4-aminophényl)-1-pipérazinyl]-3-oxo-2-propènyl}-phénol :**

Dans un ballon de 50 ml équipé d'un réfrigérant, on dissout 0,5 g (1 mmole) de l'intermédiaire 15.1 dans 5 ml d'acide chlorhydrique concentré et 5 ml d'éthanol absolu. On refroidit le mélange à 0 °C et on ajoute en plusieurs portions 1,69 g (7,5 mmoles) de chlorure d'étain (dihydrate). A la fin de l'addition, le milieu réactionnel est chauffé à reflux pendant 30 minutes. Les solvants sont ensuite évaporés sous vide, le résidu est repris par 15 ml d'eau, neutralisé par de la soude 2N et dilué par 20 ml de dichlorométhane. Le précipité obtenu est filtré sur célite et le filtrat est décanté. La phase organique est séchée sur sulfate de sodium, filtrée et concentrée sous pression réduite pour obtenir 0,3 g (67 %) d'une huile jaune.
RMN $^1H$ (100 MHz, $CDCl_3$, δ) : 7,66 (d, 1H, C=CH, J = 15,0 Hz); 7,37 (s, 2H, Ph); 6,75 (m, 4H, Ph-$NH_2$); 6,30 (d, 1H, HC=C, J = 15,0 Hz); 5,46 (s, 1H, OH); 3,80 (m, 4H, pipérazine); 3,06 (m, 4H, pipérazine); 1,46 (s, 18 H, 2x tBu).

**15.3) fumarate de N-[4-[4-[{3-[3,5-bis-(1,1-diméthyléthyl)-4-hydroxy-phényl]-1-oxo-2-propényl}-1-pipérazinyl]-phényl]]-2-thiophènecarboximidamide : <u>15</u>**

Le protocole expérimental utilisé est le même que celui décrit pour le composé **<u>1</u>**, le 2,6-bis-(1,1-diméthyléthyl)-4-{3-[4-(4-aminophényl)-1-pipérazinyl]-3-oxo-2-propènyl}-phénol remplaçant le 3,5-bis-(1,1-diméthyléthyl)-4-hydroxy-N-(4-aminophényl)-benzamide.
Le produit de la réaction est salifié en présence d'une quantité équimolaire d'acide fumarique dans l'éthanol à reflux. On obtient le composé **<u>15</u>** sous forme d'une poudre jaune avec un rendement de 22%. Point de fusion : 170,5-173 °C.
RMN $^1H$ (400 MHz, DMSO d6, δ) : 7,77 (s, 1H, thiophène); 7,67 (d, 1H, thiophène, J = 5,0 Hz); 7,48 (d, 1H, C=CH, J = 15,0 Hz); 7,39 (s, 2H, Ph); 7,34 (s large, 1H, OH); 7,13 (t, 1H, thiophène, J= 4,0 Hz); 7,05 (d, 1H, HC=C, J = 15,0 Hz); 6,92 (m, 4H, Ph-N); 6,60 (s, 2H, CH=CH fumarate); 3,78 (m, 4H, pipérazine); 3,13 (m, 4H, pipérazine); 1,41 (s, 18 H, 2x tBu).
IR : $\nu_{OH}$ : 3619 $cm^{-1}$, 3300 $cm^{-1}$; $\nu_{C=O}$ (amide) : 1640 $cm^{-1}$ ; $\nu_{C=C}$ : 1600 $cm^{-1}$ ; $\nu_{C=N}$ (amidine) : 1570 $cm^{-1}$.

**Exemple 16 : chlorhydrate de 3,5-bis-(1,1-diméthyléthyl)-4-hydroxy-N-{3-[[(2-thiényl-(imino)méthyl)amino]phényl]méthyl}-benzamide : 16**

**16.1) 3,5-bis-(1,1-diméthyléthyl)-4-hydroxy-N-[(3-nitrophényl)méthyl]-benzamide :**

Le protocole expérimental utilisé est le même que celui décrit pour l'intermédiaire 2.1, le chlorhydrate de 3-nitrobenzylamine remplaçant le chlorhydrate de 4-nitrobenzylamine. On obtient une poudre blanche avec un rendement de 63 %. Point de fusion : 210-211 °C.
RMN $^1H$ (100 MHz, DMSO, δ) : 9,12 (m, 1H, NH) ; 8,25 (m, 2H, Ph-$NO_2$) ; 7,80 (m, 4H, Ph-$NO_2$ + Ph-OH) ; 7,60 (s large, 1H, OH) ; 4,68 (d, 2H, $CH_2$, J = 6 Hz) ; 1,55 (s, 18H, 2 x tBu).

**16.2) 3,5-bis-(1,1-diméthyléthyl)-4-hydroxy-N-[(3-aminophényl)méthyl]-benzamide :**

Dans une bouteille de Parr de 250 ml, 2,40 g (6,2 mmoles) de 3,5-bis-(1,1-diméthyléthyl)-4-hydroxy-N-[(3-nitrophényl)méthyl]-benzamide sont dissous dans 45 ml d'un mélange éthanol absolu/THF (1/2) en présence de Pd/C à 10 %. Le mélange est agité sous 20 PSI d'hydrogène, à 30 °C, pendant trois heures. Après filtration sur célite, le filtrat est concentré à sec et le résidu est purifié sur colonne de silice (éluant : heptane/acétate d'éthyle : 60 /40). Les fractions pures sont collectées et concentrées sous pression réduite pour conduire à 0,94 g (45%) d'une poudre blanche. Point de fusion : 171-172 °C.
RMN $^1H$ (100 MHz, $CDCl_3$, δ) : 7,20 (m, 2H, Ph-$NH_2$) ; 6,70 (m, 4H, Ph-$NH_2$ + Ph-OH) ; 6,34 (m, 1H, NH) ; 5,55 (s, 1H, OH) ; 4,56 (d, 2H, $CH_2$, J = 6 Hz) ; 3,70 (s large, 2H, $NH_2$) ; 1,49 (s, 18H, 2 x tBu).

**16.3) chlorhydrate de 3,5-bis-(1,1-diméthyléthyl)-4-hydroxy-N-{3-[[(2-thiényl-(imino)méthyl)-amino]phényl]méthyl}-benzamide : 16**

Le protocole expérimental utilisé est le même que celui décrit pour le composé **1,** le 3,5-bis-(1,1-diméthyléthyl)-4-hydroxy-N-[(3-aminophényl)méthyl]-benzamide remplaçant le 3,5-bis-(1,1-diméthyléthyl)-4-hydroxy-N-(4-aminophényl)-benzamide. Après salification par une solution molaire de HCl dans un mélange acétone/méthanol anhydre, on obtient une poudre jaune pâle avec un rendement de 50 %. Point de fusion : 226-227 °C.
RMN $^1H$ (400 MHz, DMSO d6, δ) : 11,71 (s, 1H, $NH^+$); 9,93 (s, 1H, $NH^+$); 9,10 (s, 1H, CONH); 9,00 (s, 1H, $NH^+$); 8,18 (m, 2H, thiophène); 7,70 (s, 2H, Ph); 7,42 (m, 6H, thiophène, Ph-NH, OH); 4,50 (d, 2H, $CH_2$-NHCO, J = 5,4 Hz); 1,40 (s, 18H, 2 x tBu).
IR : $\nu_{OH}$ : 3420 $cm^{-1}$ ; $\nu_{C=O}$ (amide) : 1639 $cm^{-1}$ ; $\nu_{C=N}$ (amidine) : 1578 $cm^{-1}$.

**Exemple 17 : chlorhydrate de N-[3,5-bis-(1,1-diméthyléthyl)-4-hydroxyphényl]-N'-{{4-[(2-thiényl(imino)méthyl) amino]phényl}méthyl}-urée : 17**

**17.1) 4-amino-2,6-bis-(1,1-diméthyléthyl)-phénol :**

Dans une bouteille de Parr de 250 ml, 3,6 g (14 mmoles) de 4-nitro-2,6-bis-(1,1-diméthyléthyl)-phénol (*J. Org. Chem.* (1968), **33** (1), 223-226) sont dissous dans 60 ml d'un mélange (2/1) d'éthanol et dichlorométhane en présence d'une quantité catalytique de Pd/C à 10 %. Le mélange est agité pendant 2 heures, à 20 °C, sous 20 PSI d'hydrogène. Après filtration sur célite, le filtrat est concentré à sec sous pression réduite. La poudre rousse obtenue est suspendue dans de l'heptane (30 ml), filtrée et rincée par le même volume d'heptane. Le produit attendu est obtenu sous forme d'une poudre rose saumon avec un rendement de 50 % (1,56 g). Point de fusion : 123-124 °C.
RMN $^1H$ (100 MHz, $CDCl_3$, δ) : 6,60 (s, 2H, Ph) ; 4,65 (s large, 1H, OH) ; 3,15 (s large, 2H, $NH_2$) ; 1,42 (s, 18H, 2x tBu).

**17.2) chlorure de l'acide 4-nitrophénylacétique :**

A une solution de 0,9 g (5 mmoles) d'acide 4-nitrophénylacétique dans un mélange composé de 10 ml de dichlorométhane et de 0,5 ml de DMF, on ajoute, à 20 °C, 3,75 ml (7,5 mmoles) d'une solution 2 M de chlorure d'oxalyle dans le dichlorométhane. Après une demi-heure d'agitation, la solution est concentrée sous vide. L'huile jaune obtenue est utilisée sans purification supplémentaire dans l'étape suivante.

**17.3) 4-nitrobenzylisocyanate :**

A une solution aqueuse de 0,75 g (11,5 mmoles) d'azide de sodium, refroidie à 0 °C, on additionne lentement le chlorure de l'acide 4-nitrophénylacétique en solution dans l'acétone sec (7,5 ml). L'agitation du milieu est maintenue

pendant une heure après la fin de l'addition, à 0-5 °C. Le milieu réactionnel est ensuite dilué par 30 ml de chloroforme, décanté et la phase organique lavée par de l'eau (20 ml) suivi d'une solution saturée de chlorure de sodium (20 ml). Après séchage sur sulfate de sodium, la solution organique est filtrée et concentrée partiellement (≈ 20 ml) sous vide. Cette solution de l'acylazide dans le chloroforme est ensuite chauffée, à reflux, pendant une heure. L'isocyanate obtenu est utilisé directement, en solution, dans l'étape suivante.

**17.4) N-[3,5-bis-(1,1-diméthyléthyl)-4-hydroxyphényl]-N'-[(4-nitrophényl)méthyl]-urée :**

A la solution de l'isocyanate (intermédiaire 17.3) (5 mmoles théoriques) dans 20 ml de chloroforme, on additionne en une portion 1,1 g (5 mmoles) de 4-amino-2,6-bis-(1,1-diméthyléthyl)-phénol. Après 2 heures d'agitation, à 20 °C, le précipité apparu est filtré et rincé par du chloroforme (2x20 ml). On obtient une poudre jaune avec un rendement de 72 %. Point de fusion : 240-241 °C.
RMN $^1$H (100 MHz, DMSO d6, δ) : 8,60 (s, 1H, NH-Ph) ; 8,01 (m, 4H, Ph-$NO_2$) ; 7,30 (s, 2H, Ph-OH) ; 6,77 (m, 1H, NH-$CH_2$) ; 6,71 (s, 1H, OH) ; 4,52 (d, 2H, $CH_2$, J = 5,5 Hz) ; 1,49 (s, 18H, 2x tBu).

**17.5) N-[(4-aminophényl)méthyl]-N'-[3,5-bis-(1,1-diméthyléthyl)-4-hydroxyphényl]-urée :**

Dans un autoclave de 100 ml, on dissout 0,55 g (1,38 mmole) de N-[3,5-bis-(1,1-diméthyléthyl)-4-hydroxyphényl]-N'-[(4-nitrophényl)méthyl]-urée dans un mélange (2/1) d'éthanol et d'acétate d'éthyle, en présence de Pd/C à 10%. Après une heure et demie d'hydrogènation, à 20 °C, sous 20 PSI, on filtre le mélange sur célite et on concentre le filtrat sous vide. Le résidu d'évaporation est dilué dans 20 ml d'éther diéthylique et le produit attendu cristallise spontanément. Les cristaux sont filtrés et rincés par 20 ml d'éther diéthylique. On obtient une poudre blanche avec un rendement de 60 % (0,31 g). Point de fusion : 194-195 °C.
RMN $^1$H (100 MHz, $CDCl_3$, δ) : 7,08 (s, 2H, Ph-OH) ; 6,87 (m, 4H, Ph-$NH_2$) ; 6,15 (s, 1H, NH-Ph) ; 5,14 (s, 1H, OH) ; 4,89 (m, 1H, NH-$CH_2$) ; 4,41 (d, 2H, $CH_2$, J = 5,5 Hz) ; 3,65 (s large, 2H, $NH_2$) ; 1,40 (s, 18H, 2x tBu).

**17.6) chlorhydrate de N-[3,5-bis-(1,1-diméthyléthyl)-4-hydroxyphényl]-N'-{{4-[2-thiényl(imino-méthyl)amino] phényl}méthyl}-urée : 17**

Le protocole expérimental utilisé est le même que celui décrit pour le composé **1,** le N-[(4-aminophényl)méthyl] -N'-[3,5-bis-(1,1-diméthyléthyl)-4-hydroxyphényl]-uréeremplaçantle3,5-bis-(1,1-diméthyléthyl)-4-hydroxy-N-(4-aminophényl)-benzamide. Après salification par une solution molaire de HCl dans de l'éther diéthylique anhydre, on obtient une poudre blanche avec un rendement de 45 %. Point de fusion : 236-237 °C.
RMN $^1$H (400 MHz, DMSO d6, δ) : 11,42 (s large, 1H, $NH^+$) ; 9,77 (s large, 1H, $NH^+$) ; 8,92 (s large, 1H, $NH^+$) ; 8,54 (s, 1H, NH-Ph) ; 8,11 (m, 2H, thiophène) ; 7,41 (m, 5H, Ph-N, thiophène) ; 7,19 (s, 2H, Ph) ; 6,70 (m, 1H, NH-$CH_2$) ; 6,60 (s, 1H, OH) ; 4,35 (d, 2H, $CH_2$, J = 5,5 Hz) ; 1,34 (s, 18H, 2x tBu).
IR : $\nu_{OH}$ : 3624 $cm^{-1}$; $\nu_{C=O}$ (urée) : 1644 $cm^{-1}$ ; $\nu_{C=N}$ (amidine) : 1569 $cm^{-1}$.

**Exemple 18 : chlorhydrate de N-[5-[{3-(3,5-bis-(1,1-diméthyléthyl)-4-hydroxyphényl)-1-oxo-2-propènyl)-amino]-2-hydroxyphényl]-2-thiophènecarboximidamide : 18**

**18.1) 3-[(3,5-bis-(1,1-diméthyléthyl)-4-hydroxyphényl]-N-(4-hydroxy-3-nitrophényl)-2-propènamide :**

Dans un ballon de 50 ml contenant 10 ml de THF, on introduit 1,78 g (6,4 mmoles) d'acide 3,5-di-*tert*-butyl-4-hydroxycinnamique, 0,99 g (6,4 mmoles) de 4-amino-2-nitrophénol, préalablement dilué dans 10 ml de DMF, 0,86 g (6,4 mmoles) d'hydroxybenzotriazole et 1,32 g (6,4 mmoles) de dicyclohexylcarbodiimide. Le mélange réactionnel est agité pendant 15 heures à température ambiante, le précipité apparu est filtré et rincé avec de l'acétate d'éthyle. Après concentration de la solution sous pression réduite, le résidu est dilué dans 20 ml d'acétate d'éthyle et l'insoluble est filtré à nouveau. Le filtrat est lavé par 20 ml d'une solution saturée de carbonate de sodium suivi de 20 ml d'eau et 20 ml d'une solution saturée de chlorure de sodium. Après sèchage sur sulfate de sodium, la solution organique est filtrée et concentrée à sec sous pression réduite. Le résidu est purifié sur colonne de silice (éluant : heptane/acétate d'éthyle : 8/2). Les fractions pures sont collectées et concentrées sous vide pour conduire à 1,95 g (47 %) du composé attendu sous forme d'une poudre jaune-orangée. Point de fusion : 231-232 °C.
RMN $^1$H (100 MHz, $CDCl_3$, δ) : 10,45 (s, 1H, NH) ; 8,45 (d, 1H, Ph-$NO_2$, J = 1,7 Hz) ; 7,98 (dd, 1H, Ph-$NO_2$, J = 1,7 Hz et J = 6,8 Hz) ; 7,78 (d, 1H, -CH=CH-, J = 10,5 Hz) ; 7,75 (s, 1 H, OH) ; 7,40 (s, 2H, Ph-OH) ; 7,20 (d, 1H, Ph-$NO_2$) ; 6,48 (d, 1H, -CH=CH-) ; 5,51 (s, 1H, OH) ; 1,50 (s, 18H, 2 x tBu).

**18.2) 3-[(3,5-bis-(1,1-diméthyléthyl)-4-hydroxyphényl]-N-(4-hydroxy-3-aminophényl)-2-propènamide :**

Dans un ballon de 100 ml surmonté d'un réfrigérant, on dissout 0,9 g (2,18 mmoles) de 3-[(3,5-bis-(1,1-diméthyléthyl)-4-hydroxyphényl]-N-(4-hydroxy-3-nitrophényl)-2-propènamide dans 20 ml d'acétate d'éthyle, on ajoute 2,46 g (10,9 mmoles) de chlorure d'étain (dihydrate) et le mélange est chauffé à 70 °C pendant trois heures. Après retour à température ambiante, le milieu réactionnel est versé sur une solution agitée de bicarbonate de sodium (0,1 M), un précipité se forme, celui est éliminé par filtration sur célite. Le filtrat est décanté et la phase aqueuse est extraite par 20 ml d'acétate d'éthyle. Les phases organiques sont groupées et lavées par 20 ml d'eau suivi de 20 ml d'une solution saturée de chlorure de sodium. Après séchage sur sulfate de sodium et filtration, la solution organique est concentrée à sec, sous vide partiel. Le résidu d'évaporation est suspendu dans un mélange heptane/acétate d'éthyle (1/1) et filtré pour conduire à une poudre jaunâtre avec un rendement de 53 %. Le produit est utilisé tel quel dans l'étape suivante.

**18.3) chlorhydrate de N-[5-[{3-(3,5-bis-(1,1-diméthyléthyl)-4-hydroxyphényl)-1-oxo-2-propènyl}amino]-2-hydroxyphényl]-2-thiophènecarboximidamide : 18**

Le protocole expérimental utilisé est le même que celui décrit pour le composé **1**, le 3-[(3,5-bis-(1,1-diméthyléthyl)-4-hydroxyphényl]-N-(4-hydroxy-3-aminophényl)-2-propènamide remplaçant le 3,5-bis-(1,1-diméthyléthyl)-4-hydroxy-N-(4-aminophényl)-benzamide. La base libre est purifiée sur colonne de silice (éluant : heptane/acétate d' éthyle : 35/65). Les fractions pures sont collectées et concentrées sous pression réduite. Le résidu d'évaporation est dilué dans 10 ml d'acétone et salifié par une solution molaire de HCl dans l'éther anhydre, comme décrit précédemment. On obtient finalement 0,35 g (62%) d'une poudre jaune. Point de fusion : 199-200 °C.
RMN $^1H$ (400 MHz, DMSO, $\delta$) : 11,11 (s, 1H, $NH^+$); 10,29 (s, 1H, $NH^+$) ; 10,17 (s, 1H, $NH^+$) ; 9,71 (s, 1H, CONH) ; 8,61 (s large, 1H, OH) ; 8,14 (m, 2H, thiophène) ; 7,79 (s, 1H, Ph-N) ; 7,53 (m, 1H, Ph-N) ; 7,48 (d, 1H, -CH=CH-, J = 14,7 Hz) ; 7,37 (m, 4H, Ph-tBu + OH + Ph-N) ; 7,05 (m, 1H, thiophène) ; 6,68 (d, 1H, -CH=CH-) ; 1,41 (s, 18H, 2 x tBu).
IR : $\nu_{OH}$ : 3624 $cm^{-1}$, 3415 $cm^{-1}$; $\nu_{C=O}$ (amide) : 1656 $cm^{-1}$ ; $\nu_{C=C}$ : 1616 $cm^{-1}$ ; $\nu_{C=N}$ (amidine) : 1587 $cm^{-1}$.

**Exemple 19 : chlorhydrate de N-[3-[{3-(3,5-bis-(1,1-diméthyléthyl)-4-hydroxyphényl)-1-oxo-2-propènyl}-amino]-4-hydroxyphényl]-2-thiophènecarboximidamide : 19**

**19.1) 3-[(3,5-bis-(1,1-diméthyléthyl)-4-hydroxyphényl]-N-(2-hydroxy-5-nitrophényl)-2-propènamide :**

Le protocole expérimental utilisé est le même que celui décrit pour l'intermédiaire 18.1, le 2-amino-4-nitrophénol remplaçant le 4-amino-2-nitrophénol. On obtient une poudre jaune clair avec un rendement de 25 %. Point de fusion : 256-257 °C.
RMN $^1H$ (400 MHz, DMSO, $\delta$) : 11,79 (s large, 1H, OH) ; 9,59 (s, 1H, NH); 9,21 (s large, 1H, $Ph-NO_2$) ; 7,90 (dd mal résolu, 1H, $Ph-NO_2$, J = 8,1 Hz); 7,52 (d, 1H, -CH=CH-, J = 15,5 Hz) ; 7,47 (s, 1H, OH) ; 7,42 (s, 2H, Ph-OH); 7,15 (d, 1H, -CH=CH-) ; 7,04 (d, 1H, $Ph-NO_2$) ; 1,42 (s, 18H, 2 x tBu).

**19.2) 3-[(3,5-bis-(1,1-diméthyléthyl)-4-hydroxyphényl]-N-(2-hydroxy-5-anunophényl)-2-propènamide :**

Le protocole expérimental utilisé est le même que celui décrit pour l'intermédiaire 18.2, le 3-[(3,5-bis-(1,1-diméthyléthyl)-4-hydroxyphényl]-N-(2-hydroxy-5-nitrophényl)-2-propènamide remplaçant le 3-[(3,5-bis-(1,1-diméthyléthyl)-4-hydroxyphényl]-N-(4-hydroxy-3-nitrophényl)-2-propènamide. On obtient une poudre jaune avec un rendement de 74 %. Le produit est utilisé sans purification supplémentaire dans l'étape suivante.

**19.3) chlorhydrate de N-[5-[{3-(3,5-bis-(1,1-diméthyléthyl)-4-hydroxyphényl)-1-oxo-2-propènyl}-amino]-2-hydroxyphényl]-2-thiophènecarboximidamide : 19**

Le protocole expérimental utilisé est le même que celui décrit pour le composé **1,** le 3-[(3,5-bis-(1,1-diméthyléthyl)-4-hydroxyphényl]-N-(2-hydroxy-5-aminophényl)-2-propènamide remplaçant le 3,5-bis-(1,1-diméthyléthyl)-4-hydroxy-N-(4-aminophényl)-benzamide. Après salification par une solution molaire de HCl dans l'éther diéthylique anhydre, on obtient une poudre jaune avec un rendement de 54 %. Point de fusion : 256-257 °C.
RMN $^1H$ (400 MHz, DMSO d6, $\delta$) : 11,32 (s, 1H, $NH^+$) ; 10,67 (s, 1H, $NH^+$) ; 9,69 (s, 1H, $NH^+$) ; 9,55 (s, 1H, CONH) ; 8,70 (s large, 1H, OH) ; 8,19 (m, 2H, thiophène); 7,48 (d, 1H, -CH=CH-, J = 15,5 Hz) ; 7,40 (s, 2H, Ph-tBu) ; 7,37 (m, 2H, Ph-N) ; 7,34 (s, 1H, OH) ; 7,13 (d, 1H, -CH=CH-) ; 7,10 (m, 1H, Ph-N) ; 6,99 (m, 1H, thiophène) ; 1,41 (s, 18H, 2 x tBu).
IR : $\nu_{OH}$ : 3623 $cm^{-1}$, 3410 $cm^{-1}$; $\nu_{C=O}$ (amide) : 1652 $cm^{-1}$ ; $\nu_{C=C}$ : 1616 $cm^{-1}$ ; $\nu_{C=N}$ (amidine) : 1587 $cm^{-1}$.

**Exemple 20 : chlorhydrate de N-{4-[4-[3,4,5-trihydroxybenzoyl]-1-pipérazinyl]phényl}-2-thiophènecarboximidamide : 20**

**20.1) 5-{[4-(4-nitrophényl)-1-pipérazinyl]carbonyl}-benzène-1,2,3-triol :**

Le protocole expérimental est le même que celui décrit pour l'intermédiaire 8.1, la 1-(4-nitrophényl)pipérazine remplaçant la 4-nitrophénétylamine. On obtient une poudre jaune qui contient encore une trace d'impuretés avec un rendement de 43 %.
RMN $^1$H (100 MHz, DMSO, $\delta$) : 9,17 (s large, 2H, 2x -OH) ; 8,55 (s large, 1H, -OH) ; 7,57 (m, 4H, Ph-$NO_2$) ; 6,40 (s, 2H, Ph-OH) ; 3,59 (m mal résolu, 8 H, pipérazine).

**20.2) 5-{[4-(4-aminophényl)-1-pipérazinyl]carbonyl}-benzène-1,2,3-triol :**

Le protocole expérimental utilisé est le même que celui décrit pour l'intermédiaire 2.2, le 5-{[4-(4-nitrophényl)-1-pipérazinyl]carbonyl}-benzène-1,2,3-triol remplaçant le 3,5-bis-(1,1-diméthyléthyl)-4-hydroxy-N-[(4-nitrophényl)méthyl]-benzamide. On obtient une poudre beige avec un rendement de 61 %. Celle-ci est investie directement dans l'étape suivante sans purification supplémentaire.
RMN $^1$H (100 MHz, DMSO, $\delta$) : 9,12 (s large, 2H, 2x -OH) ; 8,55 (s large, 1H, -OH) ; 6,61 (m, 4H, Ph-$NH_2$) ; 6,34 (s, 2H, Ph-OH) ; 3,59 (m, 4H, pipérazine) ; 2,89 (m, 4H, pipérazine).

**20.3) chlorhydrate de N-{4-[4-[3,4,5-trihydroxybenzoyl]-1-pipérazinyl]phényl}-2-thiophènecarboximidamide : 20**

Le protocole expérimental utilisé est le même que celui décrit pour le composé 1, le 5- {[4-(4-aminophényl)-1-pipérazinyl]carbonyl}-benzène-1,2,3-triol remplaçant le 3,5-bis-(1,1-diméthyléthyl)-4-hydroxy-N-(4-aminophényl)-benzamide. Après traitement par une solution molaire de HCl dans l'éther diéthylique anhydre, on obtient une poudre marron avec un rendement de 25 %. Point de fusion : 198-205 °C.
RMN $^1$H (400 MHz, DMSO d6, $\delta$) : 11,38 (s, 1H, $NH^+$); 9,75 (s, 1H, $NH^+$); 9,00 (s large, 1H, OH) ; 8,75 (s, 1H, $NH^+$); 8,15 (m, 2H, thiophène); 7,39 (m, 1H, thiophène) ; 7,22 (m, 4H, Ph-N); 6,40 (s, 2H, Ph); 5,11 (s large, 2H, 2 x OH); 3,65 (m, 4H, pipérazine); 3,29 (m, 4H, pipérazine).
IR : $\nu_{OH}$ : 3399 $cm^{-1}$ ; $\nu_{C=O}$ (amide) : 1696 $cm^{-1}$ ; $\nu_{C=N}$ (amidine) : 1588 $cm^{-1}$.

**Exemple 21 : chlorhydrate de N-[3,5-bis-(1,1-diméthyléthyl)-4-hydroxyphényl]-N'-{{4-[(2-thiényl(imino)méthyl) amino]phényl}carbonylami no}-urée : 21**

**21.1) N-[3,5-bis-(1,1-diméthyléthyl)-4-hydroxyphényl]-N'-[(4-nitrophényl)-carbonylamino]-urée :**

Sous atmosphère d'argon, dans un tricol de 50 ml équipé d'une ampoule à addition, on dissout 0,22 g (0,73 mmole) de triphosgène à 20 °C. On additionne goutte à goutte à ce mélange, en une heure, une solution de 0,44 g (2 mmoles) de 4-amino-2,6-bis-(1,1-diméthyléthyl)-phénol (intermédiaire 17.1) et de 0,38 ml (2,2 mmoles) de diisopropyléthylamine dans 7 ml de dichlorométhane anhydre. Cinq minutes après la fin de l'addition, on ajoute en une seule portion une solution de 0,36 g (2 mmoles) de 4-nitrobenzoyl-hydrazide et de 0,38 ml (2,2 mmoles) de diisopropyléthylamine dans 4 ml de DMF anhydre. Après quatre heures d'agitation à 20 °C, le mélange réactionnel est concentré à sec sous pression réduite. Le résidu d'évaporation est dilué dans 40 ml d'acétate d'éthyle et la solution organique est lavée successivement par 3 fois 20 ml d'eau et 20 ml d'une solution saturée de chlorure de sodium. Après séchage sur sulfate de sodium, la solution organique est filtrée et le filtrat concentré à sec sous pression réduite. Le résidu obtenu est suspendu dans de l'heptane, agité et filtré pour conduire à une poudre jaune avec un rendement de 86 %. Point de fusion : 163-164 °C.
RMN $^1$H (100 MHz, DMSO d6, $\delta$) : 10,65 (s large, 1H, NH amide) ; 8,72 (s, 1H, NH-Ph) ; 8,38 (m, 4H, Ph-$NO_2$) ; 8,20 (s, 1H, CO-NH-NH) ; 7,36 (s, 2H, Ph-OH) ; 6,78 (s, 1H, OH) ; 1,50 (s, 18H, 2 x tBu).

**21.2) N-[(4-aminophényl)carbonylamino]-N'-[3,5-bis-(1,1-diméthyléthyl)-4-hydroxyphényl]-urée :**

Dans une bouteille de Parr de 250 ml, 0,72 g (1,68 mmoles) de N-[3,5-bis-(1,1-diméthyléthyl)-4-hydroxyphényl]-N'-[(4-nitrophényl)carbonylamino]-urée sont dissous dans 30 ml d'éthanol absolu en présence de Pd/C à 10 %. Le mélange est agité sous 20 PSI d'hydrogène, à 30 °C, pendant deux heures. Après filtration sur célite, le filtrat est concentré sous vide. Le résidu d'évaporation est suspendu dans de l'éther diéthylique (20 ml) agité et filtré pour conduire à une poudre jaune pâle avec un rendement de 75 %. Point de fusion : 245-246 °C.

RMN $^1H$ (100 MHz, DMSO d6, δ) : 9,84 (s large, 1H, NH amide) ; 8,56 (s, 1H, NH-Ph) ; 7,85 (m, 2H, Ph-$NH_2$) ; 7,74 (s, 1H, CO-NH-NH) ; 7,38 (s, 2H, Ph-OH) ; 6,78 (s, 1H, OH) ; 6,60 (m, 2H, Ph-$NH_2$) ; 5,80 (s large, 2H, $NH_2$) ; 1,50 (s, 18H, 2 x tBu).

**21.3) chlorhydrate de N-[3,5-bis-(1,1-diméthyléthyl)-4-hydroxyphényl]-N'-{{4-[(2-thiényl(imino)méthyl)amino] phényl}carbonylamino}-urée : 21**

Le protocole expérimental utilisé est le même que celui décrit pour le composé **1**, la N-[(4-aminophényl) carbonylamino]-N'-[3,5-bis-(1,1-diméthyléthyl)-4-hydroxyphényl]-urée remplaçant le 3,5-bis-(1,1-diméthyléthyl)-4-hydroxy-N-(4-aminophényl)-benzamide. La base libre est purifiée sur colonne de silice (éluant : heptane/acétate d'éthyle : 1/1). Les fractions pures sont collectées et concentrées sous pression réduite. Le résidu d'évaporation est dilué dans 15 ml d'acétone et salifié par une solution molaire de HCl dans de l'éther anhydre, comme décrit précédemment. On obtient finalement 0,40 g (58 %) d'une poudre jaune. Point de fusion : 254-255 °C.
RMN $^1H$ (400 MHz, DMSO, δ) : 11,68 (s large, 1H, $NH^+$) ; 10,32 (s, 1H, NH amide) ; 9,94 (s large, 1H, $NH^+$) ; 9,13 (s large, 1H, $NH^+$) ; 8,68 (s, 1H, NH-CO); 8,18 (m, 2H, thiophène) ; 8,07 (m, 3H, CO-NH-NH + Ph-NH) ; 7,58 (m, 2H, Ph-NH) ; 7,39 (m, 1H, thiophène) ; 7,22 (s, 2H, Ph-OH) ; 6,60 (s, 1H, OH); 1,36 (s, 18H, 2 x tBu).
IR: $\nu_{OH}$: 3627 $cm^{-1}$; $\nu_{C=O}$ (amide), $\nu_{C=O}$ (urée) : 1654 $cm^{-1}$, 1602 $cm^{-1}$ ; $\nu_{C=N}$ (amidine): 1559 $cm^{-1}$.

**Exemple 22 : chlorhydrate de N-[3,5-bis-(1,1-diméthyléthyl)-4-hydroxyphényl]-N'-{{4-[(2-thiényl(imino)méthyl) amino]phényl}méthyl}-thiourée : 22**

**22.1) N-[3,5-bis-(1,1-diméthyléthyl)-4-hydroxyphényl]-N'-[(4-nitrophényl)méthyl]-thiourée**

Le composé 22.1 est obtenu par l'action du réactif de Lawesson sur l'intermédiaire 17.4 selon un protocole expérimental décrit dans la littérature (*J. Med. Chem.* (1995), **38** (18), 3558-3565). On obtient une poudre jaune claire avec un rendement de 80 %. Point de fusion : 218-220 °C.
RMN $^1H$ (100 MHz, $CDCl_3$, δ) : 7,85 (m, 4H, Ph-$NO_2$) ; 7,70 (s, 1H, NH-Ph) ; 7,05 (s, 2H, Ph-OH) ; 6,21 (m, 1H, NH-$CH_2$) ; 5,40 (s, 1H, OH) ; 5,00 (d, 2H, $CH_2$, J = 6,5 Hz) ; 1,41 (s, 18H, 2 x tBu).

**22.2) N-[(4-aminophényl)méthyl]-N'-[3,5-bis-(1,1-diméthyléthyl)-4-hydroxyphényl]-thiourée :**

Le protocole expérimental utilisé est le même que celui décrit pour l'intermédiaire 18.2, l'intermédiaire 22.1 remplaçant le 3-[(3,5-bis-(1,1-diméthyléthyl)-4-hydroxyphényl]-N-(4-hydroxy-3-nitrophényl)-2-propènamide. On obtient une poudre beige avec un rendement de 70 %. Point de fusion : 167-169 °C.
RMN $^1H$ (100 MHz, $CDCl_3$, δ) : 7,48 (s large, 1H, NH-Ph) ; 6,95 (s, 2H, Ph-OH) ; 6,81 (m, 4H, Ph-$NH_2$) ; 5,98 (m, 1H, NH-$CH_2$) ; 5,28 (s, 1H, OH) ; 4,69 (d, 2H, $CH_2$, J = 5,5 Hz) ; 3,62 (s large, 2H, $NH_2$) ; 1,40 (s, 18H, 2 x tBu).

**22.3) chlorhydrate de N-[3,5-bis-(1,1-diméthyléthyl)-4-hydroxyphényl]-N'-{{4-[(2-thiényl(imino)méthyl)amino] phényl}méthyl}-thiourée : 22**

Le protocole expérimental utilisé est le même que celui décrit pour l'intermédiaire 17.6, l'intermédiaire 22.2 remplaçant la N-[(4-aminophényl)méthyl]-N'-[3,5-bis-(1,1-diméthyléthyl)-4-hydroxyphényl]-urée. On obtient une poudre jaune pâle avec un rendement de 15 %. Point de fusion : 203-205 °C.
RMN $^1H$ (400 MHz, DMSO d6, δ) : 11,52 (s large, 1H, $NH^+$) ; 9,86 (s large, 1H, $NH^+$) ; 8,98 (s large, 1H, $NH^+$) ; 8,39 (s, 1H, NH-Ph) ; 8,16 (m, 2H, thiophène); 7,46 (m, 6H, Ph-N, thiophène, NH-$CH_2$) ; 7,18 (s, 2H, Ph); 6,92 (s, 1H, OH); 4,80 (s large, 2H, $CH_2$) ; 1,35 (s, 18H, 2 x tBu).
IR: $\nu_{OH}$ : 3630 $cm^{-1}$ ; $\nu_{C=O}$ (urée) : 1649 $cm^{-1}$ ; $\nu_{C=N}$ (amidine): 1600 $cm^{-1}$.

**Exemple 23 : chlorhydrate de N-[3,5-bis-(1,1-diméthyléthyl)-4-hydroxyphényl]-N'-{2-{4-[(2-thiényl(imino)méthyl)amino]phényl}éthyl}-urée : 23**

**23.1) N-[3,5-bis-(1,1-diméthyléthyl)-4-hydroxyphényl]-N'-[2-(4-nitrophényl)éthyl]-urée**

Le protocole expérimental utilisé est le même que celui décrit pour l'intermédiaire 21.1, la 4-nitrophénétylamine remplaçant le 4-nitrobenzoyl-hydrazide. On obtient une poudre beige avec un rendement de 80 %. Point de fusion : 185-187 °C.
RMN $^1H$ (100 MHz, $CDCl_3$, δ) : 7,75 (m, 4H, Ph-$NO_2$) ; 7,00 (s, 2H, Ph-OH) ; 6,05 (s, 1H, OH) ; 5,18 (s, 1H, NH) ; 4,68 (m, 1H, NH-$CH_2$) ; 3,50 (m, 2H, NH-$CH_2$) ; 2,92 (m, 2H, $CH_2$) ; 1,40 (s, 18H, 2 x tBu).

**23.2) N-[2-(4-aminophényl)éthyl]-N'-[3,5-bis-(1,1-diméthyléthyl)-4-hydroxyphényl]-urée :**

Le protocole expérimental utilisé est le même que celui décrit pour l'intermédiaire 21.2, l'intermédiaire 23.1 remplaçant la N-[3,5-bis-(1,1-diméthyléthyl)-4-hydroxyphényl]-N'-[(4-nitrophényl)-carbonylamino]-urée. On obtient une poudre blanche avec un rendement de 56 %. Point de fusion : 192-194 °C.
RMN $^1H$ (100 MHz, DMSO d6, δ): 8,25 (s large, 1H, Ph-NH-CO) ; 7,22 (s, 2H, Ph-OH) ; 6,79 (m, 4H, Ph-$NH_2$) ; 6,65 (s, 1H, OH); 5,92 (m, 1H, NH-$CH_2$) ; 4,98 (s large, 2H, -$NH_2$) ; 3,31 (m, 2H, NH-$CH_2$) ; 2,65 (m, 2H, $CH_2$) ; 1,48 (s, 18H, 2 x tBu).

**23.3) chlorhydrate de N-[3,5-bis-(1,1-diméthyléthyl)-4-hydroxyphényl]-N'-{2-{4-[(2-thiényl(imino)méthyl)amino] phényl}éthyl}-urée : 23**

Le protocole expérimental utilisé est le même que celui décrit pour le composé **1,** l'intermédiaire 23.2 remplaçant le 3,5-bis-(1,1-diméthyléthyl)-4-hydroxy-N-(4-aminophényl)-benzamide. La base libre est purifiée sur colonne de silice (éluant : heptane/acétate d'éthyle : 1/1). Les fractions pures sont collectées et concentrées sous pression réduite. Le résidu d'évaporation est dilué dans 15 ml d'acétone et salifié par une solution molaire d'HCl dans l'éther anhydre, comme décrit précédemment. On obtient finalement 0,25 g (24 %) d'une poudre jaune pâle. Point de fusion : 207-210 °C.
RMN $^1H$ (400 MHz, DMSO d6, δ) : 11,48 (s large, 1H, $NH^+$) ; 9,83 (s large, 1H, $NH^+$); 8,95 (s large, 1H, $NH^+$) ; 8,50 (s, 1H, NH-CO) ; 8,18 (m, 2H, thiophène) ; 7,38 (m, 5H, Ph-NH + thiophène); 7,18 (s, 2H, Ph-OH) ; 6,55 (s, 1H, OH) ; 6,21 (m, 1H, CO-NH-$CH_2$) ; 3,35 (m, 2H, NH-$CH_2$) ; 2,78 (m, 2H, $CH_2$) ; 1,36 (s, 18H, 2 x tBu).
IR: $\nu_{OH}$ : 3631 $cm^{-1}$ ; $\nu_{C=O}$ (urée) : 1654 $cm^{-1}$, 1600 $cm^{-1}$ ; $\nu_{C=N}$ (amidine) : 1560 $cm^{-1}$.

**Exemple 24 : chlorhydrate de N-(4-{4-[(3,4-dihydro-6-méthoxy-2,5,7,8-tétraméthyl-2H-1-benzopyran-2-yl)carbonyl]-1-pipérazinyl}phényl)-2-thiophènecarboximidamide : 24**

**24.1) 1-{[3,4-dihydro-6-méthoxy-2,5,7,8-tétraméthyl-2H-1-benzopyran-2-yl]carbonyl}-4-(4-nitrophényl) pipérazine :**

Le protocole expérimental est identique à celui décrit pour l'intermédiaire 13.1, l'acide (±)-3,4-dihydro-6-méthoxy-2,5,7,8-tétraméthyl-2H-1-benzopyran-2-carboxylique (préparé selon *CHIMIA* (1991), **45** (4), 121-3) remplaçant le (±)-Trolox®. On obtient une poudre jaune.
RMN $^1H$ (100MHz, $CDCl_3$, δ) : 7,45 (m, 4H, Ph) ; 3,60 (s, 3H, $CH_3O$) ; 3,40 (m, 4H, pipérazine) ; 3,00 (m, 4H, pipérazine) ; 2,50-1,60 (m, 16H, Trolox®).

**24.2) 1-{[3,4-dihydro-6-méthoxy-2,5,7,8-tétraméthyl-2H-1-benzopyran-2-yl]carbonyl}-4-(4-aminophényl) pipérazine :**

Le protocole expérimental est identique à celui décrit pour l'intermédiaire 13.2, l'intermédiaire 24.1 remplaçant le 3,4-dihydro-2,5,7,8-tétraméthyl-2-{4-[(4-nitrophényl)-1-pipérazinyl]-carbonyl }-2H-1-benzopyran-6-ol. On obtient une huile qui est investie directement dans l'étape suivante.
RMN $^1H$ (100MHz, $CDCl_3$, δ) : 6,70 (m, 4H, Ph) ; 3,90 (d large, 4H, pipérazine); 3,60 (s, 3H, $CH_3O$) ; 3,45 (s large, 2H, $NH_2$) ; 2,90 (m, 4H, pipérazine); 2,60-1,60 (m, 18H, Trolox®).

**24.3) chlorhydrate de N-(4-{4- [(3,4-dihydro-6-méthoxy-2,5,7,8-tétraméthyl-2H-1-benzopyran-2-yl)carbonyl]-1-pipérazinyl}phényl)-2-thiophènecarboximidamide : 24**

Le protocole expérimental est le même que celui décrit pour le composé 13, l'intermédiaire 24.2 remplaçant le 3,4-dihydro-2,5,7,8-tétraméthyl-2-{4-[(4-aminophényl)-1-pipérazinyl]-carbonyl}-2H-1-benzopyran-6-ol. On obtient une poudre jaune pâle. Point de fusion : 190-195 °C.
RMN $^1H$ (400MHz, DMSO, δ) : 11,35 (s large, 1H, $NH^+$) ; 9,70 (s large, 1H, $NH^+$) ; 8,70 (s large, 1H, $NH^+$) ; 8,15 (s large, 2H, thiophène) ; 7,35 (s large, 1H, thiophène) ; 7,17 (m, 4H, Ph) ; 3,90 (d large, 4H, pipérazine) ; 3,50 (s, 3H, $CH_3O$) ; 3,15 (m, 4H, pipérazine) ; 2,55-1,55 (m, 16H, Trolox®).
IR : $\nu_{C=O}$ (amide) : 1642 $cm^{-1}$ ; $\nu_{C=N}$ (amidine) : 1618 $cm^{-1}$.

**Exemple 25 : chlorhydrate du N-[4-{4-[(3,4-dihydro-6-hydroxy-2,5,7,8-tétraméthyl-2H-1-benzopyran-2-yl)carbonyl]-1H-1,4-diazépin-1-yl}phényl]-2-thiophènecarboximidamide : 25**

**25.1) hexahydro-4-(4-nitrophényl)-1H-1,4-diazépine :**

A une solution de 2,44 g (12,2 mmoles) d'hexahydro-1H-1,4-diazépine-1-carboxylate de (1,1-diméthyl) éthyle dans 50 ml de DMF, on ajoute 3,37 g (24,4 mmoles) de carbonate de potassium et 1,89 g (13,4 mmoles) de 4-nitrofluorobenzène. Le mélange réactionnel est chauffé à 100 °C pendant 16 heures. Après refroidissement, 25 ml d'acétate d'éthyle et 50 ml d'eau sont ajoutés. La solution organique est décantée et la phase aqueuse extraite par 3 fois 50 ml d'acétate d'éthyle. Les phases organiques sont regroupées et lavées par 50 ml de saumure, séchées sur sulfate de sodium, filtrées et concentrées sous vide. On obtient 3,7g d'un solide jaune vif avec un rendement de 95 %. Ce solide est ensuite dissous dans 100 ml d'un mélange de solvants (dichlorométhane/acétate d'éthyle 1:1) auquel on ajoute goutte à goutte, à 0 °C, 20 ml d'une solution aqueuse (6N) d'acide chlorhydrique. Après agitation vigoureuse, à 20 °C, pendant 1 heure, le mélange réactionnel est décanté. La phase aqueuse est basifiée à pH = 11 par de la soude 4N et extraite par 3 fois 50 ml de dichlorométhane. Les phases organiques sont regroupées, lavées par 50 ml d'eau suivi de 50 ml de saumure, séchées sur sulfate de sodium et finalement filtrées et concentrées sous vide. On obtient 1,78 g d'une poudre jaune vif avec un rendement de 66 %. Le produit est utilisé directement dans l'étape suivante sans purification supplémentaire.
RMN $^1H$ (100MHz, $CDCl_3$, δ) : 8,10 (m, 2H, Ph) ; 6,65 (m, 2H, Ph); 3,70 (q, 4H, $CH_2N$, J = 5,2 Hz); 3,10 (t, 2H, $CH_2N$) ; 2,85 (t, 2H, $CH_2N$) ; 1,95 (q, 2H, C-$CH_2$-C); 1,65 (s large, 1H NH).

**25.2) 1-[(3,4-dihydro-6-hydroxy-2,5,7,8-tétraméthyl-2H-1-benzopyran-2-yl)carbonyl] hexahydro-4-(4-nitrophényl)-1H-1,4-diazépine :**

Dans un ballon de 50 ml, on ajoute 0,71 g (4,4 mmoles) de 1,1'-carbonyldiimidazole à une solution de 1,07 g (4,3 mmoles) de (±)-Trolox® dans 8 ml de THF anhydre. Après une heure d'agitation à 20 °C on ajoute goutte à goutte une solution de 0,95 g (4,3 mmoles) de l'intermédiaire 25.1 dans 4 ml de DMF. Le mélange réactionnel est agité pendant 16 heures à 20 °C. Après évaporation des solvants sous vide, le résidu est repris par 30 ml d'un mélange de solvants (dichlorométhane/eau 1:2). Après décantation, la phase organique est lavée par 2 fois 20 ml d'eau, séchée sur sulfate de sodium et concentrée sous vide. On obtient une poudre jaune pâle avec un rendement brut de 97 %. Le produit est utilisé directement dans l'étape suivante sans purification supplémentaire.
RMN $^1H$ (100MHz, $CDCl_3$, δ) : 8,10 (m, 2H, Ph) ; 6,60 (m, 2H, Ph) ; 4,40 (s large, 1H, OH) ; 3,50 (m, 8H, $CH_2N$) ; 2,50-1,50 (m, 18H, Trolox® + $CH_2$).

**25.3) 1-(4-aminophényl)-4-[(3,4-dihydro-6-hydroxy-2,5,7,8-tétraméthyl-2H-1-benzopyran-2-yl)carbonyl] hexahydro-1H-1,4-diazépine :**

Le protocole expérimental utilisé est le même que celui décrit pour l'intermédiaire 13.2, l'intermédiaire 25.2 remplaçant le 3,4-dihydro-2,5,7,8-tétraméthyl-2-{4-[(4-nitrophényl)-1-pipérazinyl]-carbonyl}-2H-1-benzopyran-6-ol. Le produit de la réaction est purifié sur une colonne de gel de silice (éluant : acétate d'éthyle/éther de pétrole 3 : 2). On obtient une huile avec un rendement de 57 %.

**25.4) chlorhydrate du N-[4-{4-[(3,4-dihydro-6-hydroxy-2,5,7,8-tétraméthyl-2H-1-benzopyran-2-yl)carbonyl]-1H-1,4-diazépin-1-yl}phényl]-2-thiophènecarboximidamide :**

On chauffe à 50 °C, pendant 40 heures, un mélange de 0,52 g (1,22 mmole) de l'intermédiaire 25.3 et de 0,35 g (1,22 mmole) d'iodhydrate de S-méthyl-2-thiophène thiocarboximide dans 4 ml d'isopropanol. Le mélange réactionnel est ensuite filtré et le solide obtenu est repris dans 4 ml d'une solution aqueuse saturée de carbonate de sodium et 4 ml d'acétate d'éthyle. L'ensemble est chauffé à 50 °C pendant 30 minutes, puis décanté. La phase organique est lavée 2 fois par 10 ml d'eau suivi de 10 ml de saumure. Les phases organiques sont regroupées, séchées sur sulfate de sodium, filtrées et concentrées sous pression réduite. Le solide obtenu est purifié sur une colonne de gel de silice (éluant : acétate d'éthyle/éther de pétrole 5 :1). On obtient 0,5 g de produit avec un rendement de 77 %. 0,15 g (0,29 mmole) de ce produit sont ensuite dissous dans 2 ml d'acétone. On ajoute goutte à goutte 0,84 ml (0,84 mmole) d'une solution 1N d'acide chlorhydrique dans de l'éther éthylique anhydre. L'ensemble est agité à température ambiante pendant 30 minutes. Il se forme un précipité jaune qui est filtré. Le précipité est trituré et lavé successivement par 3 fois 5 ml d'éther éthylique et 5 ml d'acétone. La poudre jaune foncé est séchée sous vide à 70 °C pendant 48 heures. Le rendement obtenu est de 80 %. Point de fusion : 180-185 °C.
RMN $^1H$ (400MHz, DMSO, δ) : 11,15 (s large, 1H, $NH^+$) ; 9,60 (s large, 1H, $NH^+$) ; 8,55 (s large, 1H, $NH^+$) ; 8,10 (s

large, 2H, thiophène) ; 7,35 (s large, 1H, thiophène) ; 7,02 (m, 4H, Ph) ; 4,80 (s large, 1H, OH) ; 3,70 (m, 8H, $CH_2N$) ; 2,50-1,40 (m, 18H, Trolox® + $CH_2$).
IR : $\nu_{OH}$ : 3412 $cm^{-1}$ ; $\nu_{C=O}$ (amide) : 1613 $cm^{-1}$; $\nu_{C=N}$ (amidine) : 1613 $cm^{-1}$.

**Exemple 26 : chlorhydrate de (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 26**

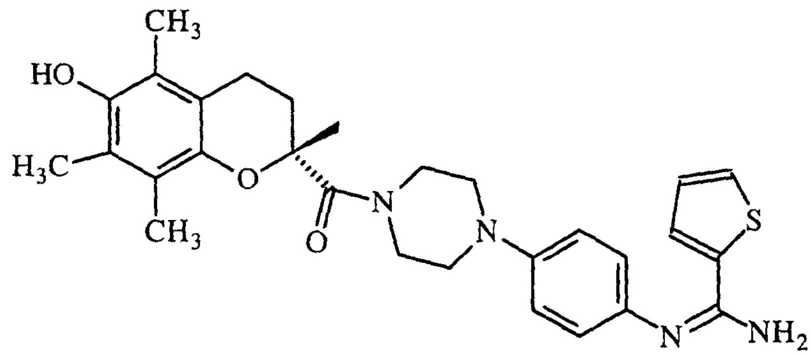

**26.1) (*R*)-3,4-dihydro-2,5,7,8-tétraméthyl-2-{4-[(4-nitrophényl)-1-pipérazinyl]-carbonyl}-2H-1-benzopyran-6-ol :**

Le protocole expérimental utilisé est le même que celui décrit pour le composé 13.1, le (*R*)-Trolox® remplaçant le (±)Trolox®. On obtient une poudre jaune vif avec un rendement de 98%. Point de fusion : 102-105 °C.

**26.2) (*R*)-3,4-dihydro-2,5,7,8-tétraméthyl-2-{4-[(4-aminophényl)-1-pipérazinyl]-carbonyl}-2H-1-benzopyran-6-ol :**

Le protocole expérimental utilisé est le même que celui décrit pour l'Intermédiaire 2.2, l'intermédiaire 26.1 remplaçant le 3,5-bis-(1,1-diméthyléthyl)-4-hydroxy-N-[(4-nitrophényl)méthyl]-benzamide. On obtient une poudre rose avec un rendement de 75%. Le produit est utilisé tel quel dans l'étape suivante. Point de fusion : 103-105 °C.

**26.3) chlorhydrate de (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 26**

Le protocole expérimental utilisé est le même que celui décrit pour le composé **13**, l'intermédiaire 26.2 remplaçant le 3,4-dihydro-2,5,7,8-tétraméthyl-2-{4- [(4-aminophényl)-1-pipérazinyl]-carbonyl }-2H-1-benzopyran-6-ol. Le produit est obtenu sous forme d'une poudre jaune pâle qui s'hydrate à l'air. Point de fusion: 195-197 °C.
Les analyses RMN et IR sont identiques au composé **13.**

$$[\alpha]_D^{20} = -43,5\ ° \qquad (c=0,11 ; DMSO)$$

**Exemple 27 : dichlorhydrate de (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 27**

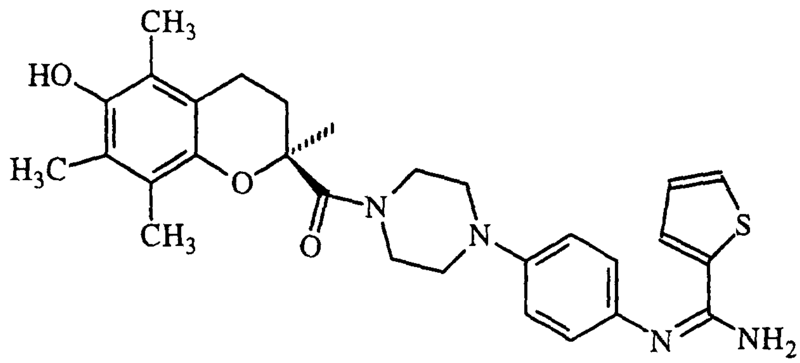

**27.1) (*S*)-3,4-dihydro-2,5,7,8-tétraméthyl-2-{4-[(4-nitrophényl)-1-pipérazinyl]-carbonyl}-2H-1-benzopyran-6-ol :**

Le protocole expérimental utilisé est le même que celui décrit pour le composé 13.1, le (*S*)-Trolox® remplaçant le (±)Trolox®. On obtient une poudre jaune avec un rendement de 73%. Point de fusion : 110-111 °C.

**27.2) (*S*)-3,4-dihydro-2,5,7,8-tétraméthyl-2-{4-[(4-aminophényl)-1-pipérazinyl]-carbonyl}-2H-1-benzopyran-6-ol :**

Le protocole expérimental utilisé est le même que celui décrit pour l'Intermédiaire 2.2, l'intermédiaire 27.1 remplaçant le 3,5-bis-(1,1-diméthyléthyl)-4-hydroxy-N-[(4-nitrophényl)méthyl]-benzamide. Après purification sur colonne de gel de silice (heptane/acétate d'éthyle : 2/8), collection et évaporation sous vide des fractions pures, on obtient une poudre beige avec un rendement de 54%. Point de fusion : 109-111 °C.

**27.3) dichlorhydrate de (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 27**

Le protocole expérimental utilisé est le même que celui décrit pour le composé **13**, l'intermédiaire 27.2 remplaçant le 3,4-dihydro-2,5,7,8-tétraméthyl-2-{4- [(4-aminophényl)-1-pipérazinyl]-carbonyl}-2H-1-benzopyran-6-ol. Le produit est obtenu sous forme d'une poudre jaune pâle qui s'hydrate à l'air. Point de fusion : 210,6-211,8 °C.
Les analyses RMN et IR sont identiques au composé **13.**

$$[\alpha]_D^{20} = +76{,}2\ ° \qquad (c=0{,}17\ ;\ DMSO)$$

Alternativement le composé **27** peut être préparé selon le protocole suivant :

**27.4) 2-thiophène carboximidate de méthyle :**

Dans un erlen de 250 ml purgé à l'argon, on introduit 10,91 g (0,1 mole) de 2-thiophène carbonitrile, 100 ml d'éther éthylique anhydre et 4,5 ml (0,11 mole) de méthanol. La solution est refroidie à 0 °C, à l'aide d'un bain de glace, et saturée par un courant d'HCl gazeux anhydre pendant 45 minutes. Le mélange réactionnel est agitée une heure supplémentaire à 0 °C et une nuit à 20 °C. Le précipité formé est filtré, lavé à l'éther éthylique et séché. Le chlorhydrate obtenu est partitionné dans un mélange de 100 ml d'eau et 150 ml d'éther éthylique. Le milieu est neutralisé par addition de 8,4 g (0,1 mole) de $NaHCO_3$ sec. Après décantation et séparation, la phase organique est lavée successivement par 2 x 30 ml d'eau et 30 ml de saumure. Après sèchage sur sulfate de magnésium, la solution organique est filtrée et concentrée sous vide. On obtient une huile incolore avec un rendement de 66 %.
RMN $^1H$ (400 MHz, $CDCl_3$, δ) : 7,58 (s large, 1H, =N-H) ; 7,42 (m, 1H, thiophène) ; 7,37 (m, 1H, thiophène) ; 7,01 (m, 1H, thiophène) ; 3,86 (s, 3H, $OCH_3$).
IR: $\nu_{C=N}$ (carboximidate) : 1630 $cm^{-1}$.

**27.5) dichlorhydrate de (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide : 27**

Dans un erlen de 150ml, sous courant d'argon, on dissout 8,2g (20 mmoles) de *(S)*-3,4-dihydro-2,5,7,8-tétraméthyl-2-{4-[(4-aminophényl)-1-pipérazinyl]-carbonyl}-2H-1-benzopyran-6-ol (obtenu comme l'intermédiaire 13.2 mais à partir du (*S*)-Trolox®) dans 60 ml de méthanol et on ajoute 4,2 g (30 mmoles) de 2-thiophène carboximidate de méthyle. Le mélange réactionnel est chauffé 18 heures à reflux. Le méthanol est évaporé sous vide et le résidu huileux brun est purifié sur une colonne de gel de silice (éluant : dichlorométhane/éthanol: 95/5). Les fractions pures sont collectées et concentrées sous vide pour conduire à une huile brune avec un rendement de 68 %. Cette huile est reprise par 22 ml d'une solution éthanolique de HCl (1,3N) et diluée par 180 ml d'acétone anhydre. Le mélange réactionnel est agité 1 heure à 0 °C. Le précipité formé est filtré et lavé successivement à l'acétone et à l'éther éthylique. On obtient, après séchage, le dichlorhydrate sous forme d'une poudre jaune pâle avec un rendement de 53 %.

**Exemple 28 : chlorhydrate de 3,5-bis-(1,1-diméthyléthyl)-4-hydroxy-N-{2-[3-[(2-thiényl(imino)méthyl)amino] phényl]éthyl}-benzamide : 28**

**28.1) 3,5-bis-(1,1-diméthyléthyl)-4-hydroxy-N-[2-(3-nitrophényl)éthyl]-benzamide**

Le protocole expérimental utilisé est le même que celui décrit pour l'intermédiaire 5.1, la 3-nitrophénétylamine (*J. Med. Chem.* (1968), **11** (1), 21-26) remplaçant la 4-nitrophénétylamine. On obtient une poudre blanche avec un rendement de 50 %. Point de fusion : 195-197 °C.
RMN $^1$H (100 MHz, $CDCl_3$, δ) : 7,86 (m, 4H, Ph-$NO_2$) ; 7,50 (s, 2H, Ph) ; 6,10 (m, 1H, NHCO) ; 5,54 (s , 1H, OH) ; 3,75 (m, 2H, $\underline{CH_2}$-NHCO) ; 3,08 (m, 2H, $\underline{CH_2}$-Ph-$NO_2$) ; 1,42 (s, 18H, 2 x tBu).

**28.2) 3,5-bis-(1,1-diméthyléthyl)-4-hydroxy-N-[2-(3-aminophényl)éthyl]-benzamide :**

Le protocole expérimental utilisé est le même que celui décrit pour l'intermédiaire 5.2, l'intermédiaire 28.1 remplaçant le 3,5-bis-(1,1-diméthyléthyl)-4-hydroxy-N-[2-(4-nitrophényl)éthyl]-benzamide. On obtient une poudre blanche (rendement de 40 %) suffisamment pure pour être investie directement dans l'étape suivante.

**28.3) chlorhydrate de 3,5-bis-(1,1-diméthyléthyl)-4-hydroxy-N-{2-[3-[(2-thiényl(imino)méthyl)-amino]phényl] éthyl}-benzamide : 28**

Le protocole expérimental utilisé est le même que celui décrit pour l'intermédiaire 1.3, l'intermédiaire 28.2 remplaçant le 3,5-bis-(1,1-diméthyléthyl)-4-hydroxy-N-(4-aminophényl)-benzamide. On obtient une poudre jaune pâle avec un rendement de 35 %. Point de fusion : 205-207 °C.
RMN $^1$H (400 MHz, DMSO d6, δ) : 11,59 (s large, 1H, $NH^+$) ; 9,89 (s, 1H, $NH^+$) ; 8,95 (s, 1H, $NH^+$) ; 8,46 (s, 1H, CONH) ; 8,17 (m, 2H, thiophène) ; 7,54 (s, 2H, $\underline{Ph}$-OH) ; 7,39 (m, 6H, thiophène, $\underline{Ph}$-NH, OH) ; 3,51 (m, 2H, $\underline{CH_2}$-NHCO) ; 2,89 (m, 2H, $\underline{CH_2}$-Ph-NH) ; 1,38 (s, 18H, 2 x tBu).
IR : $\nu_{OH}$ : 3624 $cm^{-1}$ ; $\nu_{C=O}$ (amide) : 1631 $cm^{-1}$ ; $\nu_{C=N}$ (amidine) : 1577 $cm^{-1}$.

**Exemple 29 : chlorhydrate du N-{4-(4-[2-(3,5-bis-(1,1-diméthyléthyl)-4-hydroxyphényl)-1-oxo-éthyl]-1-pipérazinyl)phényl}-2-thiophène-carboximidamide : 29**

Le protocole expérimental utilisé est le même que celui décrit pour le composé **9**, l'acide 3,5-di-*tert*-butyl-4-hydroxyphénylacétique remplaçant l'acide 3,5-di-*tert*-butyl-4-hydroxybenzoïque dans la première étape de synthèse. Poudre jaune. Point de fusion: 176-180 °C.
RMN $^1$H (400 MHz, DMSO d6, δ) : 11,30 (s large, 1H, $NH^+$) ; 9,70 (s large, 1H, $NH^+$) ; 8,65 (s large, 1H, $NH^+$) ; 8,10 (s large, 2H, thiophène) ; 7,35 (s large, 1H, thiophène); 7,12 (m, 4H, Ph-N) ; 6,95 (s, 2H, $\underline{Ph}$-OH) ; 6,80 (s large, 1H, OH); 3,60 (s large, 6H, pipérazine, $CH_2CO$); 3,10 (m, 4H, pipérazine) ; 1,35 (s, 18H, 2x tBu).
IR: $\nu_{OH}$ : 3620 $cm^{-1}$ ; $\nu_{C=O}$ (ester) : 1638 $cm^{-1}$ ; $\nu_{C=N}$ (amidine) : 1612 $cm^{-1}$.

**Exemple 30 : chlorhydrate de 3,5-bis-(1,1-diméthyléthyl)-4-hydroxy-benzoate de 2-{4-[(2-thiényl(imino)méthyl) amino]phényl}éthyle : 30**

**30.1) 3,5-bis-(1,1-diméthyléthyl)-4-hydroxybenzoate de 2-(4-nitrophényl)éthyle :**

Dans un ballon de 250 ml contenant 80 ml de THF, sous atmosphère d'argon, on introduit successivement,

sous agitation, 2,45 g (9,8 mmoles) d'acide 3,5-di-*tert*-butyl-4-hydroxybenzoïque, 1,8 g (10,8 mmoles) de 4-nitrobenzène-éthanol et 2,2 g (10,8 mmoles) de dicyclohexylcarbodiimide. Le mélange réactionnel est agité pendant 15 heures à 20 °C et le précipité apparu est filtré. Le filtrat est lavée par 2 x 30 ml d'une solution saturée de NaCl, la phase organique est séchée sur sulfate de magnésium, filtrée et concentrée sous vide. Le résidu est ensuite cristallisé à l'aide d'éther di-isopropylique. Le solide est récupéré par filtration et on obtient 2,4 g (62%) de cristaux blancs après séchage. Point de fusion : 123,5-124,5 °C.
RMN $^1H$ (100 MHz, $CDCl_3$, δ) : 7,85 (m, 4H, Ph-$NO_2$) ; 7,80 (s, 2H, <u>Ph</u>-OH) ; 5,70 (s, 1H, OH) ; 4,50 (m, 2H, O-$CH_2$) ; 3,20 (m, 2H, $CH_2$) ; 1,40 (s , 18H, 2x tBu).

**30.2) 3,5-bis-(1,1-diméthyléthyl)-4-hydroxy-benzoate de 2-(4-aminophényl)éthyle :**

Le protocole expérimental est le même que celui décrit pour l'intermédiaire 2.2, l'intermédiaire 30.1 remplaçant le 3,5-bis-(1,1-diméthyléthyl)-4-hydroxy-N-[(4-nitrophényl)-méthyl]-benzamide. On obtient une poudre blanche avec un rendement de 50 %. Point de fusion: 135-136 °C.
RMN $^1H$ (400 MHz, DMSO d6, δ) : 7,75 (s, 2H, <u>Ph</u>-OH) ; 6,70 (m, 4H, Ph-N) ; 4,90 (s large, 1H, OH) ; 4,25 (m, 2H, O-$CH_2$) ; 3,30 (s large, 2H, $NH_2$) ; 2,80 (m, 2H, $CH_2$) ; 1,40 (s, 18H, 2 x tBu).

**30.3) chlorhydrate de 3,5-bis-(1,1-diméthyléthyl)-4-hydroxy-benzoate de 2-{4-[(2-thiényl(imino)méthyl)amino] phényl}éthyle : <u>30</u>**

Le protocole expérimental est le même que celui décrit pour l'intermédiaire 1.3, l'intermédiaire 30.2 remplaçant le 3,5-bis-(1,1-diméthyléthyl)-4-hydroxy-N-(4-aminophényl)-benzamide. On obtient un solide blanc avec un rendement de 26%. Point de fusion : 145-150 °C.
RMN $^1H$ (400 MHz, DMSO d6, δ) : 11,50 (s large, 1H, $NH^+$) ; 9,80 (s large, 1H, $NH^+$) ; 8,90 (s large, 1H, $NH^+$) ; 8,20 (s large, 2H, thiophène) ; 7,85 (s, 1H, OH) ; 7,75 (s, 2H, <u>Ph</u>-OH) ; 7,47 (m, 5H, Ph-N, thiophène) ; 4,41 (m, 2H, O-$CH_2$) ; 3,08 (m, 2H, $CH_2$) ;1,40 (s, 18H, 2 x tBu).
IR : $\nu_{C=O}$ (ester) : 1700 $cm^{-1}$ ; $\nu_{C=N}$ (amidine) : 1592 $cm^{-1}$.

**<u>Exemple 31</u> : chlorhydrate de 3,5-bis-(1,1-diméthyléthyl)-4-hydroxy-benzoate de 2-{3-[(2-thiényl(imino)méthyl) amino]phényl}éthyle : <u>31</u>**

Le protocole expérimental utilisé est le même que celui décrit pour le composé **<u>30</u>**, le 3-nitrobenzène-éthanol remplaçant le 4-nitrobenzène-éthanol dans la première étape de synthèse. Poudre jaune pâle. Point de fusion : 145-148 °C.
RMN $^1H$ (400 MHz, DMSO d6, δ) : 11,50 (s large, 1H, $NH^+$) ; 9,82 (s large, 1H, $NH^+$) ; 8,99 (s large, 1H, $NH^+$) ; 8,15 (m, 2H, thiophène) ; 7,81 (s, 1H, OH); 7,75 (s, 2H, <u>Ph</u>-OH) ; 7,41 (m, 5H, Ph-N, thiophène) ; 4,41 (m, 2H, O-$CH_2$) ; 3,08 (m, 2H, $CH_2$) ; 1,38 (s, 18H, 2 x tBu).
IR: $\nu_{OH}$ : 3620 $cm^{-1}$ ; $\nu_{C=O}$ (ester) : 1707 $cm^{-1}$ ; $\nu_{C=N}$ (amidine) : 1654 $cm^{-1}$.

**<u>Exemple 32</u> : chlorhydrate de 3,5-bis-(1,1-diméthyléthyl)-4-hydroxy-benzoate de 2-{2-[(2-thiényl(imino)méthyl) amino]phényl}éthyle : <u>32</u>**

Le protocole expérimental utilisé est le même que celui décrit pour le composé **<u>30</u>,** le 2-nitrobenzène-éthanol remplaçant le 4-nitrobenzène-éthanol dans la première étape de synthèse. Poudre beige. Point de fusion : 139-145 °C.
RMN $^1H$ (400 MHz, DMSO d6, δ) : 11,50 (s large, 1H, $NH^+$) ; 9,80 (s large, 1H, $NH^+$) ; 8,65 (s large, 1H, $NH^+$) ; 8,15 (m, 2H, thiophène) ; 7,80 (s, 1H, OH); 7,70 (s, 2H, <u>Ph</u>-OH) ; 7,60 (m, 1H, Ph) ; 7,45 (m, 3H, Ph) ; 7,35 (s, 1H, thiophène) ; 4,40 (m, 2H, O-$CH_2$) ; 3,00 (m, 2H, $CH_2$) ; 1,35 (s, 18H, 2 x tBu).
IR : $\nu_{C=O}$ (ester) : 1728 $cm^{-1}$ ; $\nu_{C=N}$ (amidine) : 1649 $cm^{-1}$.

**<u>Etude pharmacologique des produits de l'invention</u>**

<u>Etude des effets sur la NO synthase constitutive neuronale de cervelet de rat</u>

L'activité inhibitrice des produits de l'invention est déterminée par la mesure de leur effets sur la transformation par la NO synthase de la [$^3H$]L-arginine en [$^3H$]L-citrulline en accord avec la méthode modifiée de Bredt et Snyder (*Proc. Natl. Acad. Sci. USA*, (1990) **87:** 682-685). Des cervelets de rats Sprague-Dawley (300g - Charles River) sont rapidement prélevés, disséqués à 4°C et homogénéisés dans un volume de tampon d'extraction (HEPES 50 mM, EDTA 1 mM, pH 7.4, pepstatin A 10 mg/ml, leupeptine 10 mg/ml). Les homogénats sont ensuite centrifugés à 21000

g pendant 15 min à 4°C. Le dosage se fait dans des tubes à essai en verre dans lesquels sont distribués 100 µl de tampon d'incubation contenant 100 mM d'HEPES (pH 7,4), 2 mM d'EDTA, 2.5 mM de $CaCl_2$, 2 mM de dithiotréitol, 2 mM de NADPH réduit et 10 µg/ml de calmoduline. On ajoute 25 µl d'une solution contenant 100 nM de [$^3$H]L-arginine (Activité spécifique : 56.4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide. Les composés des exemples 6, 7, 13 et 14 décrits ci-dessus présentent une $CI_{50}$ inférieure à 3,5 µM.

Etude des effets sur la péroxidation lipidique du cortex cérébral de rat

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol.* (1990) **186** : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4°C. Le culot est conservé à -80°C. Le jour de l'expérience, le culot est remis en suspension à la concentration de 1 g/ 15 ml et centrifugé à 515 g pendant 10 minutes à 4° C. Le surnageant est utilisé immédiatement pour la détermination de la péroxidation lipidique. L'homogénat de cortex cérébral de rat (500 µl) est incubé à 37° C pendant 15 minutes en présence des composés à tester ou du solvant (10 µl). La réaction de péroxidation lipidique est initiée par l'ajout de 50 µl de $FeCl_2$ à 1 mM, d'EDTA à 1 mM et d'acide ascorbique à 4 mM. Après 30 minutes d'incubation à 37°C, la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll. *European J. Pharmacol.* (1995) **285,** 203-206). Les composés des exemples 5, 8, 9, 10, 12, 13, 14, 16, 17, 18, 19, 20, 21, 26 et 27 décrits ci-dessus présentent tous une $CI_{50}$ inférieure à 30 µM.

## Revendications

1. Produit **caractérisé en ce qu'**il répond à la formule générale **(I)**

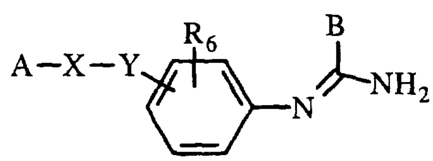

(I)

dans laquelle :

A représente :

soit un radical

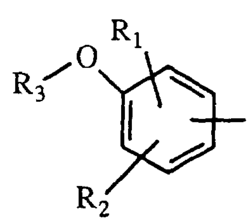

dans lequel $R_1$ et $R_2$ représentent, indépendamment, un atome d'hydrogène, un halogène, le groupe OH, un radical alkyle ou alkoxy linéaire ou ramifié ayant de 1 à 6 atomes de carbone,
$R_3$ représente un atome d'hydrogène, un radical alkyle linéaire ou ramifié ayant de 1 à 6 atomes de carbone ou un radical $-COR_4$,
$R_4$ représentant un radical alkyle linéaire ou ramifié ayant de 1 à 6 atomes de carbone,

soit un radical

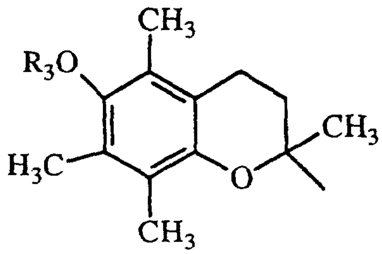

dans lequel $R_3$ a la signification indiquée ci-dessus

soit un radical

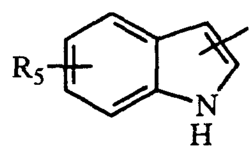

dans lequel $R_5$ représente un atome d'hydrogène, le groupe OH ou un radical alkyle ou alkoxy linéaire ou ramifié ayant de 1 à 6 atomes de carbone ;

B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;

X représente $-Z_1-$, $-Z_1-CO-$, -CH=CH-CO-, $-Z_1-NR_3-CO-$, $-Z_1-NR_3-CS-$, $-Z_1-NR_3-SO_2-$ ou une simple liaison ;

Y représente un radical choisi parmi les radicaux $-Z_2-Q$, pipérazine, homopipérazine, 2-méthylpipérazine, 2,5-diméthylpipérazine, 4-aminopipéridine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ ou $-S-Z_2-Q-$,
dans lesquels Q représente une simple liaison, $O-Z_3$ , $R_3-N-Z_3$ ou $S-Z_3$ ;

$Z_1$, $Z_2$ et $Z_3$ représentent indépendamment une simple liaison ou un radical alkylène linéaire ou ramifié ayant de 1 à 6 atomes de carbone, $Z_1$, $Z_2$ et $Z_3$ représentant de préférence $-(CH_2)_m-$, m étant un entier compris entre 0 et 6 ;

$R_6$ représente un atome d'hydrogène ou un groupe OH ;

ou un sel d'un produit de formule générale **(I),**
à l'exception toutefois du composé de formule suivante :

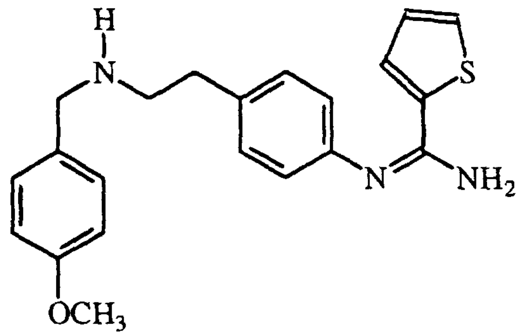

2. Produit selon la revendication 1, **caractérisé en ce qu'**il s'agit d'un des composés suivants :

- 3,5-bis-(1,1-diméthyléthyl)-4-hydroxy-N-{4-[(2-thiényl (imino)méthyl)amino]phényl }-benzamide;
- 3,5-bis-(1,1-diméthyléthyl)-4-hydroxy-N- {4-[[(2-thiényl (imino)méthyl)amino]phényl]méthyl}-benzamide;
- 4-acétoxy-3,5-diméthoxy-N- {4-[[(2-thiényl(imino)méthyl)amino]phényl]méthyl}-benzamide;
- 3,5-diméthoxy-4-hydroxy-N-{4-[[(2-thiényl(imino)méthyl)amino)phényl]méthyl}-benzamide;
- 3,5-bis-(1,1-diméthyléthyl)-4-hydroxy-N-{4-[2-[(2-thiényl-(imino)méthyl)amino]phényl]éthyl}-benzamide;
- 4-acétoxy-3,5-diméthoxy-N-{4-[2-[(2-thiényl-(imino)méthyl)-amino]phényl)éthyl}-benzamide;
- 3,5-diméthoxy-4-hydroxy-N-{4-[2-[(2-thiényl-(imino)méthyl)-amino]phényl]éthyl}-benzamide;
- 3,4,5-trihydroxy-N-{4-[2-[(2-thiényl(imino)méthyl)-amino]phényl]éthyl}-benzamide;
- N-{4-[4-[3,5-bis-(1,1-diméthyléthyl)-4-hydroxybenzoyl]-1-pipérazinyl]-phényl}-2-thiophènecarboximidamide;
- N-{4-[4-[3,5-bis-(1,1-diméthyléthyl)-4-hydroxybenzyl]-1-pipérazinyl]phényl}-2-thiophènecarboximidamide;
- N-{4-[4-[3,5-diméthoxy-4-hydroxybenzoyl]-1-pipérazinyl]-phényl}-2-thiophènecarboximidamide;
- 3,4-dihydro-6-hydroxy-2,5,7,8-tétraméthyl-N-{4-[(2-thiényl (imino)méthyl)amino]phényl}-2H-1-benzopyran-2-carboxamide;
- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- N-{4-[4-[(5-méthoxy-lH-indol-3-yl)méthylcarbonyl]-1-pipérazinyl]phényl}-2-thiophènecarboximidamide;
- N-[4-[4-[{3-[3,5-bis-(1,1-diméthyléthyl)-4-hydroxyphényl]-1-oxo-2-propényl}-1-pipérazinyl]-phényl]]-2-thiophènecarboximidamide;
- 3,5-bis-(1,1-diméthyléthyl)-4-hydroxy-N-{3-[[(2-thiényl-(imino)méthyl)amino]phényl]méthyl }-benzamide;
- N-[3,5-bis-(1,1-diméthyléthyl)-4-hydroxyphényl]-N'-{{4-[(2-thiényl(imino)méthyl)amino]phényl}méthyl}-urée;
- N-[5-[{3-(3,5-bis-(1,1-diméthyléthyl)-4-hydroxyphényl)-1-oxo-2-propényl )amino]-2-hydroxyphényl]-2-thiophènecarboximidamide;
- N-[3-[{3-(3,5-bis-(1,1-diméthyléthyl)-4-hydroxyphényl)-1-oxo-2-propényl}-amino]-4-hydroxyphényl]-2-thiophènecarboximidamide;
- N-{4-[4-[3,4,5-trihydroxybenzoyl]-1-pipérazinyl]phényl}-2-thiophènecarboximidamide;
- N-[3,5-bis-(1,1-diméthyléthyl)-4-hydroxyphényl]-N'-{{4-[(2-thiényl(imino)méthyl)amino]phényl}carbonylamino}-urée;
- N-[3,5-bis-(1,1-diméthyléthyl)-4-hydroxyphényl]-N'-{{4-[(2-thiényl(imino)méthyl)amino]phényl}méthyl}-thiourée;
- N-[3,5-bis-(1,1-diméthyléthyl)-4-hydroxyphényl]-N'-{2-{4-[(2-thiényl(imino)méthyl)amino]phényl}éthyl}-urée;
- N-(4-{4-[(3,4-dihydro-6-méthoxy-2,5,7,8-tétraméthyl-2H-1-benzopyran-2-yl) carbonyl]-1-pipérazinyl}phényl)-2-thiophènecarboximidamide;
- N-[4- {4-[(3,4-dihydro-6-hydroxy-2,5,7,8-tétraméthyl-2H-1-benzopyran-2-yl) carbonyl]-1H-1,4-diazépin-1-yl } phényl]-2-thiophènecarboximidamide;
- (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- 3,5-bis-(1,1-diméthyléthyl)-4-hydroxy-N-{2-[3- [(2-thiényl(imino)méthyl)amino] phényl]éthyl }-benzamide;
- N-{4-(4-[2-(3,5-bis-(1,1-diméthyléthyl)-4-hydroxyphényl)-1-oxo-éthyl]-1-pipérazinyl)phényl }-2-thiophènecarboximidamide;
- 3,5-bis-(1,1-diméthyléthyl)-4-hydroxy-benzoate de 2-{4-[(2-thiényl(imino)méthyl)amino]phényl}éthyle;

- 3,5-bis-(1,1-diméthyléthyl)-4-hydroxy-benzoate de 2-{3-[(2-thiényl(imino)méthyl)amino]phényl}éthyle;
- 3,5-bis-(1,1-diméthyléthyl)-4-hydroxy-benzoate de 2-{2-[(2-thiényl(imino)méthyl)amino]phényl}éthyle;

ou d'un des sels d'un de ces derniers, en particulier d'un chlorhydrate, dichlorhydrate, fumarate ou hémi-fumarate d'un de ces derniers.

**3.** Produit selon la revendication 1, **caractérisé en ce que** :

- X représente un radical alkylène linéaire ou ramifié ayant de 1 à 6 atomes de carbone et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$ ou $-NR_3-O-Z_2-$;
 ou
- X représente $-Z_1-CO-$ ou -CH=CH-CO- et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$, $-NR_3-O-Z_2-$, $-O-Z_2-Q-$ ou bien $-NR_3-CO-Q'-$ avec $Q' = R_3-N-Z_3$;
 ou
- X représente $-Z_1-NR_3-CO-$ et Y représente $-Z_2-Q-$, $-NH-Z_2-Q-$, -NH-CO-Z2-Q"- avec $Q" = O-Z_3-$, $R_3-N-Z_3-$ ou $S-Z_3-$, ou Y représente $-NR_3-SO_2-NR_3-Z_2-$ ou $-O-Z_2-Q-$;
 ou
- X représente $-Z_1-NH-CO-$ et Y représente un radical pipérazine, homopipérazine, 2-méthylpipérazine, 2,5-diméthylpipérazine, 4-aminopipéridine, $-NR_3-Z_2-Q-$, $-NR_3-NH-CO-Z_2-$, $-NH-NH-Z_2-$ ou $-NR_3-O-Z_2-$;
 ou
- X représente $-Z_1-NR_3-SO_2-$ et Y représente $-Z_2-Q"-$ avec $Q" = O-Z_3-$, $R_3-N-Z_3-$ ou $S-Z_3-$, ou bien Y représente $-NR_3-Z_2-Q-$;
 ou
- X représente $-Z_1-$ et Y représente $-O-CO-Z_2-Q-$;
 ou
- X représente $-Z_1-NR_3-CS-$ et Y représente $-NH-Z_2-Q-$, ou un radical pipérazine, homopipérazine, 2-méthylpipérazine, 2,5-diméthyl-pipérazine, 4-aminopipéridine, $-NR_3-Z_2-Q-$, $-NH-NH-Z_2-$ ou $-NR_3-O-Z_2-$;
 ou
- X représente une liaison et Y représente $-O-Z_2-NH-$, $-S-Z_2-NH-$.

**4.** Produit selon la revendication 1, 2 ou 3, **caractérisé en ce que** :

- A représente :

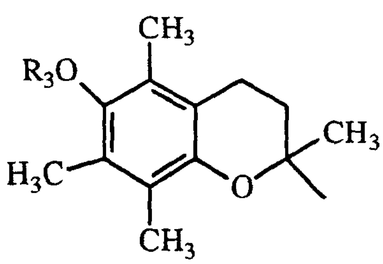

ou

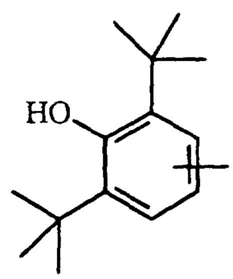

;

- X représente -CO- ou -NH-CO-;

- et Y représente un radical $-NH-Z_2-Q-$ ou pipérazine, Q représentant une liaison, $O-Z_3$ , $R_3-N-Z_3$ ou $S-Z_3$, et $Z_2$ et $Z_3$ représentant indépendamment une liaison ou un radical alkylène linéaire ou ramifié ayant de 1 à 6 atomes de carbone et $R_3$ représente un atome d'hydrogène ou un radical alkyle ayant de 1 à 6 atomes de carbone linéaire ou ramifié.

**5.** Produit selon la revendication 1 ou 2, **caractérisé en ce qu'**il s'agit d'un des composés suivants :

- 3,5-bis-(1,1-diméthyléthyl)-4-hydroxy-N-{4-[2-[2-thiényl-(iminométhyl)amino]phényl]éthyl}-benzamide;
- 3,4,5-trihydroxy-N-{4-[2-[2-thiényl(iminométhyl)-amino]phényl]éthyl}-benzamide;

- N-{4-[4-[3,5-bis-(1,1-diméthyléthyl)-4-hydroxybenzoyl]-1-pipérazinyl]phényl}-2-thiophènecarboximidamide;
- N-{4-[4-[3,5-bis-(1,1-diméthyléthyl)-4-hydroxybenzyl]-1-pipérazinyl]phényl}-2-thiophènecarboximidamide;
- 3,4-dihydro-6-hydroxy-2,5,7,8-tétraméthyl-N-{4-[2- thiényl (iminométhyl)amino]phényl}-2H-1-benzopyran-2-carboxamide;
- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- N-{4-[4-[(5 méthoxy-1H-indol-3-yl)méthylcarbonyl]-1-pipérazinyl]phényl}-2-thiophènecarboximidamide;
- 3,5-bis-(1,1-diméthyléthyl)-4-hydroxy-N-{3-[[2-thiényl-(iminométhyl)amino]phényl]méthyl}-benzamide;
- N-[3,5-bis-(1,1-diméthyléthyl)-4-hydroxyphényl]-N'-{{4-[2-thiényl(iminométhyl)amino]phényl}méthyl}-urée;
- N-[5-[{3-(3,5-bis-(1,1-diméthyléthyl)-4-hydroxyphényl)-1-oxo-2-propènyl}-amino]-2-hydroxyphényl]-2-thiophènecarboximidamide;
- N-[3-[{3-(3,5-bis-(1,1-diméthyléthyl)-4-hydroxyphényl)-1-oxo-2-propènyl}-arnino]-4-hydroxyphényl]-2-thiophènecarboximidamide;
- N-{4-[4-[3,4,5-trihydroxybenzoyl]-1-pipérazinyl]phényl}-2-thiophènecarboximidamide;
- N-[3,5-bis-(1,1-diméthyléthyl)-4-hydroxyphényl]-N'-{{4-[2-thiényl(iminométhyl)amino]phényl}carbonylamino}-urée ;
- (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;

ou d'un sel d'un de ces derniers, en particulier d'un chlorhydrate, dichlorhydrate, fumarate ou hémi-fumarate d'un de ces derniers.

6. Produit selon la revendication 1 ou 2, **caractérisé en ce qu'**il s'agit d'un des composés suivants :

- 4-acétoxy-3,5-diméthoxy-N-{4-[2-[2-thiényl-(iminométhyl)-amino]phényl]éthyl}-benzamide;
- 3,5-diméthoxy-4-hydroxy-N-{4-[2-[2-thiényl-(iminométhyl)-amino]phényl]éthyl}-benzamide ;

ou d'un sel de ces derniers, en particulier d'un chlorhydrate, dichlorhydrate, fumarate ou hémi-fumarate d'un de ces derniers.

7. Produit selon la revendication 1 ou 2, **caractérisé en ce qu'**il s'agit du N-{4-[4-[3,5-bis-(1,1-diméthyléthyl)-4-hydroxybenzyl]-1-pipérazinyl]phényl}-2-thiophènecarboximidamide ;
ou d'un sel de ce dernier, en particulier du chlorhydrate, du dichlorhydrate, du fumarate ou de l'hémi-fumarate de ce dernier.

8. Produit selon la revendication 1 ou 2, **caractérisé en ce qu'**il s'agit d'un des composés suivants :

- N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (R)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;
- (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide;

ou d'un sel d'un de ces derniers, en particulier d'un chlorhydrate, dichlorhydrate, fumarate ou hémi-fumarate d'un de ces derniers.

9. Produit selon la revendication 8, **caractérisé en ce qu'**il s'agit du (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide, ou d'un sel de ce dernier, en particulier d'un chlorhydrate, dichlorhydrate, fumarate ou hémi-fumarate de ce dernier.

10. A titre de produit industriel nouveau, produit **caractérisé en ce qu'**il répond à la formule générale **(II)A :**

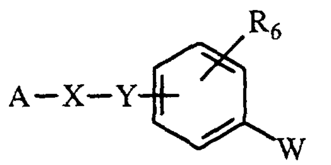

(II)A

dans laquelle :

W représente un radical amino ou nitro,

A représente :

soit un radical

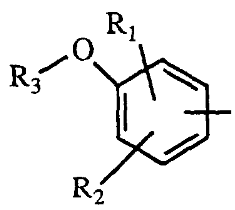

dans lequel $R_1$ et $R_2$ représentent, indépendamment, un atome d'hydrogène, un halogène, le groupe OH, un radical alkyle ou alkoxy linéaire ou ramifié ayant de 1 à 6 atomes de carbone,
$R_3$ représente un atome d'hydrogène, un radical alkyle linéaire ou ramifié ayant de 1 à 6 atomes de carbone ou un radical $-COR_4$,
$R_4$ représentant un radical alkyle linéaire ou ramifié ayant de 1 à 6 atomes de carbone,

soit un radical

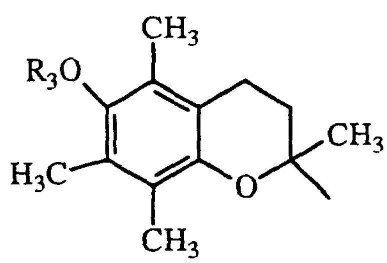

dans lequel $R_3$ a la signification indiquée ci-dessus

soit un radical

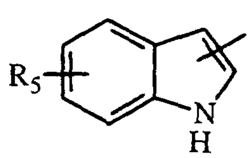

dans lequel $R_5$ représente un atome d'hydrogène, le groupe OH ou un radical alkyle ou alkoxy linéaire

ou ramifié ayant de 1 à 6 atomes de carbone ;

X représente $-Z_1-$, $-Z_1-CO-$, -CH=CH-CO-, $-Z_1-NR_3-CO-$, $-Z_1-NR_3-CS-$, $-Z_1-NR_3-SO_2-$ ou une simple liaison ;

Y représente un radical choisi parmi les radicaux $-Z_2-Q$, pipérazine, homopipérazine, 2-méthylpipérazine, 2,5-diméthylpipérazine, 4-aminopipéridine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ ou $-S-Z_2-Q-$,
dans lesquels Q représente une simple liaison, $O-Z_3$ , $R_3-N-Z_3$ ou $S-Z_3$ ;

$Z_1$, $Z_2$ et $Z_3$ représentent indépendamment une simple liaison ou un radical alkylène linéaire ou ramifié ayant de 1 à 6 atomes de carbone, $Z_1$, $Z_2$ et $Z_3$ représentant de préférence $-(CH_2)_m-$, m étant un entier compris entre 0 et 6 ;

$R_6$ représente un atome d'hydrogène ou un groupe OH ;

à l'exception toutefois du 3,5-bis-(1,1-diméthyléthyl)-4-hydroxy-N-(4-nitrophényl)-benzamide ;
ou un sel d'un produit de formule générale **(II)A,**
à l'exception toutefois des composés correspondant aux formules suivantes :

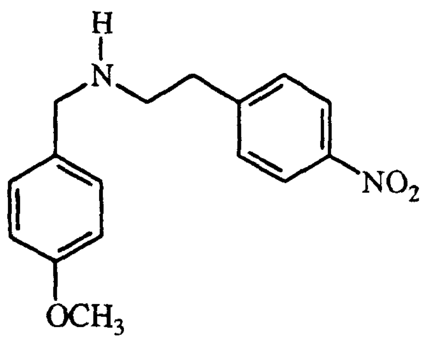

et

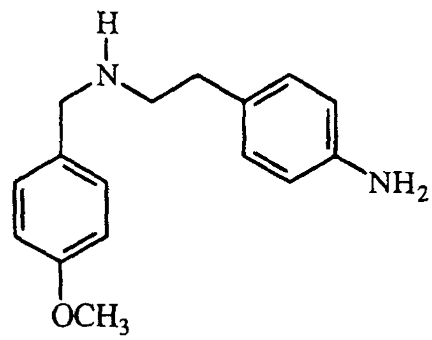

.

**11.** A titre de produit industriel nouveau selon la revendication 10, produit **caractérisé en ce qu'**il s'agit d'un des composés suivants :

- 1-{[3,4-dihydro-6-méthoxy-2,5,7,8-tétraméthyl-2H-1-benzopyran-2-yl]carbonyl}-4-(4-nitrophényl)pipérazine ;
- 1-{[3,4-dihydro-6-méthoxy-2,5,7,8-tétraméthyl-2H-1-benzopyran-2-yl]carbonyl}-4-(4-aminophényl) pipérazine ;
- hexahydro-4-(4-nitrophényl)-1H-1,4-diazépine ;
- 1-[(3,4-dihydro-6-hydroxy-2,5,7,8-tétraméthyl-2H-1-benzopyran-2-yl)carbonyl] hexahydro-4-(4-nitrophényl)-1H-1,4-diazépine ;
- 1-(4-aminophényl)-4-[(3,4-dihydro-6-hydroxy-2,5,7,8-tétraméthyl-2H-1-benzopyran-2-yl)carbonyl] hexahydro-1H-1,4-diazépine ;
- chlorhydrate du N-[4-{4-[(3,4-dihydro-6-hydroxy-2,5,7,8-tétraméthyl-2H-1-benzopyran-2-yl)carbonyl]-1H-1,4-diazépin-1-yl}phényl]-2-thiophènecarboximidamide ;
- (*R*)-3,4-dihydro-2,5,7,8-tétraméthyl-2- {4-[(4-nitrophényl)-1-pipérazinyl]-carbonyl}-2H-1-benzopyran-6-ol ;
- (*R*)-3,4-dihydro-2,5,7,8-tétraméthyl-2-{4-[(4-aminophényl)-1-pipérazinyl]-carbonyl}-2H-1-benzopyran-6-ol ;
- (*S*)-3,4-dihydro-2,5,7,8-tétraméthyl-2-{4-[(4-nitrophényl)-1-pipérazinyl]-carbonyl}-2H-1-benzopyran-6-ol ;
- (*S*)-3,4-dihydro-2,5,7,8-tétraméthyl-2-{4-[(4-aminophényl)-1-pipérazinyl]-carbonyl}-2H-1-benzopyran-6-ol ;
- 3,5-bis-(1,1-diméthyléthyl)-4-hydroxy-N-[2-(3-nitrophényl)éthyl]-benzamide ;
- 3,5-bis-(1,1-diméthyléthyl)-4-hydroxy-N-[2-(3-aminophényl)éthyl]-benzamide ;
- 3,5-bis-(1,1-diméthyléthyl)-4-hydroxybenzoate de 2-(4-nitrophényl)éthyle ;
- 3,5-bis-(1,1-diméthyléthyl)-4-hydroxy-benzoate de 2-(4-aminophényl)éthyle ;

ou d'un sel d'un de ces derniers.

**12.** Procédé de préparation d'un produit selon l'une quelconque des revendications 1 à 9, **caractérisé en ce que** l'on fait réagir, dans un alcool inférieur tel que méthanol, éthanol, alcool isopropylique ou t-butanol, de préférence dans l'alcool isopropylique, un composé de formule générale **(III)**

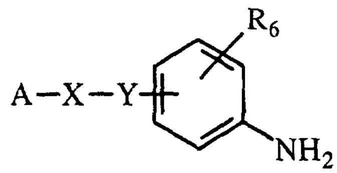

**(III)**

avec un composé de formule générale **(IV)**

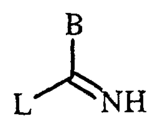

**(IV)**

ledit composé de formule générale **(IV)** pouvant éventuellement être salifié par un acide minéral G, de préférence HCl, HBr ou HI,
les composés de formules générales **(III)** et **(IV)** étant tels que :

A représente :

soit un radical

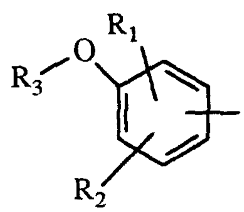

$R_1$ et $R_2$ représentant, indépendamment, un atome d'hydrogène, un halogène, le groupe OH, un radical alkyle ayant de 1 à 6 atomes de carbone linéaire ou ramifié, un radical alkoxy ayant de 1 à 6 atomes de carbone linéaire ou ramifié,
$R_3$ représentant un atome d'hydrogène, un radical alkyle linéaire ou ramifié ayant de 1 à 6 atomes de carbone ou un radical $-COR_4$,
et $R_4$ représentant un radical alkyle linéaire ou ramifié ayant de 1 à 6 atomes de carbone,

soit un radical

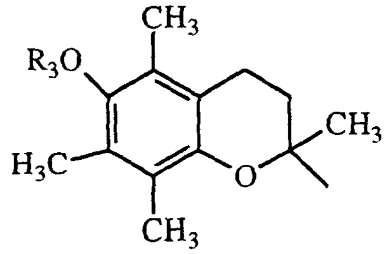

dans lequel $R_3$ a la signification indiquée ci-dessus

soit un radical

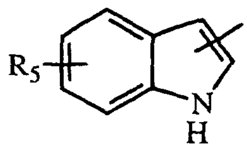

dans lequel $R_5$ représente un atome d'hydrogène, le groupe OH ou un radical alkyle ou alkoxy linéaire ou ramifié ayant de 1 à 6 atomes de carbone ;

B représente un radical alkyle ayant de 1 à 6 atomes de carbone linéaire ou ramifié, aryle carbocyclique ou hétérocyclique à 5 ou 6 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle, un alkényle ou un radical alkoxy ayant de 1 à 6 atomes de carbone et étant linéaire ou ramifié ;

L représente un groupe partant, et notamment un radical thioalkyle, acide sulfonique, acide trifluorométhanesulfonique, halogénure, alcool arylique ou tosyle ;

X représente $-Z_1-$, $-Z_1-CO-$, -CH=CH-CO-, $-Z_1-NR_3-CO-$, $-Z_1-NR_3-CS-$, $-Z_1-NR_3-SO_2-$ ou une liaison ;

Y représente $-Z_2-Q$, pipérazine, homopipérazine, 2-méthylpipérazine, 2,5-diméthyl-pipérazine, 4-aminopipéridine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ ou $-S-Z_2-Q-$,
avec Q = une liaison, $O-Z_3$ , $R_3-N-Z_3$ ou $S-Z_3$ ;

$Z_1$, $Z_2$ et $Z_3$ représentent indépendamment une liaison ou un radical alkylène linéaire ou ramifié ayant de 1 à 6 atomes de carbone;

$R_6$ représente un atome d'hydrogène ou un groupe OH.

**13.** A titre de médicament, un produit de formule générale **(I)** selon l'une quelconque des revendications 1 à 9, ou un sel pharmaceutiquement acceptable de ce produit.

**14.** Composition pharmaceutique contenant à titre de principe actif au moins un produit selon la revendication 13.

**15.** Composition pharmaceutique contenant à titre de principe actif du (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide ou un sel pharmaceutiquement acceptable de ce dernier.

**16.** Utilisation d'un produit de formule générale **(I)** selon l'une quelconque des revendications 1 à 9, ou d'un sel pharmaceutiquement acceptable de ce produit, pour fabriquer un médicament destiné à inhiber la NO synthase neuronale.

**17.** Utilisation d'un produit de formule générale **(I)** selon l'une quelconque des revendications 1 à 9, ou d'un sel pharmaceutiquement acceptable de ce produit, pour fabriquer un médicament destiné à inhiber la NO synthase inductible.

**18.** Utilisation d'un produit de formule générale **(I)** selon l'une quelconque des revendications 1 à 9, ou d'un sel pharmaceutiquement acceptable de ce produit, pour fabriquer un médicament destiné à inhiber la péroxidation lipidique.

**19.** Utilisation d'un produit de formule générale **(I)** selon l'une quelconque des revendications 1 à 9, ou d'un sel pharmaceutiquement acceptable de ce produit, pour fabriquer un médicament ayant à la fois une activité d'inhibition de la NO synthase et d'inhibition de la péroxidation lipidique.

**Patentansprüche**

**1.** Produkt, **dadurch gekennzeichnet, daß** es der allgemeinen Formel (I)

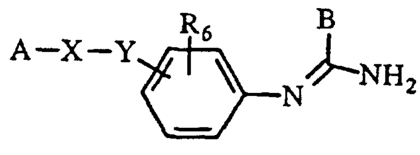

(I)

entspricht, in der:

A darstellt:

einen Rest

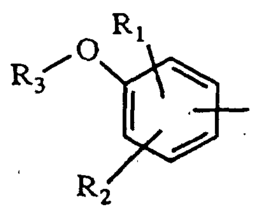

in dem $R_1$ und $R_2$ unabhängig voneinander ein Wasserstoffatom, ein Halogen, eine OH-Gruppe, einen verzweigten oder unverzweigten Alkyl- oder Alkoxy-Rest mit 1 bis 6 Kohlenstoffatomen darstellen,
$R_3$ ein Wasserstoffatom, einen verzweigten oder unverzweigten Alkyl-Rest mit 1 bis 6 Kohlenstoffatomen oder einen Rest -$COR_4$ darstellt,
$R_4$ einen verzweigten oder unverzweigten Alkyl-Rest mit 1 bis 6 Kohlenstoffatomen darstellt,

oder einen Rest

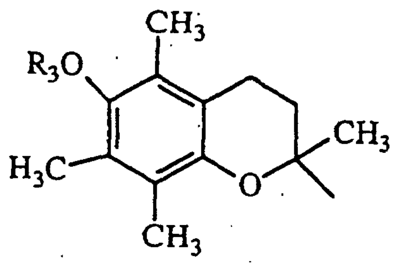

in dem $R_3$ die oben angegebene Bedeutung hat,

oder einen Rest

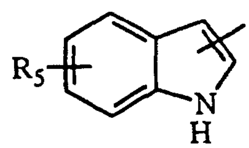

in dem $R_5$ ein Wasserstoffatom, eine OH-Gruppe oder einen verzweigten oder unverzweigten Alkyl- oder Alkoxy-Rest mit 1 bis 6 Kohlenstoffatomen darstellt;

B einen verzweigten oder unverzweigten Alkyl-Rest mit 1 bis 6 Kohlenstoffatomen, einen carbocyclischen oder heterocyclischen Aryl-Rest mit 5 oder 6 Gliedern darstellt, die 1 bis 4 Heteroatome enthalten, die aus O, S, N ausgewählt sind, und insbesondere die Reste Thiophen, Furan, Pyrrol oder Thiazol darstellt, wobei der Aryl-Rest gegebenenfalls durch eine oder mehrere Gruppen substituiert ist, die aus den verzweigten oder unverzweigten Resten Alkyl, Alkenyl oder Alkoxy mit 1 bis 6 Kohlenstoffatomen ausgewählt sind;

X $-Z_1-$, $-Z_1-CO-$, -CH=CH-CO-, $-Z_1-NR_3-CO-$, $-Z_1-NR_3-CS-$, $-Z_1-NR_3-SO_2-$ oder eine einfache Bindung darstellt;

Y einen Rest darstellt, der aus den Resten $-Z_2-Q$, Piperazin, Homopiperazin, 2-Methylpiperazin, 2,5-Dimethylpiperazin, 4-Aminopiperidin, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ oder $-S-Z_2-Q$ausgewählt ist, in denen Q eine einfache Bindung, $O-Z_3$, $R_3-N-Z_3$ oder $S-Z_3$ darstellt;

$Z_1$, $Z_2$ und $Z_3$ unabhängig voneinander eine einfache Bindung oder einen verzweigten oder unverzweigten Alkylen-Rest mit 1 bis 6 Kohlenstoffatomen darstellen, wobei $Z_1$, $Z_2$ und $Z_3$ vorzugsweise $-(CH_2)_m-$ darstellen, wobei m eine ganze Zahl zwischen 0 und 6 ist;

$R_6$ ein Wasserstoffatom oder eine OH-Gruppe darstellt;

oder ein Salz eines Produkts der allgemeinen Formel (I),
jedoch mit Ausnahme der Verbindung der folgenden Formel:

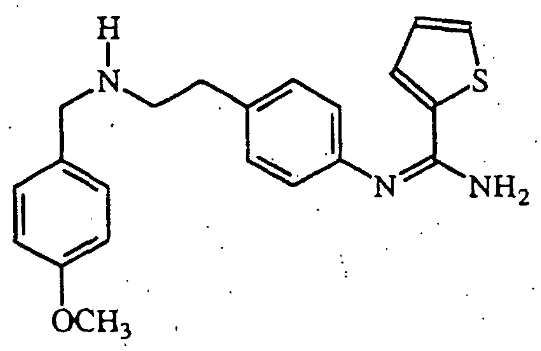

2. Produkt nach Anspruch 1, **dadurch gekennzeichnet, daß** es sich um eine der folgenden Verbindungen handelt:

- 3,5-Bis-(1,1-dimethylethyl)-4-hydroxy-N-{4-[(2-thienyl(imino)methyl)amino]phenyl}-benzamid;
- 3,5-Bis-(1,1-dimethylethyl)-4-hydroxy-N-{4-[[(2-thienyl(imino)methyl)amino]phenyl]methyl}-benzamid;
- 4-Acetoxy-3,5-dimethoxy-N-{4-[[(2-thienyl(imino)-methyl)amino]phenyl]methyl}-benzamid;
- 3,5-Dimethoxy-4-hydroxy-N-{4-[[(2-thienyl(imino)-methyl)amino]phenyl]methyl}-benzamid;
- 3,5-Bis-(1,1-dimethylethyl)-4-hydroxy-N-{4-[2-[(2-thienyl(imino)methyl)amino]phenyl]ethyl}-benzamid;
- 4-Acetoxy-3,5-dimethoxy-N-{4-[2-[(2-thienyl(imino)-methyl)amino]phenyl]ethyl}-benzamid;
- 3,5-Dimethoxy-4-hydroxy-N-{4-[2-[(2-thienyl(imino)methyl)amino]phenyl]ethyl}-benzamid;
- 3,4,5-Trihydroxy-N-{4-[2-[(2-thienyl(imino)-methyl)amino]phenyl)ethyl}-benzamid;
- N-{4-[4-[3,5-Bis-(1,1-dimethylethyl)-4-hydroxybenzoyl]-1-piperazinyl]phenyl}-2-thiophencarboximidamid;
- N-{4-[4-[3,5-Bis-(1,1-dimethylethyl)-4-hydroxybenzyl]-1-piperazinyl]phenyl}-2-thiophencarboximidamid;
- N-{4-[4-[3,5-Dimethoxy-4-hydroxybenzoyl]-1-piperazinyl]phenyl}-2-thiophencarboximidamid;
- 3,4-Dihydro-6-hydroxy-2,5,7,8-tetramethyl-N-{4-[(2-thienyl(imino)methyl)amino]phenyl}-2H-1-benzopyran-2-carboxamid;
- N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- N-{4-[4-[(5-Methoxy-1H-indol-3-yl)methylcarbonyl]-1-piperazinyl]phenyl}-2-thiophencarboximidamid;
- N-[4-[4-[{3-[3,5-Bis-(1,1-dimethylethyl)-4-hydroxyphenyl]-1-oxo-2-propenyl}-1-pierazinyl]phenyl]]-2-thiophencarboximidamid;
- 3,5-Bis-(1,1-dimethylethyl)-4-hydroxy-N-{3-[[(2-thienyl(imino)methyl)amino]phenyl]methyl}-benzamid;
- N-[3,5-Bis-(1,1-dimethylethyl)-4-hydroxyphenyl]-N'-{{4-[(2-thienyl(imino)methyl)amino]phenyl}methyl}harnstoff;
- N-[5-[{3-(3,5-Bis-(1,1-dimethylethyl)-4-hydroxyphenyl)-1-oxo-2-propenyl}amino]-2-hydroxyphenyl]-2-thiophencarboximidamid;
- N-[3-[{3-(3,5-Bis-(1,1-dimethylethyl)-4-hydroxyphenyl)-1-oxo-2-propenyl}amino]-4-hydroxyphenyl]-2-thiophencarboximidamid;
- N-{4-[4-[3,4,5-Trihydroxybenzoyl]-1-piperazinyl]phenyl}-2-thiphencarboximidamid;
- N-[3,5-Bis-(1,1-dimethylethyl)-4-hydroxyphenyl]-N'-{{4-[(2-thienyl(imino)methyl)amino]phenyl}carbonylamino}-harnstoff;
- N-[3,5-Bis-(1,1-dimethylethyl)-4-hydroxyphenyl]-N'-{{4-[(2-thienyl(imino)methyl)amino]phenyl}methyl]-thioharnstoff;
- N-[3,5-Bis-(1,1-dimethylethyl)-4-hydroxyphenyl]-N'-{2-{4-[(2-thienyl(imino)methyl)amino]phenyl}ethyl}harnstoff;
- N-(4-{4-[(3,4-Dihydro-6-methoxy-2,5,7,8-tetramethyl-2H-1-benzopyran-2-yl)carbonyl]-1-piperazinyl}phenyl)-2-thiophencarboximidamid;
- N-[4-{4-[(3,4-Dihydro-6-hydroxy-2,5,7,8-tetramethyl-2H-1-benzopyran-2-yl)carbonyl]-1H-1,4-diazepin-1-yl}phenyl]-2-thiophencarboximidamid;
- (R)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- (S)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- 3,5-Bis-(1,1-dimethylethyl)-4-hydroxy-N-{2-[3-[(2-thienyl(imino)methyl)amino]phenyl]ethyl}-benzamid;
- N-{4-(4-[2-(3,5-Bis-(1,1-dimethylethyl)-4-hydroxyphenyl)-1-oxo-ethyl]-1-piperazinyl)phenyl}-2-thiophencarboximidamid;

- 3,5-Bis-(1,1-dimethylethyl)-4-hydroxybenzoesäure-2-{4-[(2-thienyl(imino)methyl)amino]phenyl}ethylester;
- 3,5-Bis-(1,1-dimethylethyl)-4-hydroxybenzoesäure-2-{3-[(2-thienyl(imino)methyl)amino]phenyl}ethylester;
- 3,5-Bis-(1,1-dimethylethyl)-4-hydroxybenzoesäure-2-{2-[(2-thienyl(imino)methyl)amino]phenyl}ethylester;

oder eines der Salze einer dieser Verbindungen, insbesondere ein Chlorhydrat, Dichlorhydrat, Fumarat oder Hemifumarat einer dieser Verbindungen.

**3.** Produkt nach Anspruch 1, **dadurch gekennzeichnet, daß**:

X einen verzweigten oder unverzweigten Alkylen-Rest mit 1 bis 6 Kohlenstoffatomen darstellt und Y einen der Reste Piperazin, Homopiperazin, 2-Methylpiperazin, 2,5-Dimethylpiperazin, 4-Aminopiperidin, $-NR_3-Z_2-Q$, $-NR_3-NH-CO-Z_2-$, $-NH-NH-Z_2-$ oder $-NR_3-O-Z_2-$ darstellt;
oder

X $-Z_1-CO-$ oder -CH=CH-CO- darstellt und Y einen der Reste Piperazin, Homopiperazin, 2-Methylpiperazin, 2,5-Dimethylpiperazin, 4-Aminopiperidin, $-NR_3-Z_2-Q-$, $-NR_3-NH-CO-Z_2-$, $-NH-NH-Z_2-$, $-NR_3-O-Z_2-$, $-O-Z_2-Q-$ oder $-NR_3-CO-Q'-$ darstellt, wobei Q' = $R_3-N-Z_3$;
oder

X $-Z_1-NR_3-CO-$ darstellt und Y $-Z_2-Q-$, $-NH-Z_2-Q-$, $-NH-CO-Z_2-Q''-$ darstellt, wobei Q" = $O-Z_3-$, $R_3-N-Z_3-$ oder $S-Z_3-$ ist, oder Y $-NR_3-SO_2-NR_3-Z_2-$ oder $-O-Z_2-Q-$ darstellt;
oder

X $-Z_1-NH-CO-$ darstellt und Y einen der Reste Piperazin, Homopiperazin, 2-Methylpiperazin, 2,5-Dimethylpiperazin, 4-Aminopiperidin, $-NR_3-Z_2-Q-$, $-NR_3-NH-CO-Z_2-$, $-NH-NH-Z_2$oder $-NR_3-O-Z_2-$ darstellt;
oder

X $-Z_1-NR_3-SO_2-$ darstellt und Y $-Z_2-Q''-$ darstellt, wobei Q" = $O-Z_3-$, $R_3-N-Z_3-$ oder $S-Z_3-$ ist, oder Y $-NR_3-Z_2-$ Qdarstellt;
oder

X $-Z_1$ darstellt und Y $-O-CO-Z_2-Q-$ darstellt;
oder

X $-Z_1-NR_3-CS-$ darstellt und Y $-NH-Z_2-Q-$ oder einen der Reste Piperazin, Homopiperazin, 2-Methylpiperazin, 2,5-Dimethylpiperazin, 4-Aminopiperidin, $-NR_3-Z_2-Q-$, $-NH-NH-Z_2-$ oder $-NR_3-O-Z_2-$ darstellt;
oder

X eine Bindung darstellt und Y $-O-Z_2-NH-$; $-S-Z_2-NH-$ darstellt.

**4.** Produkt nach Anspruch 1, 2 oder 3, **dadurch gekennzeichnet, daß**:

A darstellt:

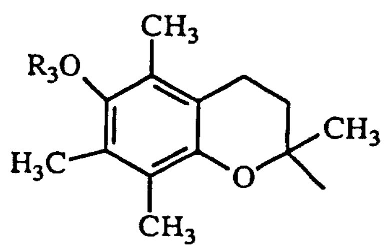

oder

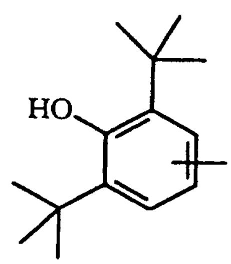

;

X -CO- oder -NH-CO- darstellt;

und Y einen Rest -NH-$Z_2$-Q- oder Piperazin darstellt, wobei Q eine Bindung, O-$Z_3$, $R_3$-N-$Z_3$ oder S-$Z_3$ darstellt und $Z_2$ und $Z_3$ unabhängig voneinander eine Bindung oder einen verzweigten oder unverzweigten Alkylen-Rest mit 1 bis 6 Kohlenstoffatomen darstellt und $R_3$ ein Wasserstoffatom oder einen verzweigten oder verzweigten Alkyl-Rest mit 1 bis 6 Kohlenstoffatomen darstellt.

**5.** Produkt nach Anspruch 1 oder 2, **dadurch gekennzeichnet, daß** es sich um eine der folgenden Verbindungen handelt:

- 3,5-Bis-(1,1-dimethylethyl)-4-hydroxy-N-{4-[2-[2-thienyl(iminomethyl)amino]phenyl]ethyl}-benzamid;
- 3,4,5-Trihydroxy-N-{4-[2-[2-thienyl (iminomethyl)-amino]phenyl]ethyl}-benzamid;
- N-{4-[4-[3,5-Bis-(1,1-dimethylethyl)-4-hydroxybenzoyl]-1-piperazinyl]phenyl}-2-thiophencarboximidamid;
- N-{4-[4-[3,5-Bis-(1,1-dimethylethyl)-4-hydroxybenzyl]-1-piperazinyl]phenyl}-2-thiophencarboximidamid;
- 3,4-Dihydro-6-hydroxy-2,5,7,8-tetramethyl-N-{4-[2-thienyl(iminomethyl)amino]phenyl}-2H-1-benzopyran-2-carboxamid;
- N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- N-{4-[4-[(5-Methoxy-1H-indol-3-yl)methylcarbonyl]-1-piperazinyl]phenyl}-2-thiophencarboximidamid;
- 3,5-Bis(1,1-dimethylethyl)-4-hydroxy-N-{3-[[2-thienyl(iminomethyl)amino]phenyl]methyl}benzamid;
- N-[3,5-Bis-(1,1-dimethylethyl)-4-hydroxyphenyl]-N'-{{4-[2-thienyl(iminomethyl)amino]phenyl}methyl}harnstoff;
- N- [5-[{3-(3,5-Bis-(1,1-dimethylethyl)-4-hydroxyphenyl)-1-oxo-2-propenyl}amino]-2-hydroxyphenyl]-2-thiophencarboximidamid;
- N-[3-[{3-(3,5-Bis-(1,1-dimethylethyl)-4-hydroxyphenyl)-1-oxo-2-propenyl}amino]-4-hydroxyphenyl]-2-thiophencarboximidamid;
- N-{4-[4-[3,4,5-Trihydrobenzoyl]-1-piperazinyl]phenyl}-2-thiophencarboximidamid;
- N-[3,5-Bis-(1,1-dimethylethyl)-4-hydroxyphenyl]-N'-{{4-[2-thienyl(iminomethyl)amino]phenyl}carbonylamino} harnstoff;
- (R)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- (S)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;

oder ein Salz einer dieser Verbindungen, insbesondere ein Chlorhydrat, Dichlorhydrat, Fumarat oder Hemifumarat einer dieser Verbindungen.

**6.** Produkt nach Anspruch 1 oder 2, **dadurch gekennzeichnet, daß** es sich um eine der folgenden Verbindungen handelt:

- 4-Acetoxy-3,5-dimethoxy-N-{4-[2-[2-thienyl-(iminomethyl)amino]phenyl]ethyl}-benzamid;
- 3,5-Dimethoxy-4-hydroxy-N-{4-[2-[2-thienyl-(iminomethyl)amino]phenyl]ethyl}-benzamid;

oder ein Salz dieser Verbindungen, insbesondere ein Chlorhydrat, Dichlorhydrat, Fumarat oder Hemifumarat einer dieser Verbindungen.

**7.** Produkt nach Anspruch 1 oder 2, **dadurch gekennzeichnet, daß** es sich um N-{4-[4-[3,5-Bis(1,1-dimethylethyl)-4-hydroxybenzyl]-1-piperazinyl]phenyl}-2-thiophencarboximidamid
oder um ein Salz dieser Verbindung, insbesondere um das Chlorhydrat, Dichlorhydrat, Fumarat oder Hemifumarat dieser Verbindung, handelt.

**8.** Produkt nach Anspruch 1 oder 2, **dadurch gekennzeichnet, daß** es sich um eine der folgenden Verbindungen handelt:

- N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- (R)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;
- (S)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid;

oder ein Salz einer dieser Verbindungen, insbesondere ein Chlorhydrat, Dichlorhydrat, Fumarat oder Hemifumarat einer dieser Verbindungen.

**9.** Produkt nach Anspruch 8, **dadurch gekennzeichnet, daß** es sich um (S)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid oder um ein Salz dieser Verbindung, insbesondere um das Chlorhydrat, Dichlorhydrat, Fumarat oder Hemifumarat dieser Verbindung, handelt.

**10.** Produkt in Form eines neuen Industrieprodukts, **dadurch gekennzeichnet, daß** es der allgemeinen Formel (II)A entspricht:

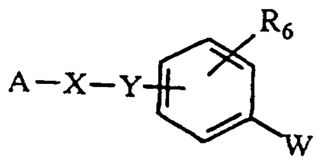

(II)A

in der:

W einen Amino- oder Nitro-Rest darstellt,

A darstellt:

entweder einen Rest

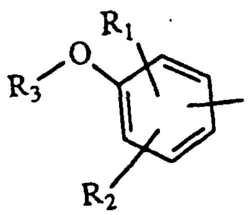

in dem $R_1$ und $R_2$ unabhängig voneinander ein Wasserstoffatom, ein Halogen, eine OH-Gruppe oder einen verzweigten oder unverzweigten Alkyl- oder Alkoxy-Rest mit 1 bis 6 Kohlenstoffatomen darstellt,

$R_3$ ein Wasserstoffatom, einen verzweigten oder unverzweigten Alkyl-Rest mit 1 bis 6 Kohlenstoffatomen oder einen Rest -$COR_4$ darstellt,

wobei $R_4$ einen verzweigten oder unverzweigten Alkyl-Rest mit 1 bis 6 Kohlenstoffatomen darstellt,

oder einen Rest

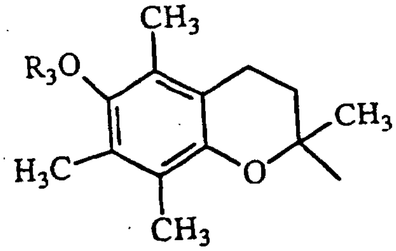

in dem $R_3$ die oben angegebene Bedeutung hat

oder einen Rest

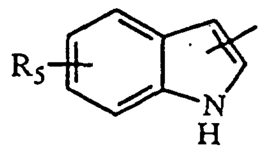

in dem $R_5$ ein Wasserstoffatom, eine OH-Gruppe oder einen verzweigten oder unverzweigten Alkyl- oder Alkoxy-Rest mit 1 bis 6 Kohlenstoffatomen darstellt;

X $-Z_1-$, $-Z_1$-CO-, -CH=CH-CO-, $-Z_1-NR_3$-CO-, $-Z_1-NR_3$-CS-, $-Z_1-NR_3-SO_2-$ oder eine einfache Bindung darstellt;

Y einen Rest darstellt, der aus den Resten $-Z_2$-Q, Piperazin, Homopiperazin, 2-Methylpiperazin, 2,5-Dimethylpiperazin, 4-Aminopiperidin, $-NR_3-Z_2$-Q-, $-NR_3$-CO-$Z_2$-Q-, $-NR_3$-NH-CO-$Z_2$-, -NH-NH-$Z_2$-, $-NR_3$-O-$Z_2$-, $-NR_3-SO_2-NR_3-Z_2-$, -O-$Z_2$-Q-, -O-CO-$Z_2$-Q- oder -S-$Z_2$-Qausgewählt ist, in denen Q eine einfache Bindung, O-$Z_3$, $R_3$-N-$Z_3$ oder S-$Z_3$ darstellt;

$Z_1$, $Z_2$ und $Z_3$ unabhängig voneinander eine einfache Bindung oder einen verzweigten oder unverzweigten Alkylen-Rest mit 1 bis 6 Kohlenstoffatomen darstellen, wobei $Z_1$, $Z_2$ und $Z_3$ vorzugsweise $-(CH_2)_m-$ darstellen, wobei m eine ganze Zahl zwischen 0 und 6 ist;

$R_6$ ein Wasserstoffatom oder eine OH-Gruppe darstellt;

wobei jedoch 3,5-Bis-(1,1-dimethylethyl)-4-hydroxy-N-(4-nitrophenyl)-benzamid ausgenommen ist;
oder ein Salz eines Produkts der allgemeinen Formel (II)A,
wobei jedoch die Produkte ausgenommen sind, die den folgenden Formeln entsprechen:

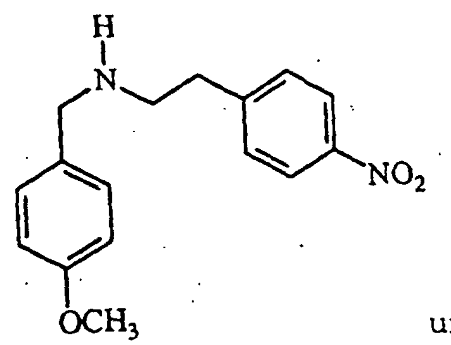

und

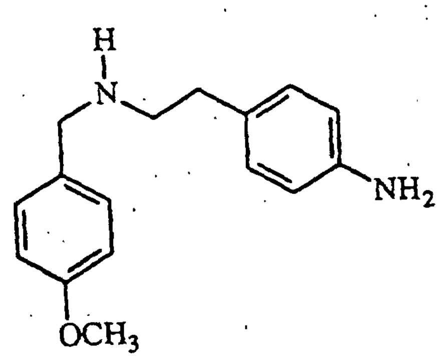

**11.** Produkt in Form eines neuen Industrieprodukts nach Anspruch 10, **dadurch gekennzeichnet, daß** es sich um eine der folgenden Verbindungen handelt:

- 1-{[3,4-Dihydro-6-methoxy-2,5,7,8-tetramethyl-2H-1-benzopyran-2-yl]carbonyl}-4-(4-nitrophenyl)piperazin;
- 1-{[3,4-Dihydro-6-methoxy-2,5,7,8-tetramethyl-2H-1-benzopyran-2-yl]carbonyl}-4-(4-aminophenyl)piperazin;
- Hexahydro-4-(4-nitrophenyl)-1H-1,4-diazepin;
- 1-[(3,4-Dihydro-6-hydroxy-2,5,7,8-tetramethyl-2H-1benzopyran-2-yl)carbonyl]hexahydro-4-(4-nitrophenyl)-1H-1,4-diazepin;
- 1-(4-Aminophenyl)-4-[(3,4-dihydro-6-hydroxy-2,5,7,8-tetramethyl-2H-1-benzopyran-2-yl)carbonyl]-hexahydro-1H-1,4-diazepin;
- N-[4-{4-[(3,4-Dihydro-6-hydroxy-2,5,7,8-tetramethyl-2H-1-benzopyran-2-yl)carbonyl]-1H-1,4-diazepin-1-yl}phenyl]-2-thiophencarboximidamid-chlorhydrat;

- (R)-3,4-Dihydro-2,5,7,8-tetramethyl-2-{4-[(4-nitrophenyl)-1-piperazinyl]carbonyl}-2H-1-benzopyran-6-ol;
- (R)-3,4-Dihydro-2,5,7,8-tetramethyl-2-(4-[(4-aminophenyl)-1-piperazinyl]carbonyl}-2H-1-benzopyran-6-ol;
- (S)-3,4-Dihydro-2,5,7,8-tetramethyl-2-{4-[(4-nitrophenyl)-1-piperazinyl]carbonyl}-2H-1-benzopyran-6-ol;
- (S)-3,4-Dihydro-2,5,7,8-tetramethyl-2-{4-[(4-aminophenyl)-1-piperazinyl]carbonyl}-2H-1-benzopyran-6-ol;
- 3,5-Bis-(1,1-dimethylethyl)-4-hydroxy-N-[2-(3-nitrophenyl)ethyl]-benzamid;
- 3,5-Bis-(1,1-dimethylethyl)-4-hydroxy-N-[2-(3-aminophenyl)ethyl]-benzamid;
- 3,5-Bis-(1,1-dimethylethyl)-4-hydroxybenzoesäure-2-(4-nitrophenyl)ethylester;
- 3,5-Bis(1,1-dimethylethyl)-4-hydroxybenzoesäure-2-(4-aminophenyl)ethylester;

oder ein Salz einer dieser Verbindungen.

**12.** Verfahren zur Herstellung eine Produkts nach einem der Ansprüche 1 bis 9, **dadurch gekennzeichnet, daß** man in einem niederen Alkohol, wie z.B. Methanol, Ethanol, Isopropylalkohol oder t-Butanol, vorzugsweise in Isopropylalkohol, eine Verbindung der allgemeinen Formel (III)

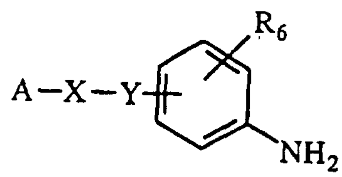

**(III)**

mit einer Verbindung der allgemeinen Formel (IV)

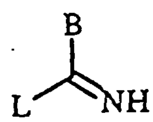

**(IV)**

reagieren läßt, wobei die Verbindung der allgemeinen Formel (IV) gegebenenfalls mit Hilfe einer Mineralsäure G, vorzugsweise HCl, BRr oder HI, in einer Salzform vorliegen kann,

wobei die Verbindungen der allgemeinen Formeln (III) und (IV) so beschaffen sind, daß:

A darstellt:

entweder einen Rest

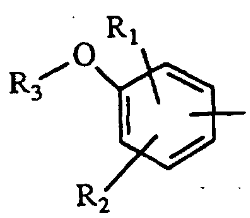

wobei $R_1$ und $R_2$ unabhängig voneinander ein Wasserstoffatom, ein Halogen, eine OH-Gruppe, einen verzweigten oder unverzweigten Alkyl-Rest mit 1 bis 6 Kohlenstoffatomen, einen verzweigten oder

unverzweigten Alkoxy-Rest mit 1 bis 6 Kohlenstoffatomen darstellt,

$R_3$ ein Wasserstoffatom, einen verzweigten oder unverzweigten Alkyl-Rest mit 1 bis 6 Kohlenstoffatomen oder einen Rest $-COR_4$ darstellt,

und $R_4$ einen verzweigten oder unverzweigten Alkyl-Rest mit 1 bis 6 Kohlenstoffatomen darstellt,

oder einen Rest

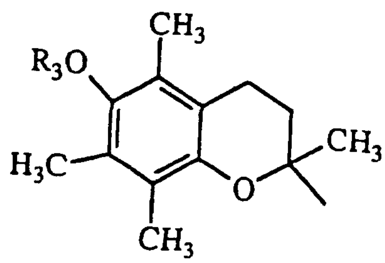

in dem $R_3$ die oben angegebene Bedeutung hat

oder einen Rest

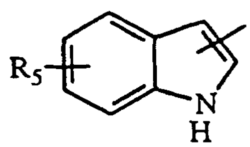

in dem $R_5$ ein Wasserstoffatom, eine OH-Gruppe oder einen verzweigten oder unverzweigten Alkyl- oder Alkoxy-Rest mit 1 bis 6 Kohlenstoffatomen darstellt;

B einen verzweigten oder unverzweigten Alkyl-Rest mit 1 bis 6 Kohlenstoffatomen, einen carbocyclischen oder heterocyclischen Aryl-Rest mit 5 oder 6 Gliedern darstellt, die 1 bis 4 Heteroatome enthalten, die aus O, S, N ausgewählt sind, und insbesondere: Thiophen, Furan, Pyrrol oder Thiazol darstellt, wobei deren Kohlenstoffatome gegebenenfalls durch eine oder mehrere Gruppen substituiert sind, die aus einem der Reste verzweigtes oder unverzweigtes Alkyl, Alkenyl oder Alkoxy mit 1 bis 6 Kohlenstoffatomen ausgewählt sind;

L eine Abgangsgruppe, insbesondere einen Thioalkyl-Rest, Sulfonsäure, Trifluormethansulfonsäure, ein Halogenid, Arylalkohol oder Tosyl darstellt;

X $-Z_1-$, $-Z_1-CO-$, $-CH=CH-CO-$, $-Z_1-NR_3-CO-$, $-Z_1-NR_3-CS-$, $-Z_1-NR_3-SO_2-$ oder eine Bindung darstellt;

Y $-Z_2-Q$, Piperazin, Homopiperazin, 2-Methylpiperazin, 2,5-Dimethylpiperazin, 4-Aminopiperidin, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ oder $-S-Z_2-Q$darstellt, wobei Q = eine Bindung, $O-Z_3$, $R_3-N-Z_3$ oder $S-Z_3$ bedeutet;

$Z_1$, $Z_2$ und $Z_3$ unabhängig voneinander eine Bindung oder einen verzweigten oder unverzweigten Alkylen-Rest mit 1 bis 6 Kohlenstoffatomen darstellen;

$R_6$ ein Wasserstoffatom oder ein OH-Gruppe darstellt.

**13.** Ein Produkt der allgemeinen Formel (I) nach einem der Ansprüche 1 bis 9 oder ein pharmazeutisch verträgliches Salz dieses Produkts in Form eines Arzneimittels.

**14.** Pharmazeutische Zusammensetzung, die als Wirkstoff mindestens ein Produkt nach Anspruch 13 enthält.

**15.** Pharmazeutische Zusammensetzung, die als Wirkstoff (S)-N-{4-[4-[(3,4-Dihydro-6-hydroxy-2,5,7,8-tetramethyl-2H-1-benzopyran-2-yl)carbonyl]-1-piperazinyl]phenyl}-2-thiophencarboximidamid oder ein pharmazeutisch verträgliches Salz dieser Verbindung enthält.

**16.** Verwendung eines Produkts der allgemeinen Formel (I) nach einem der Ansprüche 1 bis 9 oder eines pharmazeutisch verträglichen Salzes dieses Produkts zur Herstellung eines Arzneimittels zur Hemmung der neuronalen NO-Synthase.

**17.** Verwendung eines Produkts der allgemeinen Formel (I) nach einem der Ansprüche 1 bis 9 oder eines pharmazeutisch verträglichen Salzes dieses Produkts zur Herstellung eines Arzneimittels zur Hemmung der induktiblen NO-Synthase.

**18.** Verwendung eines Produkts der allgemeinen Formel (I) nach einem der Ansprüche 1 bis 9 oder eines pharmazeutisch verträglichen Salzes dieses Produkts zur Herstellung eines Arzneimittels zur Hemmung der Lipidperoxidation.

**19.** Verwendung eines Produkts der allgemeinen Formel (I) nach einem der Ansprüche 1 bis 9 oder eines pharmazeutisch verträglichen Salzes dieses Produkts zur Herstellung eines Arzneimittels, das gleichzeitig eine die NO-Synthase hemmende und eine die Lipidperoxidation hemmende Aktivität besitzt.

**Claims**

**1.** Product **characterized in that** it corresponds to the general formula **(I)**

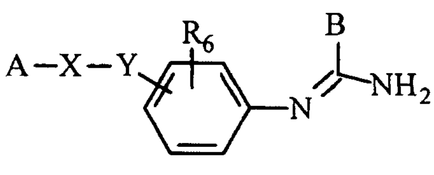

**(I)**

in which:

A represents:

either a

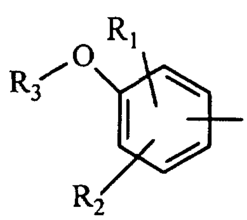

radical in which $R_1$ and $R_2$ represent, independently, a hydrogen atom, a halogen, the OH group, a linear or branched alkyl or alkoxy radical with 1 to 6 carbon atoms,
$R_3$ represents a hydrogen atom, a linear or branched alkyl radical with 1 to 6 carbon atoms or a -$COR_4$ radical,
$R_4$ representing a linear or branched alkyl radical with 1 to 6 carbon atoms,

or a

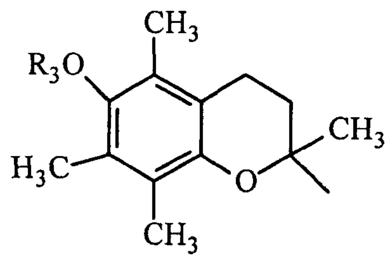

radical in which $R_3$ has the meaning indicated above

or a

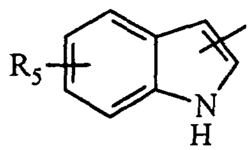

radical in which $R_5$ represents a hydrogen atom, the OH group or a linear or branched alkyl or alkoxy radical with 1 to 6 carbon atoms;

B represents a linear or branched alkyl radical with 1 to 6 carbon atoms, carbocyclic or heterocyclic aryl radical with 5 or 6 members containing 1 to 4 heteroatoms chosen from O, S, N and in particular the thiophene, furane, pyrrole or thiazole radicals, the aryl radical being optionally substituted by one or more groups chosen from the linear or branched alkyl, alkenyl or alkoxy radicals with 1 to 6 carbon atoms;

X represents $-Z_1-$, $-Z_1-CO-$, -CH=CH-CO-, $-Z_1-NR_3-CO-$, $-Z_1-NR_3-CS-$, $-Z_1-NR_3-SO_2-$ or a single bond;

Y represents a radical chosen from the $-Z_2-Q$, piperazine, homopiperazine, 2-methylpiperazine, 2,5-dimethylpiperazine, 4-aminopiperidine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ or $-S-Z_2-Q-$ radicals,
in which Q represents a single bond, $O-Z_3$, $R_3-N-Z_3$ or $S-Z_3$;

$Z_1$, $Z_2$ and $Z_3$ represent independently a single bond or a linear or branched alkylene radical with 1 to 6 carbon atoms, $Z_1$, $Z_2$ and $Z_3$ preferably representing $-(CH_2)_m-$, m being an integer from 0 and 6;

$R_6$ represents a hydrogen atom or an OH group;

or a salt of a product of general formula **(I),**
with the exception however of the compound of the following formula:

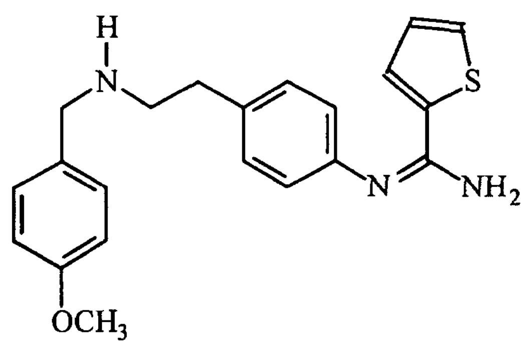

**2.** Product according to claim 1, **characterized in that** it is one of the following compounds:

- 3,5-bis-(1,1-dimethylethyl)-4-hydroxy-N- {4-[(2-thienyl (imino)methyl)amino]phenyl}-benzamide;
- 3,5-bis-(1,1-dimethylethyl)-4-hydroxy-N- {4-[[(2-thienyl (imino)methyl)amino]phenyl]methyl}-benzamide;
- 4-acetoxy-3,5-dimethoxy-N-{4-[[(2-thienyl(imino)methyl)amino]phenyl]methyl}-benzamide;
- 3,5-dimethoxy-4-hydroxy-N-{4-[[(2-thienyl(imino)methyl)amino]phenyl]methyl}-benzamide;
- 3,5-bis-(1,1-dimethylethyl)-4-hydroxy-N- {4-[2-[(2-thienyl-(imino)methyl)amino]phenyl]ethyl}-benzamide;
- 4-acetoxy-3,5-dimethoxy-N- {4-[2-[(2-thienyl-(imino)methyl)-amino]phenyl]ethyl}-benzamide;
- 3,5-dimethoxy-4-hydroxy-N-{4-[2-[(2-thienyl-(imino)methyl)-amino]phenyl]ethyl}-benzamide;
- 3,4,5-trihydroxy-N-{4-[2-[(2-thienyl(imino)methy])-amino]phenyl]ethyl}-benzamide;
- N- {4-[4-[3,5-bis-(1,1-dimethylethyl)-4-hydroxybenzoyl]-1-piperazinyl]-phenyl}-2-thiophenecarboximidamide;
- N-{4-[4-[3,5-bis-(1,1-dimethylethyl)-4-hydroxybenzyl]-1-piperazinyl]phenyl}-2-thiophenecarboximidamide;
- N-{4-[4-[3,5-dimethoxy-4-hydroxybenzoyl]-1-piperazinyl]-phenyl}-2-thiophenecarboximidamide;
- 3,4-dihydro-6-hydroxy-2,5,7,8-tetramethyl-N-{4-[(2-thienyl (imino)methyl)amino]phenyl}-2H-1-benzopyran-2-carboxamide;
- N-{4-[4-[(3,4-dihydro-6-hydroxy-2,5,7,8-tetramethyl-2H-1-benzopyran-2-yl)-carbonyl]- 1-piperazinyl]phenyl}-2-thiophenecarboximidamide;
- N-{4-[4-[(5-methoxy-1H-indol-3-yl)methylcarbonyl]-1-piperazinyl]phenyl}-2-thiophenecarboximidamide;
- N-[4-[4-[ {3-[3,5-bis-(1,1-dimethylethyl)-4-hydroxyphenyl]-1-oxo-2-propenyl}-1 -piperazinyl]-phenyl]]-2-thiophenecarboximidamide;
- 3,5-bis-(1,1-dimethylethyl)-4-hydroxy-N- {3-[[(2-thienyl-(imino)methyl)amino]phenyl]methyl}-benzamide;
- N-[3,5-bis-(1,1-dimethylethyl)-4-hydroxyphenyl]-N'- {{4-[(2-thienyl(imino)methyl)amino]phenyl} methyl} -urea;
- N-[5-[{3-(3,5-bis-(1,1-dimethylethyl)-4-hydroxyphenyl)- 1-oxo-2-propenyl} amino]-2-hydroxyphenyl]-2-thiophenecarboximidamide;
- N-[3-[{3-(3,5-bis-(1,1-dimethylethyl)-4-hydroxyphenyl)-1-oxo-2-propenyl}-amino]-4-hydroxyphenyl]-2-thiophenecarboximidamide;
- N-{4-[4-[3,4,5-trihydroxybenzoyl]-1-piperazinyl]phenyl}-2-thiophenecarboximidamide;
- N-[3,5-bis-(1,1-dimethylethyl)-4-hydroxyphenyl]-N'- {{4-[(2-thienyl(imino)methyl)amino]phenyl}carbonylamino}-urea;
- N-[3,5-bis-(1,1-dimethylethyl)-4-hydroxyphenyl]-N'-{{4-[(2-thienyl(imino)methyl)amino)phenyl}methyl}-thiourea;
- N-[3,5-bis-(1,1-dimethylethyl)-4-hydroxyphenyl]-N'- {2-{4-[(2-thienyl(imino)methyl)amino]phenyl} ethyl} -urea;
- N-(4-{4-[(3,4-dihydro-6-methoxy-2,5,7,8-tetramethyl-2H-1-benzopyran-2-yl) carbonyl]-1-piperazinyl}phenyl)-2-thiophenecarboximidamide;
- N-[4-{4-[(3,4-dihydro-6-hydroxy-2,5,7,8-tetramethyl-2H-1-benzopyran-2-yl) carbonyl]-1H-1,4-diazepin-1-yl}phenyl]-2-thiophenecarboximidamide;
- (R)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- (S)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- 3,5-bis-(1,1-dimethylethyl)-4-hydroxy-N-{2-[3-[(2-thienyl(imino)methyl)amino] phenyl]ethyl}-benzamide;
- N- {4-(4-[2-(3,5-bis-(1,1-dimethylethyl)-4-hydroxyphenyl)-1-oxo-ethyl]-1-piperazinyl)phenyl} -2-thiophenecarboximidamide;
- 2- {4-[(2-thienyl(imino)methyl)amino]phenyl} ethyl 3,5-bis-(1,1-dimethylethyl)-4-hydroxy-benzoate;
- 2- {3-[(2-thienyl(imino)methyl)amino]phenyl} ethyl 3,5-bis-(1,1-dimethylethyl)-4-hydroxy-benzoate;
- 2-{2-[(2-thienyl(imino)methyl)amino]phenyl}ethyl 3,5-bis-(1,1-dimethylethyl)-4-hydroxy-benzoate;

or one of the salts of one of the latter, in particular a hydrochloride, dihydrochloride, fumarate or hemi-fumarate of one of the latter.

**3.** Product according to claim 1, **characterized in that**:

- X represents a linear or branched alkylene radical with 1 to 6 carbon atoms and Y represents a piperazine, homopiperazine, 2-methylpiperazine, 2,5-dimethylpiperazine, 4-aminopiperidine, $-NR_3-Z_2-Q-$, $-NR_3-NH-CO-Z_2-$, $-NH-NH-Z_2-$ or $-NR_3-O-Z_2-$ radical;
  or
- X represents $-Z_1-CO-$ or -CH=CH-CO- and Y represents a piperazine, homopiperazine, 2-methylpiperazine, 2,5-dimethylpiperazine, 4-aminopiperidine, $-NR_3-Z_2-Q-$, $-NR_3-NH-CO-Z_2-$, $-NH-NH-Z_2-$, $-NR_3-O-Z_2-$, $-O-Z_2-Q-$

or $-NR_3-CO-Q'-$ radical with $Q' = R_3-N-Z_3$;
or

- X represents $-Z_1-NR_3-CO-$ and Y represents $-Z_2-Q-$, $-NH-Z_2-Q-$, $-NH-CO-Z_2-Q''-$ with $Q'' = O-Z_3-$, $R_3-N-Z_3-$ or $S-Z_3-$, or Y represents $-NR_3-SO_2-NR_3-Z_2-$ or $-O-Z_2-Q-$;
or
- X represents $-Z_1-NH-CO-$ and Y represents a piperazine, homopiperazine, 2-methylpiperazine, 2,5-dimethyl-piperazine, 4-aminopiperidine, $-NR_3-Z_2-Q-$, $-NR_3-NH-CO-Z_2-$, $-NH-NH-Z_2-$ or $-NR_3-O-Z_2-$ radical;
or
- X represents $-Z_1-NR_3-SO_2-$ and Y represents $-Z_2-Q''-$ with $Q'' = O-Z_3-$, $R_3-N-Z_3-$ or $S-Z_3-$, or Y represents $-NR_3-Z_2-Q-$;
or
- X represents $-Z_1-$ and Y represents $-O-CO-Z_2-Q-$;
or
- X represents $-Z_1-NR_3-CS-$ and Y represents $-NH-Z_2-Q-$, or a piperazine, homopiperazine, 2-methylpiperazine, 2,5-dimethyl-piperazine, 4-aminopiperidine, $-NR_3-Z_2-Q-$, $-NH-NH-Z_2-$ or $-NR_3-O-Z_2-$ radical;
or
- X represents a bond and Y represents $-O-Z_2-NH-$, $-S-Z_2-NH-$.

**4.** Product according to claim 1, 2 or 3, **characterized in that**:

- A represents:

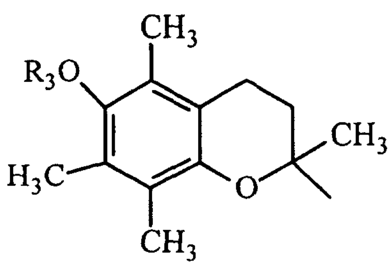

or

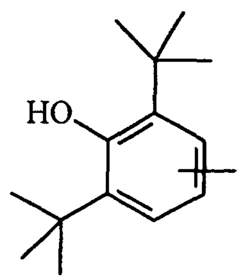

;

- X represents -CO- or -NH-CO-;

- and Y represents an $-NH-Z_2-Q-$ or piperazine radical, Q representing a bond, $O-Z_3$, $R_3-N-Z_3$ or $S-Z_3$, and $Z_2$ and $Z_3$ representing independently a bond or a linear or branched alkylene radical with 1 to 6 carbon atoms and $R_3$ represents a hydrogen atom or a linear or branched alkyl radical with 1 to 6 carbon atoms.

**5.** Product according to claim 1 or 2, **characterized in that** it is one of the following compounds:

- 3,5-bis-(1,1-dimethylethyl)-4-hydroxy-N-{4-[2-[2-thienyl-(iminomethyl)amino]phenyl]ethyl}-benzamide;
- 3,4,5-trihydroxy-N-{4-[2-[2-thienyl(iminomethyl)-amino]phenyl]ethyl}-benzamide;
- N-{4-[4-[3,5-bis-(1,1-dimethylethyl)-4-hydroxybenzoyl]-1-piperazinyl]phenyl}-2-thiophenecarboximidamide;
- N-{4-[4-[3,5-bis-(1,1-dimethylethyl)-4-hydroxybenzyl]-1-piperazinyl]phenyl}-2-thiophenecarboximidamide;
- 3,4-dihydro-6-hydroxy-2,5,7,8-tetramethyl-N-{4-[2- thienyl (iminomethyl)amino]phenyl}-2H-1-benzopyran-2-carboxamide;
- N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- N-{4-[4-[(5 methoxy-1H-indol-3-yl)methylcarbonyl]-1-piperazinyl]phenyl}-2-thiophenecarboximidamide;
- 3,5-bis-(1,1-dimethylethyl)-4-hydroxy-N-{3-[[2-thienyl-(iminomethyl)amino]phenyl]methyl}-benzamide;
- N-[3,5-bis-(1,1-dimethylethyl)-4-hydroxyphenyl]-N'-{{4-[2-thienyl(iminomethyl)amino]phenyl}methyl}-urea;
- N-[5-[{3-(3,5-bis-(1,1-dimethylethyl)-4-hydroxyphenyl)-1-oxo-2-propenyl}-amino]-2-hydroxyphenyl]-2-thiophenecarboximidamide;
- N-[3-[{3-(3,5-bis-(1,1-dimethylethyl)-4-hydroxyphenyl)-1-oxo-2-propenyl}-amino]-4-hydroxyphenyl]-2-thiophenecarboximidamide;
- N- {4-[4-[3,4,5-trihydroxybenzoyl]- 1-piperazinyl]phenyl}-2-thiophenecarboximidamide;
- N-[3,5-bis-(1,1-dimethylethyl)-4-hydroxyphenyl]-N'-{ {4-[2-thienyl(iminomethyl)amino]phenyl}carbonylamino}-urea;

- (R)-N- {4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- (S)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;

or a salt of one of the latter, in particular a hydrochloride, dihydrochloride, fumarate or hemi-fumarate of one of the latter.

**6.** Product according to claim 1 or 2, **characterized in that** it is one of the following compounds:

- 4-acetoxy-3,5-dimethoxy-N- {4-[2-[2-thienyl-(iminomethyl)-amino]phenyl]ethyl}-benzamide;
- 3,5-dimethoxy-4-hydroxy-N-{4-[2-[2-thienyl-(iminomethyl)-amino]phenyl]ethyl}-benzamide;

or a salt of the latter, in particular a hydrochloride, dihydrochloride, fumarate or hemi-fumarate of one of the latter.

**7.** Product according to claim 1 or 2, **characterized in that** it is the N-{4-[4-[3,5-bis-(1,1-dimethylethyl)-4-hydroxybenzyl]-1-piperazinyl]phenyl}-2-thiophenecarboximidamide;
or a salt of the latter, in particular the hydrochloride, dihydrochloride, fumarate or hemi-fumarate of the latter.

**8.** Product according to claim 1 or 2, **characterized in that** it is one of the following compounds:

- N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- (R)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;
- (S)-N-{4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide;

or a salt of one of the latter, in particular a hydrochloride, dihydrochloride, fumarate or hemi-fumarate of one of the latter.

**9.** Product according to claim 8, **characterized in that** it is (S)-N- {4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide, or a salt of the latter, in particular a hydrochloride, dihydrochloride, fumarate or hemi-fumarate of the latter.

**10.** As a new industrial product, product **characterized in that** it corresponds to the general formula **(II)A:**

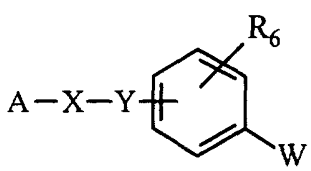

**(II)A**

in which:

W represents an amino or nitro radical,

A represents:

either a

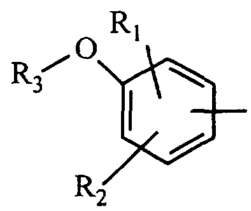

radical in which $R_1$ and $R_2$ represent, independently, a hydrogen atom, a halogen, the OH group, a linear or branched alkyl or alkoxy radical with 1 to 6 carbon atoms,

$R_3$ represents a hydrogen atom, a linear or branched alkyl radical with 1 to 6 carbon atoms or a $-COR_4$ radical,

$R_4$ representing a linear or branched alkyl radical with 1 to 6 carbon atoms,

or a

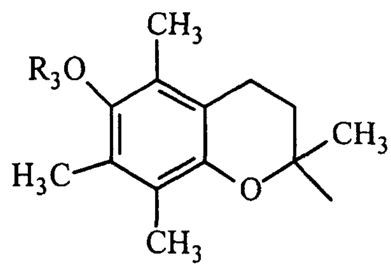

radical in which $R_3$ has the meaning indicated above

or a

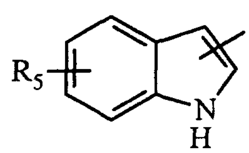

radical in which $R_5$ represents a hydrogen atom, the OH group or a linear or branched alkyl or alkoxy radical with 1 to 6 carbon atoms;

X represents $-Z_1-$, $-Z_1-CO-$, -CH=CH-CO-, $-Z_1-NR_3-CO-$, $-Z_1-NR_3-CS-$, $-Z_1-NR_3-SO_2-$ or a single bond;

Y represents a radical chosen from the $-Z_2-Q$, piperazine, homopiperazine, 2-methylpiperazine, 2,5-dimethylpiperazine, 4-aminopiperidine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ or $-S-Z_2-Q-$ radicals, in which Q represents a single bond, $O-Z_3$, $R_3-N-Z_3$ or $S-Z_3$;

$Z_1$, $Z_2$ and $Z_3$ represent independently a single bond or a linear or branched alkylene radical with 1 to 6 carbon atoms, $Z_1$, $Z_2$ and $Z_3$ preferably representing $-(CH_2)_m-$, m being an integer from 0 and 6;

$R_6$ represents a hydrogen atom or an OH group;

with the exception however of 3,5-bis-(1,1-dimethylethyl)-4-hydroxy-N-(4-nitrophenyl)-benzamide;
or a salt of a product of general formula **(II)A,**
with the exception however of the compounds corresponding to the following formulae:

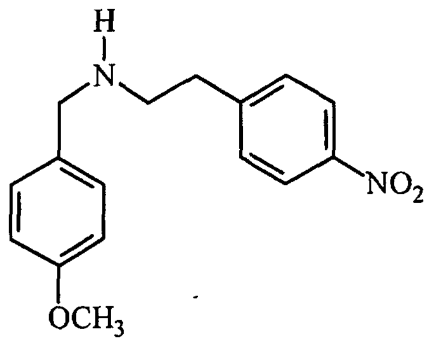

and

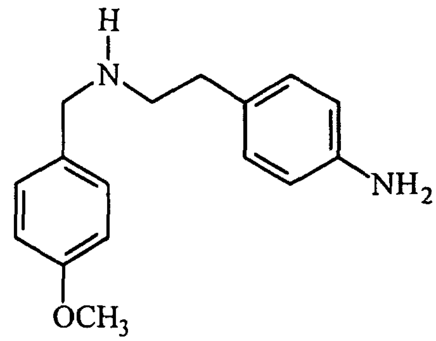

**11.** As a new industrial product according to claim 10, product **characterized in that** it is one of the following compounds:

- 1-{[3,4-dihydro-6-methoxy-2,5,7,8-tetramethyl-2H-1-benzopyran-2-yl]carbonyl}-4-(4-nitrophenyl)piperazine;
- 1-{[3,4-dihydro-6-methoxy-2,5,7,8-tetramethyl-2H-1-benzopyran-2-yl]carbonyl}-4-(4-aminophenyl)piperazine;
- hexahydro-4-(4-nitrophenyl)-1H-1,4-diazepine ;
- 1-[(3,4-dihydro-6-hydroxy-2,5,7,8-tetramethyl-2H-1-benzopyran-2-yl)carbonyl] hexahydro-4-(4-nitrophenyl)-lH-1,4-diazepine;
- 1-(4-aminophenyl)-4-[(3,4-dihydro-6-hydroxy-2,5,7,8-tetramethyl-2H-1-benzopyran-2-yl)carbonyl] hexahydro-1H-1,4-diazepine;
- N-[4-{4-[(3,4-dihydro-6-hydroxy-2,5,7,8-tetramethyl-2H-1- benzopyran-2-yl)carbonyl]-1H-1,4-diazepin-1-yl} phenyl]-2-thiophenecarboximidamide hydrochloride;
- (*R*)-3,4-dihydro-2,5,7,8-tetramethyl-2-{4-[(4-nitrophenyl)-1-piperazinyl]-carbonyl}-2H-1-benzopyran-6-ol;
- (*R*)-3,4-dihydro-2,5,7,8-tetramethyl-2-{4-[(4-aminophenyl)-1-piperazinyl]-carbonyl}-2H-1-benzopyran-6-ol;
- (*S*)-3,4-dihydro-2,5,7,8-tetramethyl-2- {4-[(4-nitrophenyl)-1-piperazinyl]-carbonyl} -2H-1-benzopyran-6-ol;
- (*S*)-3,4-dihydro-2,5,7,8-tetramethyl-2-{4-[(4-aminophenyl)-1-piperazinyl]-carbonyl}-2H-1-benzopyran-6-ol;
- 3,5-bis-(1,1-dimethylethyl)-4-hydroxy-N-[2-(3-nitrophenyl)ethyl]-benzamide;
- 3,5-bis-(1,1-dimethylethyl)-4-hydroxy-N-[2-(3-aminophenyl)ethyl]-benzamide;
- 2-(4-nitrophenyl)ethyl 3,5-his-(1,1-dimethylethyl)-4-hydroxybenzoate;
- 2-(4-aminophenyl)ethyl 3,5-bis-(1,1-dimethylethyl)-4-hydroxy-benzoate;

or a salt of one of the latter.

**12.** Process for the preparation of a product according to any one of claims 1 to 9, **characterized in that** a compound of general formula **(III)**

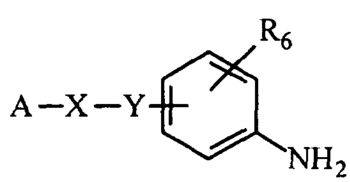

**(III)**

is reacted, in a lower alcohol such as methanol, ethanol, isopropyl alcohol or t-butanol, preferably in isopropyl alcohol, with a compound of general formula **(IV)**,

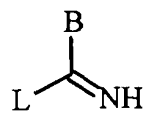

(IV)

said compound of general formula **(IV)** being optionally able to be salified by a mineral acid G, preferably HCl, HBr or HI, the compounds of general formulae **(III)** and **(IV)** being such that:

A represents:

either a

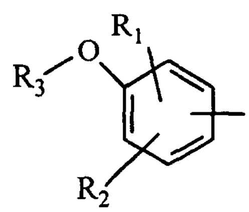

radical, $R_1$ and $R_2$ representing, independently, a hydrogen atom, a halogen, the OH group, a linear or branched alkyl radical with 1 to 6 carbon atoms, a linear or branched alkoxy radical with 1 to 6 carbon atoms,
$R_3$ representing a hydrogen atom, a linear or branched alkyl radical with 1 to 6 carbon atoms or a $-COR_4$ radical,
and $R_4$ representing a linear or branched alkyl radical with 1 to 6 carbon atoms,

or a

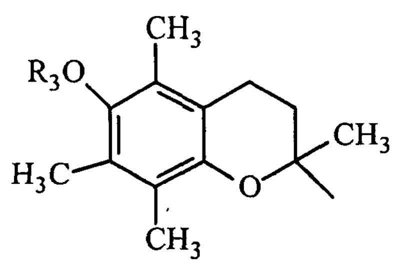

radical in which $R_3$ has the meaning indicated above

or a

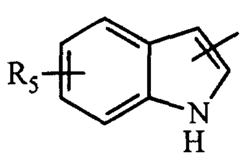

radical in which $R_5$ represents a hydrogen atom, the OH group or a linear or branched alkyl or alkoxy radical with 1 to 6 carbon atoms;

B represents a linear or branched alkyl radical with 1 to 6 carbon atoms, carbocyclic or heterocyclic aryl radical with 5 or 6 members containing 1 to 4 heteroatoms chosen from O, S, N and more particularly: thiophene, furane, pyrrole or thiazole, the carbons of which are optionally substituted by one or more groups chosen from an alkyl, an alkenyl or an alkoxy radical with 1 to 6 carbon atoms and being linear or branched;

L represents a parting group, and in particular a thioalkyl radical, sulphonic acid, trifluoromethanesulphonic acid, halide, aryl alcohol or tosyl;

X represents $-Z_1-$, $-Z_1-CO-$, -CH=CH-CO-, $-Z_1-NR_3-CO-$, $-Z_1-NR_3-CS-$, $-Z_1-NR_3-SO_2-$ or a bond;

Y represents $-Z_2-Q$, piperazine, homopiperazine, 2-methylpiperazine, 2,5-dimethyl-piperazine, 4-aminopiperidine, $-NR_3-Z_2-Q-$, $-NR_3-CO-Z_2-Q-$, $-NR_3-NH-CO-Z_2-$, $-NH-NH-Z_2-$, $-NR_3-O-Z_2-$, $-NR_3-SO_2-NR_3-Z_2-$, $-O-Z_2-Q-$, $-O-CO-Z_2-Q-$ or $-S-Z_2-Q-$,
with Q = a bond, $O-Z_3$ , $R_3-N-Z_3$ or $S-Z_3$;

$Z_1$, $Z_2$ and $Z_3$ represent independently a bond or a linear or branched alkylene radical with 1 to 6 carbon atoms;

$R_6$ represents a hydrogen atom or an OH group.

**13.** As a medicament, a product of general formula **(I)** according to any one of claims 1 to 9, or a pharmaceutically acceptable salt of this product.

**14.** Pharmaceutical composition containing as active ingredient at least one product according to claim 13.

**15.** Pharmaceutical composition containing as active ingredient (S)-N- {4-[4-[(3,4-dihydro-6-hydroxy-2,5,7,8-tetramethyl-2H-l -benzopyran-2-yl)-carbonyl]-1-piperazinyl]phenyl}-2-thiophenecarboximidamide or a pharmaceutically acceptable salt of the latter.

**16.** Use of a product of general formula **(I)** according to any one of claims 1 to 9, or of a pharmaceutically acceptable salt of this product, for producing a medicament intended to inhibit neuronal NO synthase.

**17.** Use of a product of general formula **(I)** according to any one of claims 1 to 9, or of a pharmaceutically acceptable salt of this product, for producing a medicament intended to inhibit inducible NO synthase.

**18.** Use of a product of general formula **(I)** according to any one of claims 1 to 9, or of a pharmaceutically acceptable salt of this product, for producing a medicament intended to inhibit lipidic peroxidation.

**19.** Use of a product of general formula **(I)** according to any one of claims 1 to 9, or of a pharmaceutically acceptable salt of this product, for producing a medicament having both an inhibition activity on NO synthase and on lipidic peroxidation.